# EUROPEAN PATENT APPLICATION

(11) **EP 1 190 710 A1**
(43) Date of publication of application: **27.03.2002**
(21) Application number: 00927740.1
(22) Date of filing: 10.05.2000
(51) Int. Cl.: A61K 31/00, C07C 27/00, C07D 211/00, C07D 213/00

(54) **PREVENTIVE OR THERAPEUTIC DRUGS FOR DIABETES**

(30) Priority: 13.05.1999 JP 13237599
(71) Applicant: SHIONOGI & CO., LTD., Osaka-shi, Osaka 541-0045 (JP)
(72) Inventor: YANO, Toshisada, c/o Shionogi & Co Ltd, Osaka-shi, Osaka 553-0002 (JP); SAKAGUCHI, Isako, c/o Shionogi & Co Ltd, Koka-gun, Shiga 520-3423 (JP); KATSUURA, Goro, c/o Shionogi & Co Ltd, Koka-gun, Shiga 520-3423 (JP)
(74) Representative: VOSSIUS & PARTNER
(86) International application number: JP0002992
(87) International publication number: WO0124786

(57) **Abstract**

A composition for use as a preventive or therapeutic agent for diabetes, containing a compound of the formula (I): 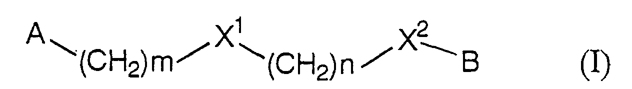 wherein, A is optionally substituted aryl or optionally substituted heteroaryl; B is lower alkyl, optionally substituted aryl and the like; X¹ is -O-, -S-, or -N R^{a}- wherein R^{a} is hydrogen or lower alkyl; X² is -NR^{b}CO-, -CONR^{b}-, -NR^{b}CONR^{b}-, -SO₂-, -NR^{b}SO₂-, -D-, -D-O-, -D-CO-, -D-SO₂-, -D-NR^{b}CO- or -D- NR^{b}SO₂- wherein D is a divalent heterocyclic group; R^{b} is hydrogen or optionally substituted lower alkyl; m is an integer of 0 to 3;
n is an integer of 2 to 5.

## Description

### Technical Field

The present invention relates to pharmaceutical compositions such as a preventive or therapeutic agent for diabetes.

### Background Art

Diabetes is usually divided into two types: insulin dependent diabetes mellitus (type I, IDDM) accompanied with the decrease of insulin-producing cells and non-insulin dependent diabetes mellitus (typeII, NIDDM) which is considered to be generated by the decrease of insulin sensibility. As the clinical situation, 90% or more of the patients of diabetes are involved in the latter. In the non-insulin dependenct diabetes mellitus (type II diabetes), while the concentration of insulin in blood is high, the sensibility of somatic cells to insulin is decreased due to insulin resistance. Thus, the intake of glucose existing in blood into somatic cell is inhibited. Nowadays, only thiazolidin derivatives (e.g., troglitazone, pioglitazone, rosiglitazone) are on the market as the therapeutic agent for type II diabetes which improves insulin resistance.

On the other hand, documents disclosing aryl sulfonamide derivatives includes, for example, WO96/33160 (use: insecticide), SU1596696 (use: insecticide, growth prpmoter) and JP(A)07/206815 (use: insecticide,antibiotics), wherein any preventive or therapeutic agent for diabetes is not described.

Therefore, the development of new preventive or therapeutic agents for type I or II diabetes has been desired.

### Disclosure of Invention

The present inventors have intensively studied to find out that amide derivatives, sulfoneamide derivatives and other compounds have effects lowering the concentration of glucose in blood and thus useful as preventive or therapeutic agents for diabetus, and have accomplished the present invention shown below.
(1) A composition for use as a preventive or therapeutic agent for diabetes, containing a compound of the formula (I): 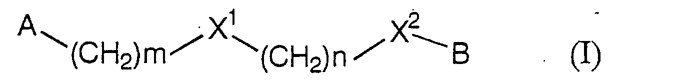 wherein A is optionally substituted aryl or optionally substituted heteroaryl; B is lower alkyl, optionally substituted aryl, optionally substituted aryl(lower)alkyl, optionally substituted aryl(lower)alkenyl, optionally substituted heteroaryl, optionally substituted heteroaryl(lower)alkyl, or optionally substituted heteroaryl(lower)alkenyl;
   X¹ is -O-, -S-, or -NR^{a}- wherein R^{a} is hydrogen or lower alkyl;
   X² is -NR^{b}CO-, -CONR^{b}-, -NR^{b}CONR^{b}-, -SO₂-, -NR^{b}SO₂-, -D-, -D-O-, -D-CO-,
   -D-SO₂-, -D-NR^{b}CO- or -D-NR^{b}SO₂- wherein D is a divalent heterocyclic group; R^{b} is hydrogen or optionally substituted lower alkyl;m is an integer of 0 to 3; n is an integer of 2 to 5;
   prodrug, pharmaceutically acceptable salt, or solvate thereof.
(2) The composition for use as a preventive or therapeutic agent for diabetes described in above (1) wherein A is optionally substituted phenyl, optionally substituted naphtyl, optionally substituted pyridyl, optionally substituted quinoryl, optionally substituted isoquinoryl, optionally substituted furyl, optionally substituted thienyl, optionally substituted benzofuryl, or optionally substituted benzothienyl.
(3) The composition for use as a preventive or therapeutic agent for diabetes described in above (2) wherein A is optionally substituted phenyl.
(4) The composition for use as a preventive or therapeutic agent for diabetes described in above (3) wherein A is phenyl substituted with one to three halogens.
(5) The composition for use as a preventive or therapeutic agent for diabetes described in above (4) wherein A is phenyl substituted with two halogens.
(6) The composition for use as a preventive or therapeutic agent for diabetes described in above (3) wherein A is phenyl substituted with halogen and lower alkyl.
(7) The composition for use as a preventive or therapeutic agent for diabetes described in above (3) wherein A is phenyl substituted with lower alkyl or haloganated lower alkyl.
(8) The composition for use as a preventive or therapeutic agent for diabetes described in above (3) wherein A is phenyl substituted with substituted phenyloxy.
(9) The composition for use as a preventive or therapeutic agent for diabetes described in above (3) wherein A is one or more group(s) selected from the group consisting of halogen, lower alkyl, lower alkoxy and haloganated lower alkyl and phenyl which is substituted with substituted phenyloxy.
(10) The composition for use as a preventive or therapeutic agent for diabetes described in above (1) wherein B is lower alkyl, optionally substituted phenyl, optionally substituted benzyl, optionally substituted 2-phenylvinyl, optionally substituted pyridyl, optionally substituted 2-pyridylvinyl, optionally substituted furyl, optionally substituted 2-furylvinyl, optionally substituted thienyl, optionally substituted 2-thienylvinyl, or optionally substituted thiomorphorinyl.
(11) The composition for use as a preventive or therapeutic agent for diabetes described in above (1) wherein when B is an optionally substituted group, the substituent being selected from the group consisting of halogen, amino, carboxyl, hydroxy, cyano, lower alkyl, optionally substituted alkoxy, lower alkoxycarbonyl, halogenated lower alkyl, aryloxy, and heteroaryloxy.
(12) The composition for use as a preventive or therapeutic agent for diabetes described in above (1) wherein X¹ is O.
(13) The composition for use as a preventive or therapeutic agent for diabetes described in above (1) wherein X² is -NHCO-, -NHCONH-, NR^{b}SO₂, -SO₂-, -D-, -D-O-, -D-SO₂-, -D-NR^{b}CO-, or -D-NR^{b}SO₂- wherein D is piperidin-1,4-diyl or piperazin-1,4-diyl, and R^{b} is the same as above.
(14) The composition for use as a preventive or therapeutic agent for diabetes described in above (1) wherein m is 0.
(15) The composition for use as a preventive or therapeutic agent for diabetes described in above (1) wherein n is 3.
(16) The composition for use as a preventive or therapeutic agent for diabetes described in above (1) wherein A is optionally substituted phenyl; B is lower alkyl, optionally substituted phenyl, optionally substituted benzyl, optionally substituted 2-phenylvinyl, optionally substituted pyridyl, optionally substituted 2-pyridylvinyl, optionally substituted furyl, optionally substituted 2-furylvinyl, optionally substituted thienyl, optionally substituted 2-thienylvinyl or optionally substituted thiomorphoryl; X¹ is O; X² is -NHCO-, -NHCONH-, -SO₂-, -NR^{b} SO₂-, -D-, -D-O-, -D-SO₂-, -D-NR^{b}CO- or -D-NR^{b}SO₂- wherein D is piperidin-1,4-diyl or piperazin-1,4-diyl, R^{b} is the same meaning as above);
   m is 0; and n is 3.
(17) A compound of the formula(II): 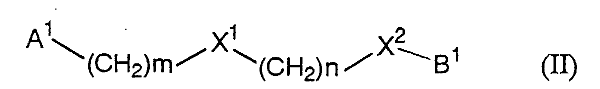 wherein A¹ is any one of groups shown by the formulae: 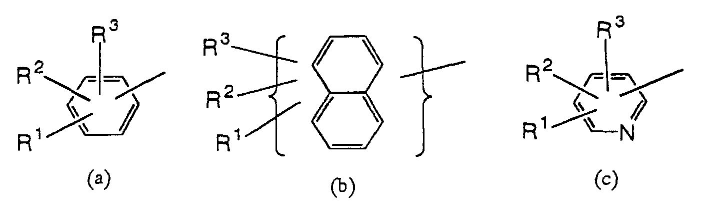 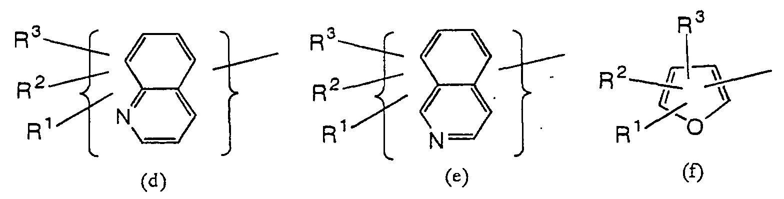 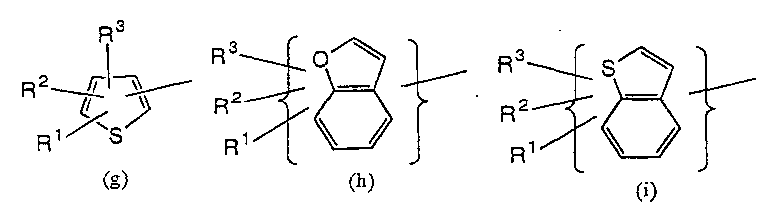 wherein R¹, R² and R³ are each independently hydrogen, halogen, lower alkyl, lower alkoxy, halogenated lower alkyl, or substituted phenyloxy;
   B¹ is lower alkyl or any one of groups shown by the formulae: 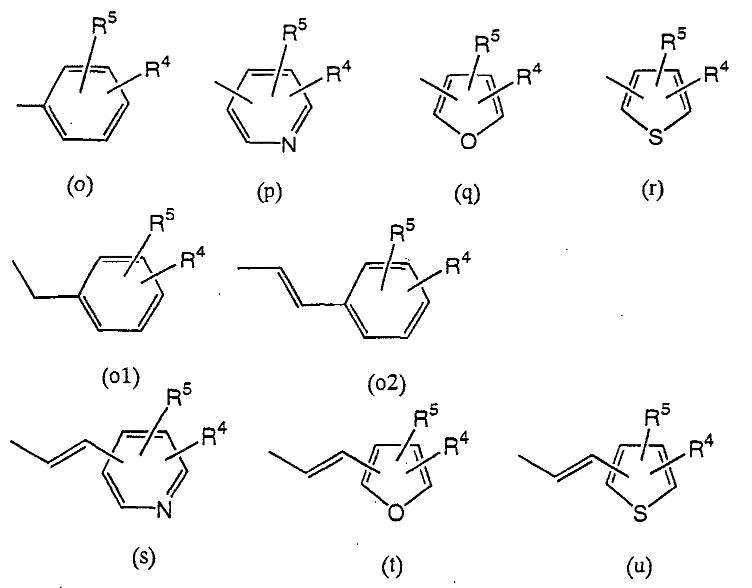 wherein R⁴ and R⁶ are each independently hydrogen, halogen, amino, carboxyl, hydroxy, cyano, lower alkyl, optionally substituted lower alkoxy, lower alkoxy carbonyl, halogenated lower alkyl, aryloxy or heteroaryloxy;
   X¹ is -O-, -S- or -NR^{a}- wherein R^{a} is hydrogen or lower alkyl; X² is -NR^{b}CO-, -CONR^{b}-, -NR^{b}CONR^{b}-, -SO₂-, -NR^{b}SO₂-, or any one of groups shown by the formulae: 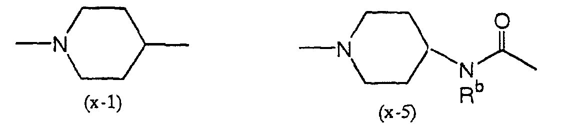 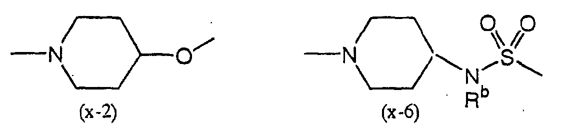 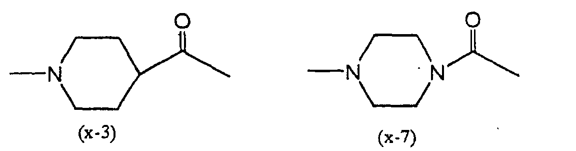 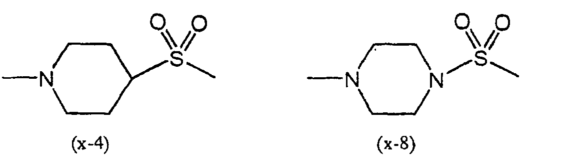 wherein each R^{b} is independently hydrogen or optionally substituted lower alkyl;
   m is an integer of 0 to 3;
   n is an integer of 2 to 5;
   excluding the following 1) and 2):
   1) A¹ is the group of (a), R¹ is hydrogen, R² and R³ are chloro; B¹ is the group of (o), and R⁴ is chloro or methyl, and R⁵ is hydrogen; X¹ is O; X² is NR^{b} SO₂; m is 0; and n is 2, and
   2) A is the group of (a), R¹ is hydrogen, R² and R³ are any groups selected from the group consisting of hydrogen, halogen and lower alkoxy; X¹ is O; X² is the group of (x-1); B¹ is lower alkyl or phenyl; m is 0; and n is 3; prodrug, pharmaceutically acceptable salt, or solvate thereof.
(18) The compound described in above (17) wherein A¹ is any one of groups shown by the formulae: 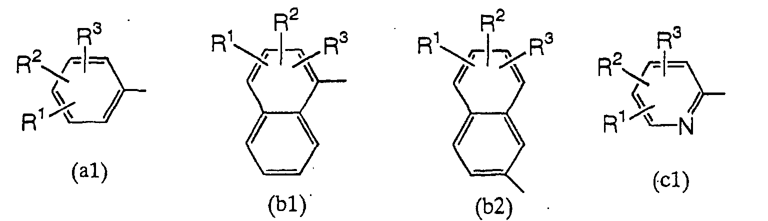 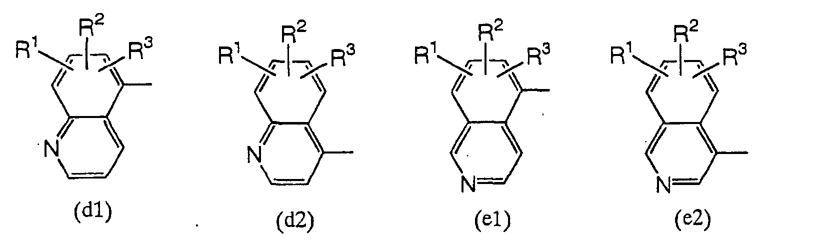 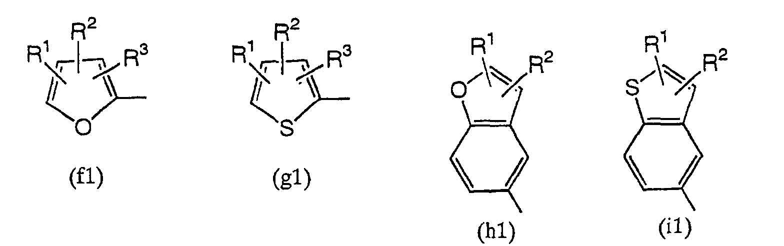 wherein each symbol is the same meaning as described above; B¹ is any one of groups shown by the formulae: 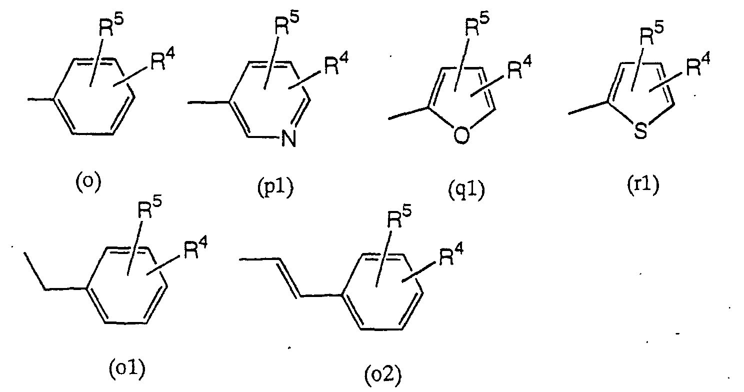 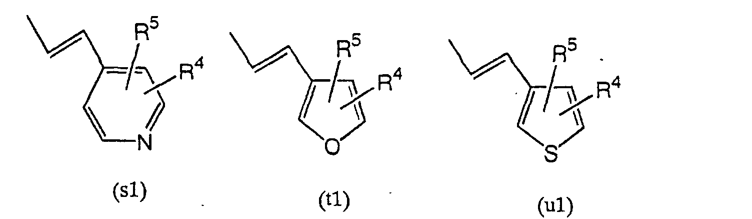 wherein R⁴ and R⁵ are the same meanings as described above.
(19) The compound described in above (18) wherein A¹ is the group of (a1); R¹ is hydrogen; R² is halogen, lower alkyl, lower alkoxy, halogenated lower alkyl, or substituted phenyloxy; and R³ is hydrogen, halogen, lower alkyl, lower alkoxy, or halogenated lower alkyl.
(20) The compound described in above (19) wherein R³ is hydrogen.
(21) The compound described in above (18) wherein B¹ is the group of (o), (o1) or (o2); R⁴ is hydrogen, and R⁵ is hydrogen, halogen, amino, carboxyl, hydroxy, cyano, lower alkyl, optionally substituted lower alkoxy, lower alkoxycarbonyl, halogenated lower alkyl, aryloxy, or heteroaryloxy.
(22) The compound described in above (17) or (18) wherein X¹ is O.
(23) The compound described in above (17) wherein X² is -NHCO-, -NHCONH-, -NHSO₂- or any one of groups shown by the formulae: 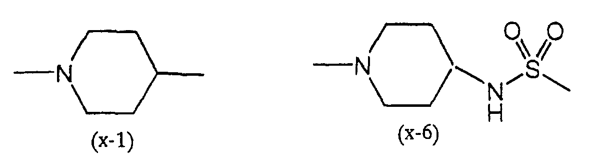 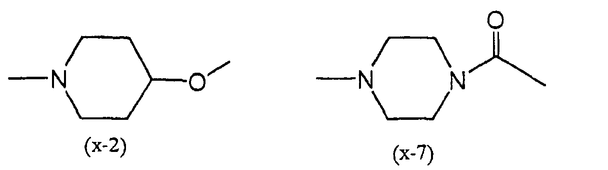 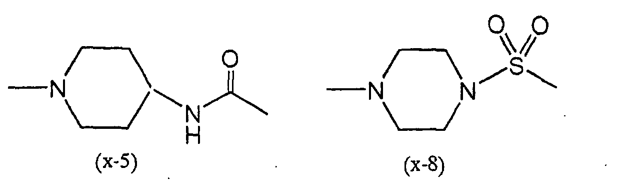
(24) The compound described in above (17) or (18) wherein m is 0.
(25) The compound described in above (17) or (18) wherein n is 3.
(26) The compound described in above (17) or (18) wherein A¹ is the group of (a1); R¹ is hydrogen; R² is halogen, lower alkyl, lower alkoxy, halogenated lower alkyl or substituted phenyloxy; R³ is hydrogen, halogen, lower alkyl, lower alkoxy or halogenated lower alkyl; B¹ is the group of (o), (o1) or (o2); R⁴ is hydrogen, R⁵ is hydrogen, halogen, amino, carboxyl, hydroxy, cyano, lower alkyl, optionally substituted lower alkoxy, lower alkoxycarbonyl, halogenated lower alkyl, aryloxy, or heteroaryloxy; X¹ is 0; X² is -NHCO-, -NHCONH-, -NHSO₂- or any one of groups shown by 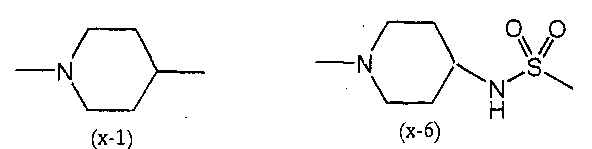 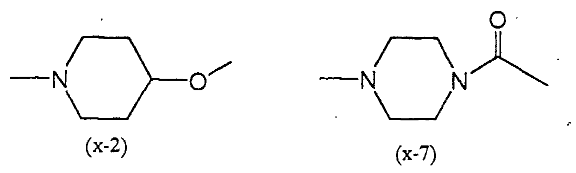 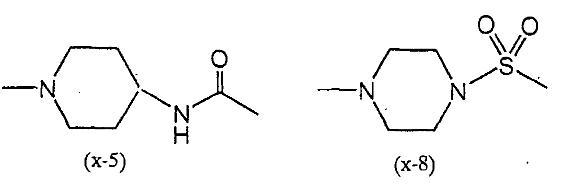 m is 0 and n is 3.
(27) The compound described in above (26) wherein R³ is, hydrogen; and R⁵ is hydrogen, halogen, carboxyl, hydroxy, lower alkyl, optionally substituted lower alkoxy, lower alkoxycarbonyl or halogenated lower alkyl.
(28) A pharmaceutical composition containing a compound described in any one of above (17) to (27).
(29) A composition for use as a preventive or therapeutic agent for diabetes containing a compound described in any one of above (17) to (27).
(30) A composition for use as a preventive or therapeutic agent for diabetes containing a compound described in above (27).
(31) A method for the prevention or therapy of diabetes, which comprises administering a compound described in any one of above (1) to (27).
(32) Use of a compound described in any one of above (1) to (27) for production of a composition for use as a preventive or therapeutic agent for diabetes.

### Best Mode for Carrying Out the Invention

Terms used herein are explained below. Unless otherwise mentioned, each term, by itself or as part of another, has the following meaning.

Examples of lower alkyl include a straight or branched C1 to C6 alkyl such as methyl, ethyl, n-propyl, i-propyl, n -butyl, i-butyl, sec-butyl, t-butyl, n-pentyl, i-pentyl, neo-pentyl, tert-pentyl, n -hexyl, and the like, preferred is C1 to C4 alkyl, and more preferred is methyl and n-butyl.

Examples of lower alkenyl include a straight or branched C2 to C6 alkenyl such as vinyl, aryl, i-propenyl, 2-butenyl, 3-pentenyl, 2-hexenyl and the like, preferred is C2 to C4 alkenyl, and more preferred is vinyl.

Lower alkoxy includes oxy connected with the above lower alkyl, such as methoxy, ethoxy, i-propoxy, tert-butoxy, pentyloxy, hexyloxy and the like and preferred is methoxy.

Halogen means F, Cl, Br and I and preferred is F or Cl.

Aryl means a monocyclic or fused aromatic hydrocarbon group such as phenyl, α-naphthyl, β-naphthyl, anthryl, indenyl, phenantryl and the like, preferred is phenyl, α -naphthyl, β -naphthyl and more preferred is phenyl.

Heteroaryl means an aromatic monocyclic or polycyclic group containing the same or different heteroatom selected from O, S and N.

The monocyclic group includes a 5- to 6-memberd cyclic group containing one to four hetero atom as the aromatic cyclic group such as pyridyl, furyl, thienyl, tetrazolyl, pyrolyl, pyrazolyl, imidazolyl, oxazolyl, thiazolyl, thiadiazolyl, oxazinyl, triazinyl and the like and preferred is pyridyl (e.g., 2-pyridyl, 3-pyridyl, 4-pyridyl), furyl (e.g.,2-furyl, 3-furyl) or thienyl (e.g., 2-thienyl, 3-thienyl).

The polycyclic group includes a 2- or 3-cyclic heterocyclic group containing one to five hetero atom and preferred is a 8- to 14-membered cyclic group such as quinolyl, isoquinolyl, indolyl, benzoimidazolyl, indazolyl, indolizinyl, benzofuryl, benzothienyl, acrydinyl, phenanthrydinyl and the like, and preferably quinolyl (e.g., 4-quinolyl, 5-quinolyl), isoquinolyl (e.g., 4-isoquinolyl, 5-isoquinolyl), benzofuryl (e.g., 5- benzo [b] furyl), and benzothienyl (e.g.,5-benzo [b] thienyl).

When the aryl or heteroaryl has a substituent, examples of the substituent include the same or different group selected from the group consisting of halogen, lower alkyl, lower alkoxy, lower alkylthio, hydroxy, amino, carboxyl, cyano, nitro, lower alkylcarbonyl, lower alkoxycarbonyl, halogenated lower alkyl, aryloxy, heteroaryloxy and the like. These may be located at any substitutable position in a range of one to five, preferably one to three. The preferable substituent on the cyclic group shown by A is above R¹, R² and R³ and a preferable substituent on the cyclic group shown by B is above R⁴.

Preferable A is optionally substituted phenyl (e.g., phenyl, 2,3-dichlorophenyl, 2,4-diflorophenyl, phenyl substituted with chloro and methyl and dimethylphenyl), optionally substituted naphtyl (e.g., α - or β -naphtyl, 4-chloro-l-naphthyl), optionally substituted pyridyl (e.g., 4-pyridyl, 6-chloro-2-hydroxy-4-pyridyl), optionally substituted quinolyl (e.g., 5-quinolyl, 6-chloro-8-hydroxy-5-quinolyl), optionally substituted isoquinolyl (e.g., 4-isoquinolyl, 6-chloro-4-isoquinolyl), optionally substituted furyl (e.g.,2-furyl, 4-chloro-2-furyl), optionally substituted thienyl (e.g.,2-thienyl, 4-chloro-2-thienyl), optionally substituted benzofuryl (e.g., 5-benzo[b]furyl, 3-chloro-5-benzo [b] furyl) or optionally substituted benzothienyl (e.g., 5-benzo [b] thienyl, 3-chloro-5-benzo [b] thienyl) and the like. Preferred is each group described in above A¹. More preferably, A is phenyl substituted by one to three, especially two halogens, phenyl substituted with halogen and lower alkyl, phenyl substituted with two lower alkyl, phenyl substituted with substituted phenyloxy, or phenyl substituted with.one or more groups selected from group consisting of halogen, lower alkyl, lower alkoxy and halogenated lower alkyl, and phenyl substituted with substituted phenloxy. In oral administration, A is more preferred phenyl substituted with substituted phenyloxy and the like.

B is preferably n-butyl, optionally substituted phenyl, optionally substituted benzyl, optionally substituted 2-phenylvinyl, optionally substituted pyridyl (e.g., 3-pyridyl, 4-pyridyl), optionally substituted 2-pyridyl vinyl (e.g., 2-(4-pyridyl) vinyl), optionally substituted furyl (e.g., 2-furyl, 3-furyl), optionally substituted 2-furylvinyl (e.g., 2-(3-furyl)vinyl), optionally substituted thienyl (e.g., 2-thienyl, 3-thienyl) or optionally substituted 2-thienylvinyl (e.g., 2-(3-thienyl)vinyl) and the like. These substituent is preferably halogen, amino, carboxyl, lower alkyl (e.g., methyl, t-butyl), lower alkoxy (e.g., methoxy), 4-pyridyloxy, phenoxy and the like. The location of substituent is preferred para position. More preferable is. optionally substituted phenyl, optionally substituted 2-phenyl-vinyl or piridyl. As B, each group in B' described above is included.

X¹ is preferably 0 or S, and more preferably O.

An Embodiment of two valent heterocyclic group in X² shown by D includes an aromatic or non-aromatic five to six membered group containing one to two same or different hetero atom selected from O, S and N, preferably non-aromatic group containing N atom, and more preferably piperidin-diyl (e.g., piperidin-1,4-diyl), piperadin-diyl (e.g., piperazin-1,4-diyl) and the like.

Examples of the substituent in "optionally substituted lower alkyl" in R^{b} include amino, substituted amino (substituent: lower alkyl such as methyl, ethyl and the like), hydroxy, carboxyl, lower alkoxy carbonyl (e.g., methoxy carbonyl, ethoxy carbonyl and the like), optionally substituted aryl (e.g., p-carboxyphenyl, p-methoxycarbonylphenyl), optionally substituted and optionally fused hetero cycle (e.g., futalimide and the like), and preferably carboxy.

Examples of R^{b} includes hydrogen, methyl, ethyl, propyl, aminomethyl, aminoethyl, methylaminopropyl, hydroxypropyl, carboxyethyl, methoxycarbonylethyl, p-carboxybenzyl, p-methoxybenzyl, phthalimidepropyl and the like, and preferably carboxymethyl and the like.

m is preferably 0.

n is preferably 3.

Preferable embodiments of the compound (I) include the above compound (II). In compound (II), A¹ is preferably the group of (a1), (b1), (b2), (c1), (d1), (d2), (e1), (e2), (f1), (g1), (h1) or (i1), and more preferable group is (a1) or (c1). Most preferably, A¹ is the group of (a1) and R¹ is hydrogen, R² is halogen (e.g., F, Cl and the like), lower alkyl (e.g., methyl, t-butyl and the like), lower alkoxy (e.g., methoxy, ethoxy and the like), halogenated lower alkyl (e.g., CF₃ and the like) or substituted phenyloxy (example of substituent: methyl, t-butyl, methoxy, CF₃, halogen and the like); R³ is hydrogen, halogen (e.g., F, Cl and the like), lower alkyl (e.g., methyl, t-butyl and the like), lower alkoxy (e.g., methoxy, ethoxy and the like) or halogenated lower alkyl (e.g.,CF₃ and the like). Most preferably, R³ is hydrogen. When R² and R³ are halogen, m- and p-position is preferable.

B¹ is preferably the group of (o), (o1) or (o2). R⁴ is preferably hydrogen, R⁵ is preferably hydrogen, halogen, amino, carboxyl, hydroxy, cyano, lower alkyl (e.g., methyl, ethyl, isopropyl, t-butyl and the like), optionally substituted lower alkoxy (e.g., methoxy, carboxymethoxy, methoxycarbonyl-methoxy and the like), lower alkoxycarbonyl (e.g., methoxycarbonyl and the like), halogenated lower alkyl (e.g., CF₃ and the like), aryloxy (e.g.,phenyloxy) or heteroaryloxy (e.g., 2-thienyloxy, 4-pyridyloxy and the like).

X¹ is preferably O. X² is preferably -NHCO-, -NHCONH-, -NHSO₂- or any of the groups shown below:

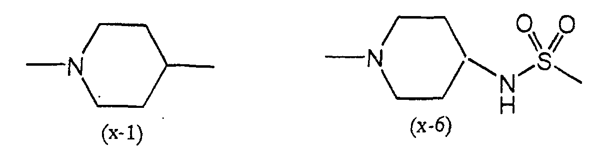

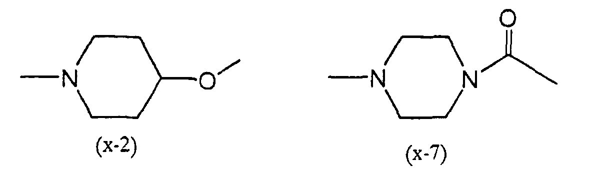

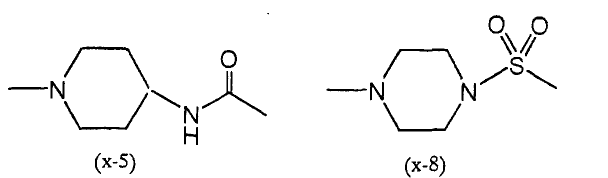

m is preferably 0. n is preferably 3.

As compound (II), preferably, A¹ is the group of (a1); R¹ is hydrogen; R² is halogen, lower alkyl, lower alkoxy, halogenated lower alkyl, or substituted phenyloxy; R³ is hydrogen, halogen, lower alkyl, lower alkoxy, or halogenated lower alkyl; B¹ is the group of (o), (o1) or (o2); R⁴ is hydrogen, R⁵ is hydrogen, halogen, amono, carboxyl, hydroxy, cyano, lower alkyl, optionally substituted lower alkoxy, lower alkoxycarbonyl, halogenated lower alkyl, aryloxy or heteroaryloxy; X¹ is O; X² is -NHCO-, -NHCONH-, -NHSO₂- or described above the group shown by (x-1), (x-2), (x-5), (x-6), (x-7) or (x-8): m is 0 and n is 3. In the above group, more preferably, R³ is hydrogen; R⁵ is hydrogen, halogen, carboxyl, hydroxy, lower alkyl, optionally substituted lower alkoxy, lower alkoxy carbonyl or halogenated lower alkyl.

The typical preparation of compound (I) is exemplified below and the compound (II) can be prepared likewise.

### (Preparation 1) (for X² is -NR^{b}SO₂-, -NR^{b}CO- or -NR^{b}CONR^{b}-)

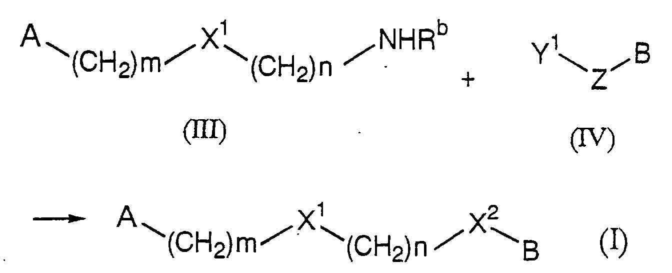

wherein Y¹ is a leaving group (e.g, halogen and the like); Z is SO₂, CO, CONR^{b}; the other symbols are the same meaning as described above.

The compound (III) and the compound (IV) are reacted, if necessary in the presence of a base, to give the compound (I). As the base, carbonate (K₂CO₃, Na₂CO₃ and the like), NaOH, tertiary amine (e.g., Et₃N) and the like can be used. Furthermore, KI may be used together with them. As a solvent, CH₃CN, dimethylformamide (DMF), dimethylsulfoxide (DMSO) tetrahydrofuran (THF) and the like can be used. The reaction temperature is usually about 10 to 200°C, preferably room temperature to about 110°C, reaction time is several hours to several ten hours, preferably about one to 20 hours, more preferably about 3 to 15 hours. The compound (III) and the compound (IV) are prepared by known reactions or may be commercially available.

### (Preparation 2) (For X² is -SO₂-)

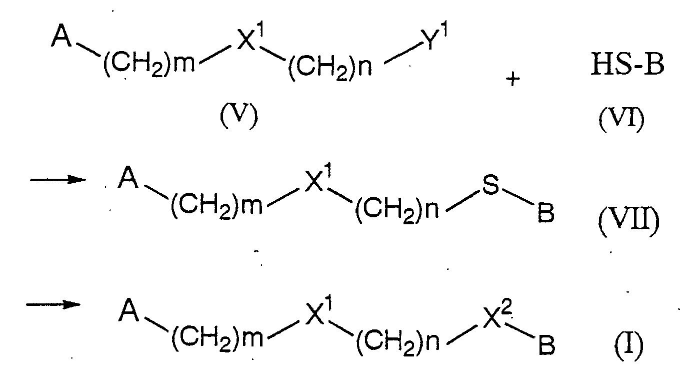

wherein the each symbol is the same meaning as described above.

The compound (V) and the compound (VI) are reacted, if necessary in the presence of a base, to give the compound (VII). As the base, carbonate (K₂CO₃, Na₂CO₃ and the like), NaOH, t-BuOK, tertiary amine (e.g.,Et₃N) and the like can be used. Moreover, KI may be used together with them. As a solvent, CH₂Cl₂, CH₃CN, dimethylformamide (DMF), dimethylsulfoxide (DMSO), tetrahydrofuran (THF) and the like can be used. The reaction temperature is usually about room temperature to 100°C, preferably room temperature to about 60°C. The reaction time is several hours to several ten hours, preferably about 1 to 20 hours, more preferably about 3 to 15 hours. The compound (V) and the compound (VI) are prepared by known reactions or may be commercially available products.

Next, the compound (VII) is oxidized to give the compound (I). As oxidizing agent, m-chloroperoxybenzoic acid, hydrogen peroxide, peroxy acid, potassium permanganate, periodic acid and the like can be used. As a solvent, CH₂Cl₂, CH₃CN, dimethylformamide (DMF), dimethylsulfoxide (DMSO), tetrahydrofuran (THF) and the like can be used. The reaction temperature is usually about 0 to 50°C, preferably about 0°C to room temperature. The reaction time is several hours to several ten hours, preferably about 1 to 20 hours, more preferably about 3 to 15 hours.

### (Preparation 3) (For X² is -D-, -D-O-, -D-NR^{b}CO-, -D-NR^{b}SO₂-)

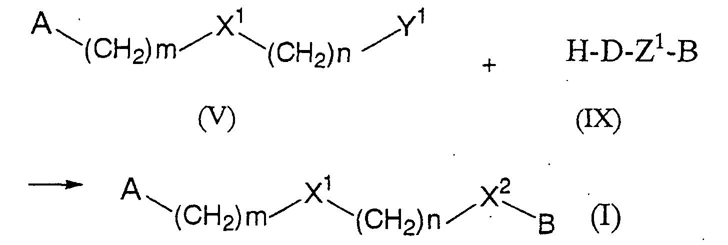

wherein Z¹ is single bond, -O-, -NR^{b}CO-, -NR^{b}SO₂-, the other symbol is the same meaning as described above.

The compound (V) and the compound (IX) are reacted, if necessary in the presence of a base, to give the compound (I). As the base, carbonate (K₂CO₃, Na₂CO₃ and the like), NaOH, tertiary amine and the like can be used. Moreover, KI may be used together with them. As a solvent, CH₃CN, dimethylformamide (DMF), dimethylsulfoxide (DMSO), and the like can be used. The reaction temperature is usually about 10 to 200°C, preferably room temperature to about 110°C. The reaction time is several hours to several ten hours, preferably about 1 to 20 hours, more preferably about 3 to 15 hours. The compound (IX) are prepared by known reactions or may be commercially available products.

### (Preparation 4) (For X² is -D-CO-, -D-SO₂-)

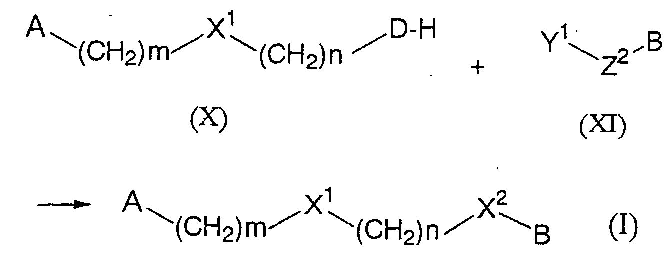

wherein Z² is CO, SO₂; the other symbol is the same meaning as described above.

The compound (X) and the compound (XI) are reacted, if necessary in the presence of a base, to give the compound (I). As the base, carbonate (K₂CO₃, Na₂CO₃ and the like), NaOH, tertiary amine (e.g.,Et₃N) and the like can be used. Moreover, KI may be used together with them. As a solvent, CH₃CN, dimethylformamide (DMF), dimethylsulfoxide (DMSO), tetrahydrofuran (THF) and the like can be used. The reaction temperature is usually about 10 to 200°C, preferably room temperature to about 110°C. The reaction time is several hours to several ten hours, preferably about 1 to 20 hours, more preferably about 3 to 15 hours. The compound (III) and compound (IV) are prepared by known reactions or may be commercially available products.

Prior to the above each reaction, a functional group may be protected by a method well known to skilled persons and if necessary deprotected after the reaction.

Examples of the pharmaceutically acceptable salt of compound (I) include salts formed with inorganic bases, ammonia, organic bases, inorganic acids, organic acids, basic amino acids, halogen ions or the like, and inner molecular salts. Examples of the inorganic base include alkaline metal (e.g., Na, K and the like) and alkaline earth metal (e.g., Ca, Mg and the like). Examples of the organic base include trimethylamine, triethylamine, choline, procaine, ethanol amine or the like. Examples of the inorganic acid include hydrochloric acid, hydrobromic acid, sulfuric acid, nitric acid, phosphoric acid and the like. Examples of the organic acid include p-toluene sulfonic acid, methanesulfonic acid, formic acid, trifluoroacetic acid and maleic acid, oxalic acid and the like. Examples of the basic amino acid include lysine, arginine, ornithine, histidine and the like. Compound (I) may be solvate such as water, alcohol and the like.

Prodrug means a derivarive of the present invention compound, which has a chemically or metabolically decomposable group and is converted, by solvolysis or under physiological conditions, to a compound of present invention which is pharmaceutically active *in vivo.* A selection and a preparation of an appropriate prodrug-derivative is described in, e.g., Design of Prodrugs, Elsevier, Amsterdam 1985.

When the compound of present invention has a carboxy group, examples of the prodrug include an ester derivative prepared by reacting a proper alcohol with an original acidic compound or an amide derivative prepared by reacting a proper amine with an original acid anhydride compound such as methyl ester, ethyl ester, n-propyl ester, isopropyl ester, n-butyl ester, isobutyl ester, tert-butyl ester, morphorinoethyl ester and the like.

When the compound of present invention has a hydroxyl group, examples of the prodrug include an acyloxy derivative prepared by reacting a proper acylharide or a proper acid anhydrate with a hydroxyl group-containing compound such as -OCOC₂H₅-, -OCO(t-Bu), -OCOC₁₅H₁₃-. -OCO(m-COONa-Ph)-, -OCOCH₂CH₂COONa-, -OCOCH(NH₂)CH₃-, -OCOCH₂N(CH₃)₂-and the like.

When the compound of present invention has an amino group, examples of prodrug include an amide derivative prepared by reacting a proper acid halide or a proper mixed acid anhydrate with an amino group-containing compound such as -NHCO(CH₂)₂₀CH₃-, -NHCOCH(NH₂)CH₃-and the like.

The compound (I), especially compound (II) can be administered orally or parenterally to animal including man as a pharmaceutical composition, especially preventive or therapeutic agent for diabetes. Examples of the administered form include granules, tablets, capsules, injections and the like. In formulation, various additives can be used such as excipients, disingrators, binders, lubricants, stabilizers, coloring agents, coating agents if necessary. Although the dosage of the compound of the present invention may vary depending on the age, weight, conditions of the patient, and the administration route and the like, the daily dose for adult can generally be about 20 to 1000 mg for oral administration. For parenteral administration, the daily dose can be about 2 to 100 mg.

When the compound (I) is administered, glucose availability increases and glucose concentration in blood decreases. As the mechanism of this effect, the possibility is thought that (1) increase of reactivity against insulin in each organ (improvement of insulin resistance), (2) acceleration of insulin secretion by increase of sensitibity against glucose in the pancreas, (3) accceleration of insulin product in the pancreas. Namely, compound (I) is a preventive and therapeutic agent against type I and II diabetes, preferably type II diabetes.

### (Example)

### (abbreviation)

Me : methyl, Et : ethyl, tBu : t-butyl, EtOH : ethanol, DMF : dimethylfolme amide, THF : tetrahydrofulan, EtOAc : ethyl acetate, n-Hex : n-hexane

### Reference 1

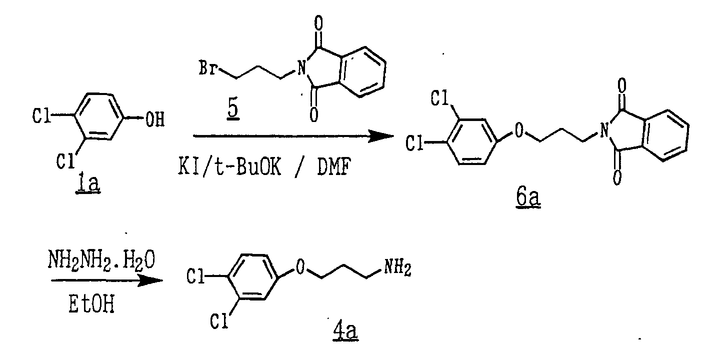

### (1) Preparation of 6a

### N-[3-(3,4-Dichloro-phenoxy)-propyl]phthalimide

DMF solution (100 mL) containing 3,4-dichloro phenol (1a) (10 g) and t-BuOK (6.9g) was mixed at room temperature for 1.5 hours. To the solution was added N-(3-bromo-propyl) phthalimide (18g) and KI (potassium iodide) (2.0g) and mixed at 90°C for 14 hours. The reacted solution was poured to water and extracted with ethyl acetate. The organic layer was washed with water and saturated brine, dried over MgSO₄ and the solvent was evaporated under reduced pressure. The resultant crude crystal was washed with ether to give (6a) (19.47g)(90%).
NMR(CDCl₃) δ ppm(200 MH_{z})
2.178(2H,quint.,J=9Hz), 3.091(2H,t.J=9Hz), 3.991(2H,t,J=9Hz), 6.642(1H,dd,J1=13Hz,J2=4Hz), 6.856(1H,d,J=4Hz), 7.267(1H,d,J=13Hz), 7.55 ∼ 7.90(4H,m)

### (2) Preparation of 4a

### 3-(3,4-Dichloro-phenoxy)-propylamine

(6a)(19.47g) was dissolved in EtOH (200ml). Thereafter, hydrazine monohydrate (5.8g) was added. The mixture was mixed for 2 hours under refluxing. Deposited crystal was filtered and filtrate was evaporated under reduced pressure to give (4a)(10.6g)(86%) as oily product.
NMR(CDCl₃) δ ppm (300 MH_{z})
1.30∼1.60(2H,m), 1.916(2H,quint.,J=7Hz), 2.897(2H,t.J=7Hz), 4.019(2H,t,J=7Hz), 6.753(1H,dd,J1=9Hz,J2=3Hz), 6.997(1H,d,J=3Hz), 7.307(1H,d,J=9Hz)
IR (Nujol) cm-1 3000,2923,2854,2788,2750,2613,2459,2069,1590,1566,1481,1467,1386,1296,12 32

### Example 1

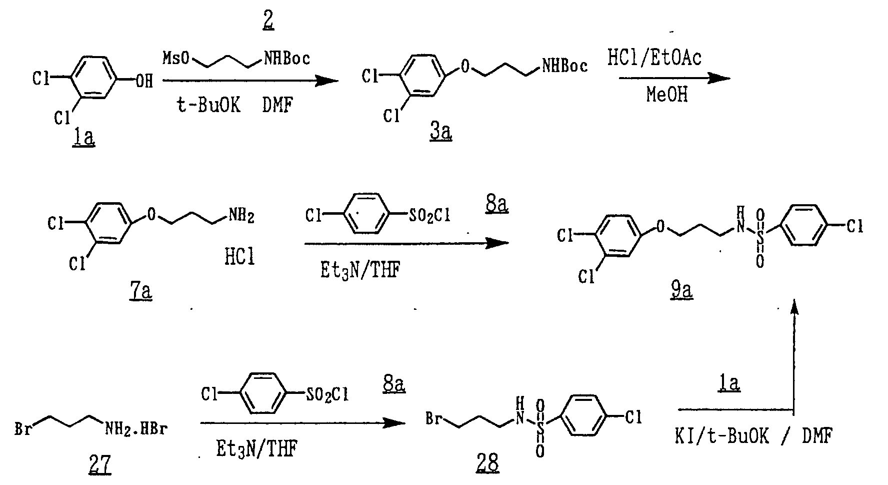

### (1) Preparation of 3a

### [3-(3,4-Dichloro-phenoxy)-propyl]-carbamic acid ter-butyl ester

DMF solution (30 mL) containing 3,4-dichloro-phenol (1a)(2.94g) and tBuOK (2.02g) was mixed at room temperature for 1.5 hours. To the solution were added (2) (4.8g) and mixed for 11 hours at 70°C. The reaction solution was poured to water and extracted with ethyl acetate. The organic layer was washed with water and saturated brine, dried over MgSO₄ and the solvent was evaporated under reduced pressure. The resultant oily product was purified by silica gel chromatography (toluene/ethyl acetate=25/1) to give (3a)(5.31g)(92%).
NMR(CDCl₃) δ ppm (300 MH_{z})
1.441(S,9H), 1.968(2H,quint.,J=6Hz), 3.308(2H,q,,J=6Hz), 3.980(2H,t,J=6Hz), 4.5∼4.75(1H,m), 6.745(1H,dd,J1=9Hz,J2=3Hz), 6.986(1H,d,J=3Hz), 7.311(1H,d,J=9Hz)

### (2) Preparation of 7a

### 3-(3,4-Dichloro-phenoxy)-propylamine HCl

(3a) (5.3g) was dissolved in methanol (30ml). Thereafter, 4N-HCl/ethyl acetate solution (35ml) was added. After the solution was mixed at room temperature for 2.5 hours, the solvent was evaporated to give white crystal (7a) (3.61g) (88%).
IR (Nujol) cm-1
3000,2923,2854,2788,2750,2613,2459,2069,1590,1566,1481,1467,1386,1296,12 32

### (3) Preparation of 28

### N-(3-Bromo-Propyl)-4-chloro-benzenesulfonamide

3-bromo-propylamine · HBr (27)10g, p-chlorobenzenesulfonyl chloride (8a)(10.1g) and triethylamine(16.2g) were dissolved in THF (150ml) and mixed at room temperature for 10 hours. The resultant reacted solution was poured to water and extracted with ethyl acetate. The organic layer was washed with water and saturated brine, dried over MgSO₄ and the solvent was evaporated under reduced pressure. The resultant oily product was purified by silica gel chromatography (toluene/ethyl acetate=20/1) to give the crude crystal (28) (13.9g)
(97%).
NMR(CDCl₃) δ ppm (300 MH_{z})
2.041(2H,quint.,J=6Hz), 3.135(2H,q,J=6Hz), 3.424(2H,t,J=6Hz), 4.90∼5.02(1H,m), 7.508(2H,d,J=9Hz), 7.821(2H,d,J=9Hz)

### (4) Preparation of 9a

### 4-Chloro-N-[3-(3,4-dichloro-phenoxy)-propyl]-benzenesulfonamide

### (Method A)

(7a)(2.56g), p-chlorobenzenesulfonyl chloride (8a)(2.24g) and triethylamine (2.53g) were dissolved in THF (30ml) and mixed at room temperature for 10 hours. The resultant reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=25/1) and recrystallized with ethyl acetate/ethyl ether/n-hexane to give white crystal(9a) (4.0g) (88%)(melting point;86.5∼87.5°C).

| Elementary analysis(%): C15H14Cl3NO3S | | | | | |
|---|---|---|---|---|---|
| Calcd. | C=45.65, | H=3.58, | N=3.55, | Cl=26.95, | S=8.12 |
| Found | C=45.66, | H=3.59, | N=3.66, | Cl=267.00, | S=8.20 |

NMR(CDCl₃)δ ppm (300 MH_{z}) 1.960(2H,quint.,J=6Hz), 3.189(2H,q,J=6Hz), 3.930(2H,t.J=6Hz), 4.70∼4.85(1H,m), 6.681(1H,dd,J1=9Hz,J2=3Hz), 6.915(1H,d,J=3Hz), 7.311(1H,d,J=9Hz), 7.451(2H,d,J=9Hz), 7.785(2H,d,J=9Hz)

### (Method B)

DMF solution (45ml) containing 3,4-dichlorophenol (la)(4.5 g) and tBuOK 3.1g was mixed at room temperature for 1.5 hours. (28)(9.15g) and KI (1.37g) were added and mixed at 100°C for 20hours. The reacted solution was poured to water and was extracted with ethyl acetate. The organic layer was washed with water and saturated brine, dried over MgSO₄. The solvent was evaporated under reduced pressure. The resultant oily product was purified by silica gel chromatography (toluenelethyl acetate=20/1) and recrystallized with ethyl acetate/ethyl ether/n-hexane to give (9a)(7.49g)(69%).

According to method A of Example 1(4), compound of example 2∼11 was prepared. However, in example 6 , p-chlorobenzoyl chloride (30) was used instead of p-chlorobenzene sulfonyl chloride (8a). The structural formulae were shown below.

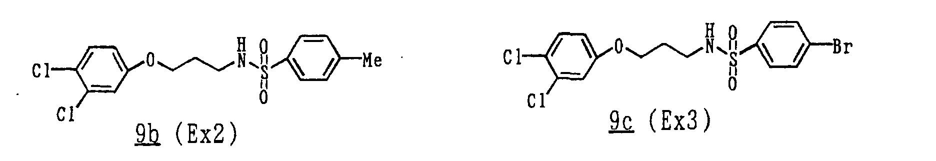

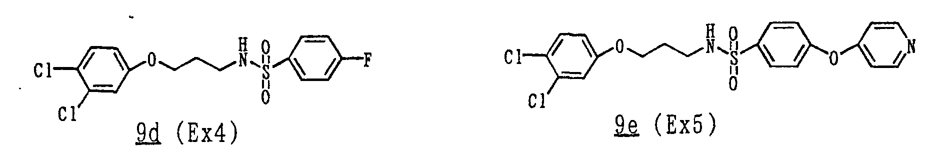

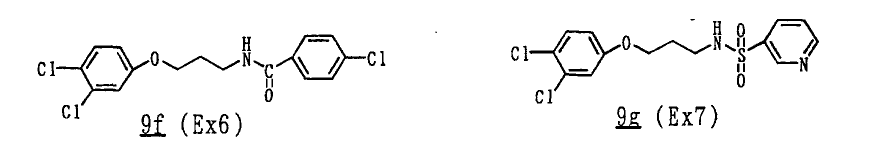

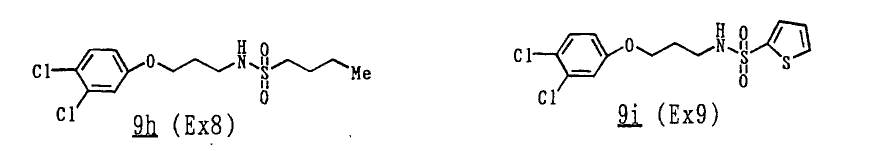

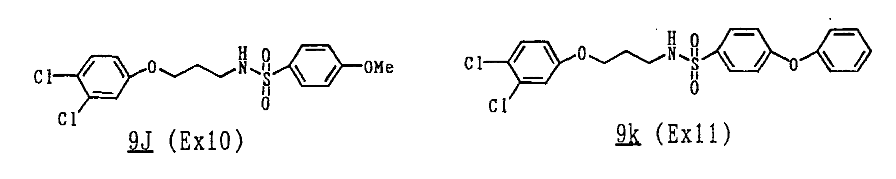

### Example 2

### Compound 9b

| Elementary analysis (%): C16H17Cl2NO3S | | | | | |
|---|---|---|---|---|---|
| Calcd. | C=51.35, | H=4.58, | N=3.74, | Cl=18.94, | S=8.57 |
| Found | C=51.25, | H=4.48, | N=3.90, | Cl=18,97, | S=8.46 |

Melting point: 117.0∼118.0°C
NMR(CDCl₃)δ ppm (300 MH_{z})
1.944(2H,quint.,J=7Hz), 2.406(3H,s), 3.167(2H,q,J=7Hz), 3.924(2H,t,J=7Hz), 4.60∼4.75(1H,m), 6.682(1H,dd,J1=9Hz,J2=3Hz), 6.895(1H,d,J=3Hz), 7.22∼7.36(3H,m), 7.728(2H,d,J=8Hz)

### Example 3

### Compound 9c

| Elementary analysis (%): C15H14BrCl2NO3S | | | | | | |
|---|---|---|---|---|---|---|
| Calcd. | C=41.03, | H=3.21, | N=3.19, | Br=18.20, | Cl=16.15, | S=7.30 |
| Found | C=40.99, | H=3.15, | N=3.29, | Br=18.21, | Cl=16.01, | S=7.25 |

Melting point: 96.0∼97.5°C
NMR(CDCl₃) δ ppm (300 MH_{z})
1.958(2H,quint.,J=6Hz), 3.188(2H,q,J=6Hz), 3.936(2H,t,J=6Hz), 4.75∼4.90(1H,m), 6.674(1H,dd,J1=9Hz,J2=3Hz), 6.916(1H,d,J=3Hz), 7.312(1H,d,J=9Hz), 7.613(2H,d,J=9Hz), 7.709(2H,d,J=9Hz)

### Example 4

### Compound 9d

| Elementary analysis (%): C15H14Cl2FNO3S | | | | | | |
|---|---|---|---|---|---|---|
| Calcd. | C=47.63, | H=3.73, | N=3.70, | Cl=18.75, | F=5.02, | S=8.48 |
| Found | C=47.58, | H=3.64, | N=3.86, | Cl=18.76, | F=4.96, | S=8.46 |

Melting point: 63.0∼64.0°C
NMR(CDCl₃) δ ppm (300 MH_{z})
1.954(2H,quint.,J=6Hz), 3.168(2H,q,J=6Hz), 3.936(2H,t,J=6Hz), 4.90∼5.05(1H,m), 6.680(1H,dd,J1=9Hz,J2=3Hz), 6.906(1H,d,J=3Hz), 7.10∼7.35(3H,m), 7.82∼7.94(2H,m)

### Example 5

### Compound 9e

| Elementary analysis (%): C20H18Cl2N2O4S.0.25H2O | | | | | |
|---|---|---|---|---|---|
| Calcd. | C=52.47, | H=4.07, | N=6.12, | Cl=15.49, | S=7.00 |
| Found | C=52.00, | H=3.76, | N=6.09, | Cl=15.91, | S=7.10 |

Melting point: 117.0∼117.5°C
NMR(CDCl₃) δ ppm (300 MH_{z})
1.993(2H,quint.,J=6Hz), 3.217(2H,q,J=6Hz), 3.964(2H,t,J=6Hz), 5.30∼5.40(1H,m), 6.693(1H,dd,J1=9Hz,J2=3Hz), 6.85∼7.00(3H,m), 7.85∼7.95(2H,m), 8.40∼8.70(2H,m)

### Example 6

### Compound 9f

| Elementary analysis (%): C16H14Cl3NO2 | | | | |
|---|---|---|---|---|
| Calcd. | C=53.58, | H=3.9.3, | N=3.91, | Cl=29.66, |
| Found | C=53.48, | H=3.79, | N=3.95, | Cl=29.59, |

Melting point: 117.5∼118.5°C
NMR(CDCl₃) δ ppm (300 MH_{z})
2.119(2H,quint.,J=6Hz), 3.650(2H,q,J=6Hz), 4.066(2H,t,6Hz), 6.40∼6.60(1H,m), 6.740(1H,dd,J1=9Hz,J2=3Hz), 6.983(1H,d,J=3Hz), 7.324(1H,d,9Hz), 7.406(2H,d,J=8Hz), 7.696(2H,d,J=8Hz)

### Example 7

### Compound 9g

| Elementary analysis (%): C14H14Cl2N2O3S | | | | | |
|---|---|---|---|---|---|
| Calcd. | C=46.55, | H=3.91, | N=7.75, | Cl=19.63, | S=8.89 |
| Found | C=46.42, | H=3.87, | N=7.67, | Cl=19.85, | S=9.00 |

Melting point: 114.0∼115.0°C
NMR(CDCl₃) δ ppm (300 MH_{z})
1.994(2H,quint.,J=6Hz), 3.241(2H,q,J=6Hz), 3.964(2H,t,J=6Hz), 5.00∼5.15(1H,m), 6.697(1H,dd,J1=9Hz,J2=3Hz), 6.915(1H,d,J=3Hz), 7.309(1H,d,J=9Hz), 7.40∼7.60(1H,m), 8.10∼8.20(1H,m), 8.70∼8.90(1H,m), 9.00∼9.20(1H,m)

### Example 8

### Compound 9h

NMR(CDCl₃) δ ppm (300 MH_{z})
0.928(2H,t,J=7Hz), 1.429(2H,sextet.,7Hz), 1.70∼1.84(2H,m), 2.047(2H,quint.,J=6Hz), 2.95∼3.05(2H,m), 1.327(2H,q,J=7Hz), 4.040(2H,t,J=6Hz), 4.45∼4.55(1H,m), 6.741(1H,dd,J1=9Hz,J2=3Hz), 6.994(1H,d,J=3Hz), 7.325(1H,d,J=9Hz)

### Example 9

### Compound 9i

| Elementary analysis (%): C13H13Cl2NO3S2 | | | | | |
|---|---|---|---|---|---|
| Calcd. | C=42.63, | H=3.58, | N=3.82, | Cl=19.36, | S=17.51 |
| Found | C=42.56, | H=3.49, | N=3.93, | Cl=19.32, | S=17.71 |

Melting point: 114.0∼115.0°C
NMR(CDCl₃) δ ppm (300 MH_{z})
1.999(2H,quint.,J=6Hz), 3.266(2H,q,J=6Hz), 3.977(2H,t,J=6Hz), 4.70∼4.83(1H,m), 6.714(1H,dd,J1=9Hz,J2=3Hz), 6.934(1H,d,J=3Hz), 7.05∼7.10(1H,m), 7.313(1H,d,J=9Hz), 7.55∼7.66(2H,m)

### Example 10

### Compound 9J

| Elementary analysis (%): C16H17Cl2NO4S | | | | | |
|---|---|---|---|---|---|
| Calcd. | C=49.24, | H=4.39, | N=3.59, | Cl=18.17, | S=8.21 |
| Found | C=49.30, | H=4.31, | N=3.69, | Cl=18.06, | S=8.40 |

Melting point: 94.5∼95.5°C
NMR(CDCl₃)δ ppm (300 MH_{z})
1.940(2H,quint.,J=6Hz), 3.155(2H,q,J=6Hz), 3.845(3H,s), 3.922(2H,t,J=6Hz), 4.65∼4.75(1H,m), 6.676(1H,dd,J1=9Hz,J2=3Hz), 6.88∼6.98(3H,m), 7.296(1H,d,J=9Hz), 7.776(2H,d,J=9Hz)

### Example 11

### Compound 9k

| Elementary analysis (%): C21H19Cl2NO4S | | | | | |
|---|---|---|---|---|---|
| Calcd. | C=55.76, | H=4.23, | N=3.10, | Cl=15.67, | S=7.09 |
| Found | C=55.73, | H=4.18, | N=3.16, | Cl=15.67, | S=7.22 |

Melting point: 87.0∼88.0°C
NMR(CDCl₃) δ ppm (300 MH_{z})
1.967(2H,quint.,J=6Hz), 3.173(2H,q,J=6Hz), 3.956(2H,t,J=6Hz), 4.70∼4 80(1H,m) 6.696(1H,dd,J1=9Hz,J2=3Hz), 6.928(1H,d,J=3Hz), 6.98∼7.85(10H,m)

### Example 12

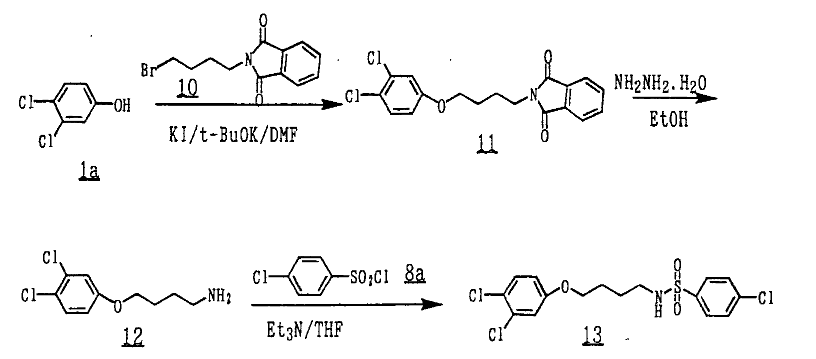

### (1) Preparation of 11

### N- [4-(3,4-Dichloro-phenoxy)-butyl]phthalimide

DMF solution (100ml) containing 3,4-dichloro-phenol(1a)(6g) and t-BuOK (4.1g) was mixed at room temperature for 1.5 hours. N-(4-bromobutyl) phtalimide (10)(11.1g) and KI(1.83g) were added and mixed at 95°C for 12hour. The reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=25/1) and washed with ethyl ether/n-hexane to give (11)(12.1g)(90%).
NMR(CDCl₃) δ ppm (300 MH_{z})
1.75∼1.98(4H,m), 3.765(2H,t,J=7Hz), 3.957(2H,t,J=6Hz), 6.723(1H,dd,J1=9Hz,J2=3Hz), 6.955(1H,d,J=3Hz), 7.283(1H,d,J=9Hz), 7.68∼7.90(4H,m)

### (2)Preparation of 12

### 4-(3,4-Dichloro-phenoxy)-butylamine

(11)(12g) was dissolved in EtOH (120ml). Thereafter, hydrazine monohydrate (3.3g) was added. The mixture was mixed for 2 hours under refluxing. Deposited crystal was filtrated and the filtrate was evaporated under reduced pressure to give (12)(5.89g)(76%) as oily product.
MMR(CDCl₃) δ ppm (300 MH_{z})
1.55∼2.00(6H,m), 2.781(2H,t,J=7Hz), 3.940(2H,t,J=7Hz), 6.740(1H,dd,J1=9Hz,J2=3Hz), 6.980(1H,d,J=3Hz), 7.306(1H,d,J=9Hz)

### (3)Preparation of 13

### 4-Chloro-N-[3-(3,4-dichloro-phenoxy)-butyl]-benzenesulfonamide

(12)(1.5g), p-chlorobenzenesulfonyl chloride (8a)(1.35g) and triethylamine (1.3g) were dissolved in THF (12ml) and mixed at room temperature for 10 hours. The resultant reaction solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=25/1) and recrystallized with ethyl ether/n-hexane to give (13) (2.5g)(98%).

| Elementary analysis (%): C16H16Cl3NO3S | | | | | |
|---|---|---|---|---|---|
| Calcd. | C=47.02, | H=3.95, | N=3.43, | Cl=26.02, | S=7.84 |
| Found | C=46.93, | H=3.90, | N=3.54, | Cl=25.99, | S=7.92 |

Melting point: 78.0∼79.0°C
NMR(CDCl₃) δ ppm (300 MH_{z})
1.60∼1.85(4H,m), 3.038(2H,q,J=6Hz), 3.889(2H,t,J=6Hz), 4.65∼4.75(1H,m), 6.698(1H,dd,J1=9Hz,J2=3Hz), 6.934(1H,d,J=3Hz), 7.308(1H,d,J=9Hz), 7.494(2H,d,J=9Hz), 7.808(2H,d,J=9Hz)

### Example 13

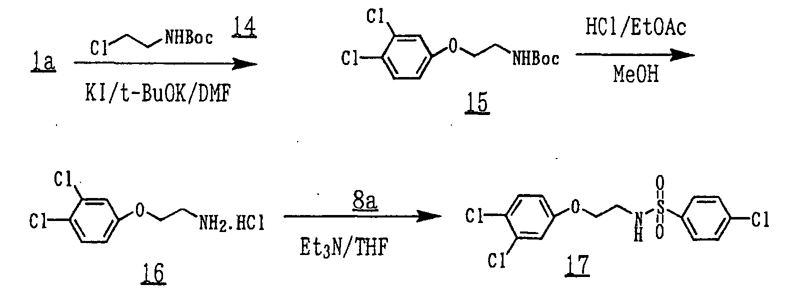

### (1) Preparation of 15

### [2-(3,4-Dichloro-phenoxy)-ethyl]-carbamic acid ter-butyl ester

DMF solution (35ml) containing 3,4-dichloro-phenol (1a)(5g) and t-BuOK (3.44g) was mixed at room temperature for 1.5 hours. To the solution were added 1-chloro-2-tert-butoxycarbonylamino ethyl ester(2a) (6.07g) and mixed at 95°C for 11 hours. The reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=25/1) to give (15)(9.1g)(97%) as oily product.
NMR(CDCl₃) δ ppm (300 MH_{z})
1.458(9H,s), 3.519(2H,q,J=5Hz), 3.979(2H,t,J=5Hz), 4.85∼5.05(1H,m), 6.70∼ 7.02(2H,m), 7.317(1H,d,J=9Hz)

### (2) Preparationn of 16

### 2-(3,4-Dichloro-phenoxy)-ethyl amine HCl

(15)(9.0 g) was dissolved in methanol (60ml). Thereafter, 4N-HCl/ethyl acetate solution (45ml) was added. After mixing reaction at room temperature for 2.5 hours, the solvent was evaporated to give white crystal (16) (5.4g) (75.8%).
IR (Nujol) cm-1 3220,2915,2777,2742,2688,2650,257982540,2508,2436,2044,1599,1570,1518,1 475,1466,1411,1379,1284

### (3) Preparation of 17

### 4-Chloro-N-[2-(3,4-dichloro-phenoxy)-ethyl]-benzenesulfonamide

(16)(1.0g), p-chlorobenzenesulfonyl chloride (8a)(0.92g) and triethylamine (1.12g) were dissolved in THF (20ml) and mixed at room temperature for 6 hours. The resultant reaction solution was poured to water and extracted with ethyl acetate. The organic layer was washed with water and saturated brine, dried over MgSO₄. The solvent was evaporated under reduced pressure. The resultant crystal was purified by silica gel chromatography (toluenelethyl acetate=20/1) and recrystallized with ethyl acetate/ethyl ether/n-hexane to give (17)(1.4g)(89%).

| Elementary analysis (%): C14H12Cl3NO3S | | | | | |
|---|---|---|---|---|---|
| Calcd. | C=44.17, | H=3.18, | N=3.68, | Cl=27.94, | S=8.32 |
| Found | C=44.06, | H=3.12, | N=3.83, | Cl=27.74, | S=8.55 |

Melting point: 115.0∼116.0°C
NMR(CDCl₃)δ ppm (300 MH_{z})
3.376(2H,q,J=5Hz), 3,964(2H,t,J=5Hz), 5,00∼5.10(1H,m), 6.647(1H,dd,J1=9Hz,J2=3Hz), 6.878(1H,d,J=3Hz), 7.303(1H,d,J=9Hz), 7.477(2H,d,J=9Hz), 7.814(2H,d,J=9Hz)

### Example 14

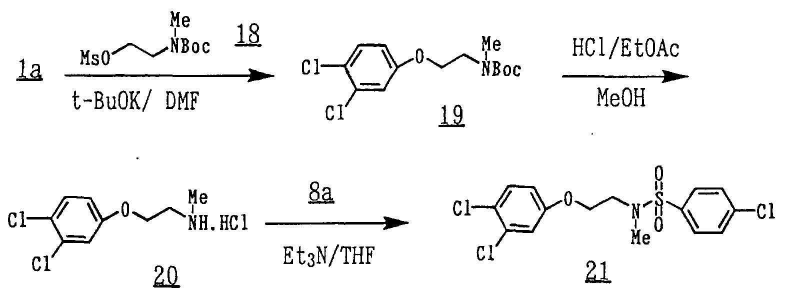

### (1)Preparation of 19;

### [2-(3,4-Dichloro-phenoxy)-ethyl)-methyl-carbamic acid tert-butyl ester

DMF solution (15ml) containing 3,4-dichlorophenol(la)(2.34g) and t-BuOK (1.62g) was mixed at room temperature for 1.5hours. To the solution were added (18) (4.0g) and mixed at 95°C for 12hours. The reacted solution was poured to water and extracted with ethyl acetate. The organic layer was washed with water and saturated brine, dried over MgSO₄. The solvent was evaporated under reduced pressure. The resultant oily product was purified by silica gel chromatography (toluenelethyl acetate=20/1) to give (19)(3.16g)(69%).
NMR(CDCl₃) δ ppm (300 MH_{z})
1.476(9H,s), 2.979(3H,s), 3.597(2H,t,J=6Hz), 4.043(2H,t,J=6Hz), 6.704(1H,dd,J1=9Hz,J2=3Hz), 6.976(1H,d,J=3Hz), 7.269(1H,d,J=9Hz)

### (2)Preparation of 20

### [2-(3,4-Dichloro-phenoxy)-ethyl]-methyl-amine HCl

(19)(3.0g) was dissolved in methanol (20ml). Thereafter, 4N-HCl/ ethyl acetate solution (25ml) was added. After the solution was mixed and reacted at room temperature 4 hours, the solvent was evaporated to give white crystal (20)(1.05g)(44%).
IR (Nujol) cm-1
3050,2925,2852,2702,2468,2420,1606,1591,1570, 1475,1463,1454,1396,1355,1280,1263,1229,

### (3) Preparation of 21

### 4-Chloro-N-[2-(3,4-dichloro-phenoxy)-ethyl]-N-methyl-benzenesulfonamide

(20)(0.95g), p-chlorobenzenesulfonyl chloride (8a)(0.82g) and triethylamine (1.31g) were dissolved in THF (20ml) and mixed at room temperature for 6 hours. The resultant reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=25/1) and recrystallized with ethyl ether/n-hexane to give (21) (1.2g)(82%).

| Elementary analysis (%): C15H14Cl3NO3S | | | | | |
|---|---|---|---|---|---|
| Calcd. | C=45.65, | H=3.58, | N=3.55, | Cl=26.95, | S=8.12 |
| Found | C=45.60, | H=3.50, | N=3.67, | Cl=26.78, | S=8.14 |

Melting point: 109.5∼110.5°C
NMR(CDCl₃) δ ppm (300 MH_{z})
2.925(3H,s), 3.451(2H,t,J=6Hz), 4.114(2H,t,J=6Hz), 6.697(1H,dd,J1=9Hz,J2=3Hz), 6.932(1H,d,J=3Hz), 7.322(1H,d,J=9Hz), 7.503(2H,d,J=9Hz), 7.748(2H,d,J=9Hz)

### Example 15

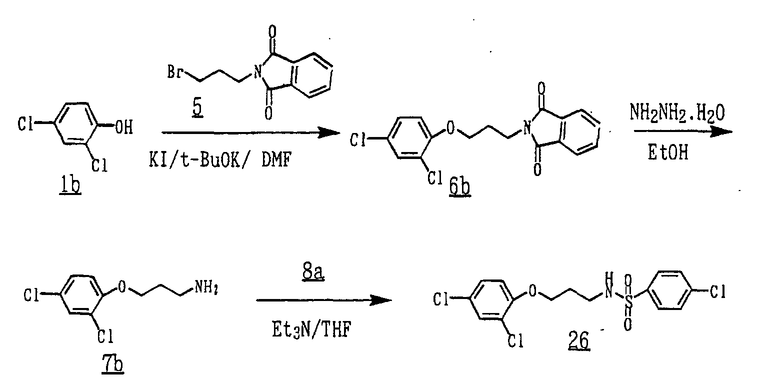

### (1)Preparation of 6b

### N-[3-(2,4-Dichloro-phenoxy)-propyl]phthalimide

DMF solution (60ml) containing 2,4-dichloro-phenol (1b)(6g) and t-BuOK (4.13g) was mixed at room temperature for 3 hours. N-(3-bromopropyl) phtalimide (5)(11.8g) and KI (1.83g) were added and mixed at 100°C for 10 hours. The reacted solution was poured to water and extracted with ethyl acetate. The organic layer was washed with water and saturated brine, dried over MgSO₄. The solvent was evaporated under reduced pressure. The resultant crude crystal was washed with ether to give (6b)(11.3g)(87%).
NMR(CDCl₃) δ ppm (300 MH_{z})
2.238(2H,quint.,J=6Hz), 3.943(2H,t,J=6Hz), 4.074(2H,t,J=6Hz), 6.816(1H,d,J=9Hz), 7.148(1H,dd,J1=9Hz,J2=3Hz), 7.300(1H,d,J=3Hz), 7.68∼7.90(4H,m)

### (2) Preparation of 7b

### 3-(2,4-Dichloro-phenoxy)-propylamine

(6b)(12g) was dissolved in EtOH (120ml). Thereafter, hydrazine monohydrate (3.43g) was added. The mixture was mixed under refluxing for 4 hours. The deposited crystal was filtrated and the filtrate was evaporated under reduced pressure to give (7b)(1.8g)(24%) as oily product.
NMR(CDCl₃) δ ppm (300 MH_{z})
1.30∼1.80(2H,m), 1.969(2H,quint.,J=6Hz), 2.949(2H,t,J=6Hz), 4.099(2H,t,J=6Hz), 6.850(1H,d,J=9Hz), 7.166(1H,dd,J1=9Hz,J2=3Hz), 7.353(1H,d,J=3Hz)

### (3)Preparation of 26

### 4-Chloro-N-[3-(2,4-dichloro-phenoxy)-propyl]-benzenesulfonamide

(7b) 0.8g, p-chlorobenzenesulfonyl chloride (8a) 0.81g and triethylamine (0.73g) were dissolved in THF (10ml) and mixed at room temperature for 4 hours. The resultant reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=10/1) and recrystallized with ethyl ether/n-hexane to give (26)(1.2g)(84%).

| Elementary analysis (%): C15H14Cl3NO3S | | | | | |
|---|---|---|---|---|---|
| Calcd. | C=45.65, | H=3.58, | N=3.55, | Cl=26.95, | S=8.12 |
| Found | C=45.70, | H=13.57, | N=13.58, | Cl=26.86, | S=7.92 |

Melting point: 108.0∼109.0°C
NMR(CDCl₃) δ ppm (300 MH_{z})
2.014(2H,quint.J=6Hz), 3.250(2H,q,J=6Hz), 4.023(2H,t,J=6Hz), 5.25∼5.40(1H,m), 6.759(1H,d,J=9Hz), 7.174(1H,dd,J1=9Hz,J2=3Hz), 7.375(1H,d,3Hz), 7.437(2H,d,J=9Hz), 7.797(2H,d,J=9Hz)

### Example 16

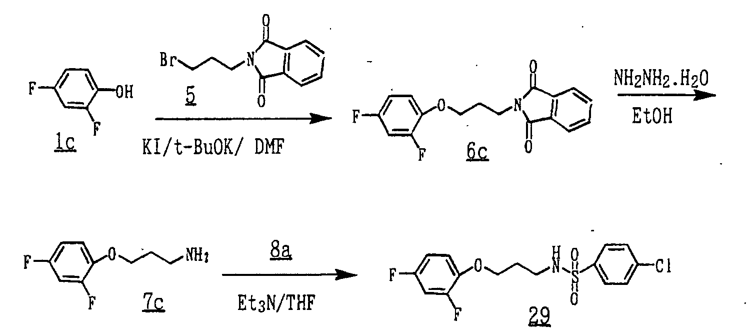

### (1) Preparation of 6c

### N-[3-(2,4-Difluoro-phenoxy)-propyl]phthalimide

DMF 65mlsolution containing 2,4-difluoro-phenol (lc)(6g) and tBuOK (5.17g) was mixed at room temperature for 1 hours. N-(3-bromopropyl) phtalimide (5)(13.6g) and KI (1.53g) were added and mixed at 95°C for 11 hours. The reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=10/1) to give (6c)(13.2g)(90%).
NMR(CDCl₃)δ ppm (300 MH_{z})
2,196(2H,quint.,J=6Hz), 3.923(2H,t,J=6Hz), 4.062(2H,t,J=6Hz), 6.70∼6.95(3H,m), 7.65∼7.90(4H,m)

### (2) Preparation of 7c;

### 3-(2,4-Difluoro-phenoxy)-propylamine

(6c)(13.2g) was dissolved in EtOH (130ml). Thereafter, hydrazine monohydrate (4.16g) was added. The mixture was mixed 4 hours under refluxing. The deposited crystal was filtrated and the filtrate was evaporated under reduced pressure to give (7c) (6.0g) (77%) as oily product.
NMR(CDCl₃) δ ppm (300 MH_{z})
1.90∼2.10(2H,m), 1.959(2H,quint.,J=6Hz), 2.047(2H,t,J=6Hz), 4.004(2H,t,J=6Hz), 6.70∼6.97(3H,m)

### (3) Preparation of 29

### 4-Chloro-N-[3-(2,4-difluoro-phenoxy)-propyl]-benzenesulfonamide

(7c)(1.5g), p-chlorobenzenesulfonyl chloride (8a)(1.78g) and triethylamine (1.62g) were dissolved in THF (25ml) and mixed at room temperature for 10 hours. The resultant reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=25/1) and recrystallized with ethyl ether/n-hexane to give (29) 2.3g (79%).

| Elementary analysis (%): C15H14ClF2NO3S | | | | | | |
|---|---|---|---|---|---|---|
| Calcd. | C=49.80, | H=3.90, | N=3,87, | Cl=9.80, | F=10.50, | S=8.86 |
| Found | C=49.90, | H=4.013, | N=3.87, | Cl=9.85, | F=10.52, | S=8.84 |

Melting point: 98.0∼99.0°C
NMR(CDCl₃)δ ppm (300 MH_{z})
1.971(2H,quint.,J=6Hz), 3.222(2H,q,J=6Hz), 4.016(2H,t,6Hz), 4.95∼5.05(1H,m), 6.70∼6.90(3H,m), 7.803(2H,d,J=9Hz)

### Example 17

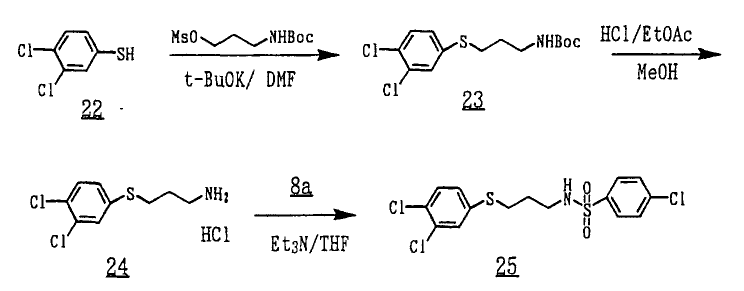

### (1) Preparation of 23

### [3-(3,4-Dichloro-phenylsulfanyl)-propyl]-carbamic acid tert-butyl ester

DMF (17ml) solution containing 3,4-dichloro-benzenethiol (22)(1.48g) and tBuOK (0.93g) was mixed at room temperature for 1 hours. To the solution was added (2) (2.3g) and mixed at 95°C for 10 hours. The reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate=25/1) to give (23) (2.48g) (88%).
NMR(CDCl₃) δ ppm (300 MH_{z})
1.442(9H,s), 1.819(2H,quint.,J=7Hz), 2.933(2H,t,J=7Hz), 3.240(2H,q,J=7Hz), 4.50∼4.70(1H,m), 7.135(1H,dd,J1=8Hz,J2=2Hz), 7.335(1H,d,J=8Hz), 7.384(1H,d,J=2Hz)

### (2) Preparation of 24

### 3-(3,4-Dichloro-phenylsulfanyl) propylamine HCl

(23)(2.38g) was dissolved in methanol (15ml). Thereafter, 4N-HCl/ethyl acetate solution (15ml) was added. After the solution was mixed and reacted at room temperature for 2.5 hours, the solvent was evaporated to give white crystal (24)(1.5g)(78%).
IR (Nujol) cm-1
2956,2924,2854,2409.2058,1707,1604,1572,1545,1483,1462,1431,1408,1371,12 67,

### (3) Preparation of 25

### 4-Chloro-N-[3-(3,4-dichloro-phenylsulfanyl)-propyl]-benzenesulfonamide

(24) 1.0g, p-chlorobenzenesulfonyl chloride (8a)(0.82g) and triethylamine (1.3g) were dissolved in THF(15ml) and mixed at room temperature for 10 hours. The resultant reacted solution was poured to water and extracted with ethyl acetate. The organic layer was washed with water and saturated brine, dried over MgSO₄. The solvent was evaporated under reduced pressure. The resultant oily product was purified by silica gel chromatography (toluenelethyl acetate=20/1) and recrystallized with ethyl ether/n-hexane to give (25)(1.3g)(87%).

| Elementary analysis (%): C15H14Cl3NO2S2 | | | | | |
|---|---|---|---|---|---|
| Calcd. | C=43.86, | H=3.44, | N=3.41, | Cl=25.89, | S=15.61 |
| Found | C=43.87, | H=3.29, | N=3.49, | Cl=25.80, | S=15.71 |

Melting point: 84.0∼85.0°C
NMR(CDCl₃) δ ppm (300 MH_{z})
1.805(2H,quint.,J=7Hz), 2.918(2H,t,J=7Hz), 3.099(2H,q,J=7Hz), 4.65∼4.80(1H,m), 7.094(1H,dd,J1=9Hz,J2=3Hz), 7.30∼7.38(2H,m), 7.481(2H,d,J=9Hz), 7.784(2H,d,J=9Hz)
The reaction scheme of example 18 and 19 was shown below.

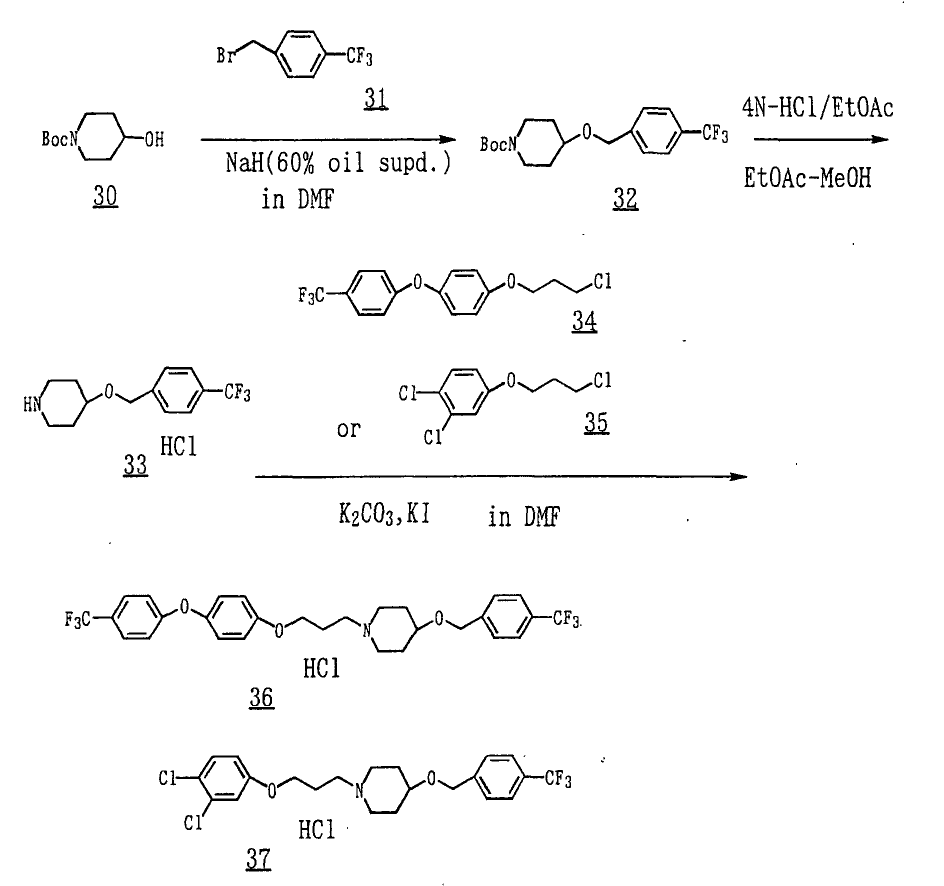

### Example 18

### (1) Preparation of 32 : 4-(4-Trifluoromethyl-benzyloxy)-piperidine-1-carboxylic acid tert-butyl ester

DMF solution (35ml) containing compound (30)(5g) and NaH (60% oil suspend.) was mixed in at room temperature for 1.5 hours. To the solution was added compound (31)(7.17g) and mixed at 50°C for 13 hours. The reacted solution was poured to water and extracted with ethyl acetate. The organic layer was washed with water and saturated brine, dried over MgSO₄. The solvent was evaporated under reduced pressure. The resultant oily product was purified by silica gel chromatography (toluene/ethyl acetate = 25/1∼10/1) to give compound(32)(8.35g)(94%).
NMR (CDCl₃) δ ppm (300 MH_{z})
1.460(9H,s), 1.50∼1.90(4H,m), 3.06∼3.86(5H,m), 4.608(2H,s), 7.459(2H,d,J=8Hz), 7.603(2H,d,J=8Hz)

### (2) Preparation of 33 : 4-(4-Trifluoromethyl-benzyloxy)-piperidine

Compound (32) (8.35g) was dissolved in ethyl acetate (10ml) and methanol (30ml). Thereafter, 4N-HCl ethyl acetate solution (8.7ml) was added and mixed at room temperature for 1.5 hours. The solvent was evaporated to give crude corresponding hydrochloride(33)(5.72g).

### (3) Preparation of 36 : 4-(4-Trifluoromethyl-benzyloxy)-1-{3-[4-(4-trifluoromethyl-phenoxy)-phenoxy]-propyl}-piperidine

Compound(33)(1.28g), (34)(1.5g), potassium carbonate(1.19g) and potassium iodide (0.36g) were added in DMF (15ml) and mixed at 100°C for 18 hours. The reacted solution was poured to water and extracted with ethyl acetate. The organic layer was washed with water and saturated brine, dried over MgSO₄. The solvent was evaporated under reduced pressure. The resultant oily product was purified by silica gel chromatography (chloroform/methanol=30/1) to give the free form of compound (36)(2.26g, 90%). This correspoding hydrochloride was produced by 4N-HCl ethyl acetate solution and recrystallized with methanol and 2-propanol to give the corresponding hydrochloride (36).

| Elementary analysis (%):C29H29F6NO3.HCl (corresponding hydrochloride) | | | | | |
|---|---|---|---|---|---|
| Calcd. | C=59.04, | H=5.13, | N=2.37, | Cl=6.01, | F=19.32 |
| Found | C=58.99, | H=5.07, | N=2.51, | Cl=5.83, | F=19.12 |

NMR (CDCl₃)δ ppm (300 MH_{z})
1.65∼2.25(8H,m), 2.528(2H,t,J=7Hz), 2.752.86(2H,m), 3.403.50(1H,m), 4.017(2H,t,J=6Hz), 4.601(2H,s), 6.88∼7.64(12H,m)

### Example 19

### Preparation of 37 : 1-(3,4-Dichlorophenyloxy-propyl)-4-(4-trifluoromethyl-benzyloxy)-piperidine

Compound(33)(1.30g), (35)(1.35g), potassium carbonate (1.22g) and potassium iodide (0.37g) were added to DMF(15mL) and the solution was mixed at 100°C for 18 hours. The reacted solution was poured to water and extracted with ethyl acetate. The organic layer was washed with water and saturated brine, dried over MgSO₄. The solvent was evaporated under reduced pressure. The resultant oily product was purified by silica gel chromatography (chloroform/methanol = 25/1) to give free form of (37) (1.98g(%)). This correspoding hydrochloride was produced by 4N-HCl ethyl acetate solution and recrystallized with 2-propanol-ethyl ether to give the corresponding hydrochloride (36).

| Elementary analysis(%):C₂₂H₂₄Cl₂F₃NO₂ • HCl (corresponding hydrochloride) | | | | | |
|---|---|---|---|---|---|
| Calcd. | C=52.98, | H=5.05, | N=2.81, | Cl=21.32, | F=11.43 |
| Found | C=52.73, | H=4.94, | N=2.90, | Cl=21.22, | F=11.35 |

NMR (CDCl₃) δ ppm (300 MH_{z})
1.960(2H,quint.,J=6Hz), 3.189(2H,q.J=6Hz), 3.930(2H,t.J=6Hz), 4.70∼4.85(1H,m), 6.681(1H,dd,J1=9Hz J2=3Hz), 6.915(1H,d,J=3Hz), 7.311(1H,d,J=9Hz), 7.451(2H,d,J=9Hz), 7.785(2H,d,J-9Hz)

### Example 20

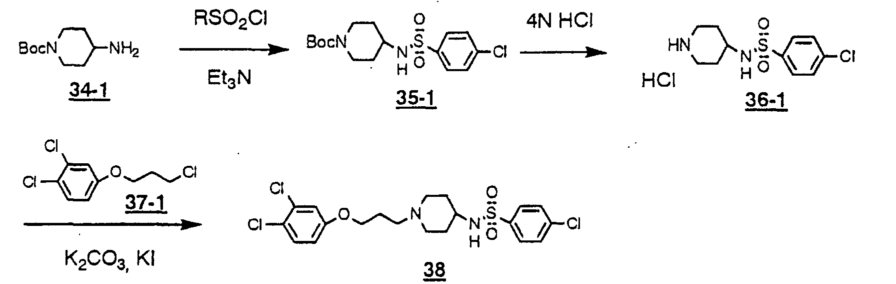

### (1) Preparation of 35 : 4 -(4-Chloro-benzenesulfonylamino)-piperidine-1-carboxylic acid tert-butyl ester

Compound (34-1) (2.50g), p-chlorobenzenesulfonyl chloride (2.89g), triethylamine (3.30ml) were dissolved in tetrahydrofuran (37ml) and mixed at room temperature for 16hours. The resultant reacted solution was poured to saturated aqueous sodium bicarbonate and extracted with chloroform. The organic layer was washed with water and saturated brine, dried over MgSO₄ and the solvent was evaporated under reduced pressure. The resultant oily product was purified by silica gel chromatography (hexane: ethyl acetate=3:1) to give non coloress oily product (35-1) (4.71g(quant) ).
NMR (CDCl₃)δ ppm (300 MH_{z})
1.430(9H, s), 1.200 ∼ 1.600(2H, m), 1.700 ∼ 1.820(2H, m), 2.700 ∼ 2.850(2H, m), 3.240 ∼ 3.370(1H, m), 3.850 ∼ 4.00(2H, m), 4.462(1H, d, *J =* 8Hz), 7.495(2H, d, *J =* 9Hz), 7.818(2H, d, *J =* 9Hz)

### (2) Preparation of 36-1: 4 -Chloro-N-piperidin-4-yl-benzenesulfonamide hydrochloride

Compound (35-1)(4.71g) was dissolved in methanol (25ml). Thereafter, 4N-HCl/ethyl acetate solution (6.28ml) was added. After the solution was mixed and reacted at room temperature for 4hours, the solvent was evaporated to give white crystal (2.80g) (36-1) (72%).

### (3) Preparation of 38: 4 -Chloro-N-{1-[3-(3,4-dichloro-phenoxy)-propyl]-piperidin-4-yl}-benzenesulfonamide

To DMF solution (21ml) containing compound(37-1)(1.39g) and (36-1)(1.50g) were added potassium carbonate (1.33g) and iodide potassium (400mg) and mixed at 85°C for 8hours. After DMF was evaporated, saturated aqueous sodium bicarbonate was poured to the residue, extracted with ethyl acetate. The organic layer was washed with water and saturated brine, dried over MgSO₄. The solvent was evaporated under reduced pressure. The resultant oily product was purified by silica gel chromatography (chloroform:methanol=15:1) to give colorless oily product(38)(1.04g)(45%).

### Example 21

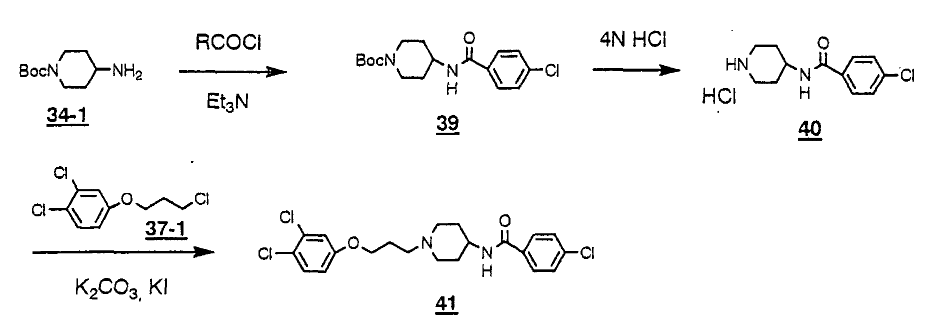

### (1) Preparation of 39: 4 -{[1-(4-Chloro-phenyl)-methanoyl]-amino}-piperidine-1-carboxylic acid tert-butyl ester

Compound (34-1) (1.92g), p-chlorobenzoyl chloride (1.34g), triethylamine (2.56ml) was dissolved in tetrahydrofuran (30ml) and mixed at room temperature for 6 hours. The resultant reacted solution was poured to saturated aqueous potassium carbonate and extracted with chloroform. The organic layer was washed with water and saturated brine, dried over MgSO₄. The solvent was evaporated under reduced pressure. The resultant oily product was purified by silica gel chromatography (hexane: ethyl acetate=3:1) to give the white crystal (39)(2.97g) (91%).
NMR (CDCl₃) δ ppm (300 MH_{z})
1.400 ∼ 1.470(2H, m), 1.467(9H, s), 1.980 ∼ 2.080(2H, m), 2.850 ∼ 2.980(2H, m), 4.030∼ 4.200(3H, m), 5.939(1H, br-s), 7.411(2H, d, *J* = 8Hz), 7.696(2H, d, *J* = 8Hz)

### (2) Preparation of 40: 4 -Chloro-N-piperidin-4-yl-benzamide hydrochloride

Compound (39) (2.97g) was dissolved in methanol (15ml). Thereafter, 4N-HCl/ethyl acetate solution (4.38ml) was added. After the solution was mixed and reacted at room temperature for 16 hours, the solvent was evaporated to give white crystal (40)(2.30g)(95%).

### (3) Preparation of 41: 4 -Chloro-N-{ 1 -[3 -(3, 4-dichloro-phenoxy)-propyl]-piperidin-4-yl}-benzamide

To DMF solution (20 ml) containing compound(37-1)(1.54g) and (40)(1.47g) were added potassium carbonate (1.48g) and potassium iodide (444mg) and mixed at 85°C for 8hours. After DMF was evaporated, saturated potassium carbonate solution was poured to the residue, extracted with ethyl acetate. The organic layer was washed with water and saturated brine, dried over MgSO₄. The solvent was evaporated under reduced pressure. The resultant oily product was purified by silica gel chromatography (chloroform:methanol=20:1) to give white crystal(41)(2.03g)(86%).
NMR (CDCl₃) δ ppm (300 MH_{z})
1.540 ∼ 1.690(2H, m), 1.988(2H, quint, *J* = 7.8Hz),2.020 ∼ 2.110(2H, m), 2.160 ∼ 2.280(2H, m), 2.558(2H, t, *J* = 7.2Hz), 2.900 ∼ 3.000(2H, m), 3.993(2H, t, *J* = 6Hz), 3.900 ∼ 4.200(1H, m), 5.900 ∼ 6.000(1H, m), 6.750(1H, dd, *J* = 9Hz, 3Hz), 7.001(1H, d, *J* = 3Hz), 7.310(1H, d, *J* = 9Hz), 7.407(2H, d, *J =* 9Hz), 7.695(2H, d, *J=* 8Hz)

### Example 22

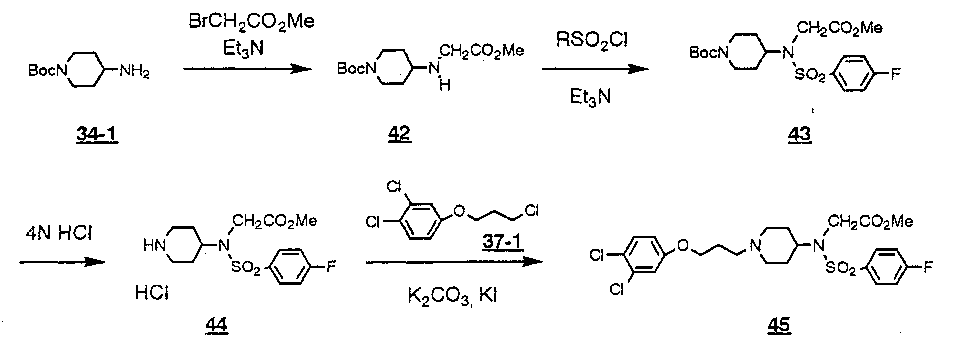

### (1)Preparation of 42: 4 -(Methoxycarbonylmethyl-amino)-piperidine-1-carboxylic acid tert-butyl ester

Compound (34-1) (2.17g), bromomethyl acetate (1.33g) and triethylamine (2.27ml) were dissolved in tetrahydrofuran (36ml) and mixed at room temperature for 16 hours. The insoluble product was removed by kiriyama funnel from the reacted solution and the solvent was evaporated from resultant filtrate under reduced pressure. The resultant oily product was purified by silica gel chromatography (chloroform:methanol=20:1) to give coloress oily product (42) 3.01g(quant).
NMR(CDCl₃) δ ppm (300 MH_{z})
1.20 ∼ 1.36(2H, m), 1.452(9H, s), 1.75 ∼ 1.87(2H, m), 2.56 ∼ 2.68(1H, m), 2.70 ∼ 2.90(2H, m), 3.462(2H, s), 3.741(3H,s), 3.94 ∼ 4.15(2H, m)

### (2) Preparation of 43:4-[(4 -Fluoro-but- 2 -ene- 1 -sulfonyl)-methoxycarbonylmethyl-amino]-piperidine- 1 -carboxylic acid tert-butyl ester

Compound (42) (1.50g), p-fluorobenzenesulfonyl chloride (1.29g), triethylamine (1.46ml) and 4-dimethyl aminopyridine (202mg) were dissolved in acetonitrile (28ml) and mixed at room temperature for 16 hours. The resultant reacted solution was washed with 10% oxalic acid solution and saturated aqueous potassium carbonate. The organic layer was washed with saturated brine, dried over MgSO₄. The solvent was evaporated under reduced. pressure. The resultant oily product was purified silica gel chromatography (hexane: ethyl acetate=3:1) to give (43) (2.12g) (89%) colorless oily product.
NMR (CDCl₃)δ ppm (300 MH_{z})
1.30 ∼ 1.48(1H, m), 1.432(9H, s), 1.60 ∼ 1.72(3H, m), 2.54 ∼ 2.74(2H, m), 3.56 ∼ 3.70(1H, m), 3.734(3H, s), 4.037(2H, s), 4.06 ∼ 4.20(2H, m), 7.199(2H, t, *J =* 9Hz), 8.017(2H, dd, *J =* 9Hz, 5Hz)

### (3) Preparation of 44:[(4-Fluoro-benzenesulfonyl)-piperidin-4-yl-amino]-acetic acid methyl ester hydrochloride

Compound (43)(1.61g) was dissolved in methanol (8.1ml). Thereafter, to the solution was added 4N-HCl/ethyl acetate solution (1.87ml). After the solution was mixed and reacted at room temperature for 16hours, the solvent was evaporated to give the white crystal (44)(1.10g)(80%).

### (4) Preparation of 45:[{1-[3-(3,4-Dichloro-phenoxy)-propyl]-piperidin-4 -yl}-(4-fluoro-benzene sulfonyl)-amino]-acetic acid methyl ester

To DMF solution (16ml) containing compound (37-1) (863mg) and (44) (1.10g) were added potassium carbonate (828mg) and potassium iodide (249mg) and mixed at 85°C for 16hours. After DMF was evaporated, to the residue was poured saturated aqueous potassium carbonate and extracted with ethyl acetate. The organic layer washed with water and saturated brine, dried over MgSO₄. The solvent was evaporated under reduced pressure. The resultant oily product was purified by silica gel chromatography (chloroform:methanol=30:1) to give the colorless oily product (45)(1.81g)(quant).

| Elementary analysis (%) : C23H27Cl2FN2O5S • 0.1(EtOH) • HCl | | | | | | |
|---|---|---|---|---|---|---|
| Calcd. | C =48.47, | H =4.95, | N =4.92, | Cl =18.66, | F =3.33, | S=5.63 |
| Found | C =48.50, | H =5.02, | N =4.88, | Cl =18.51, | F =3.31, | S =5.58 |

NMR (CDCl₃) δ ppm (300 MH_{z})
1.480 ∼ 1.800(4H, m), 1.84 ∼ 2.02(4H, m), 2.443(2H, t, *J* = 7Hz), 2.850 ∼ 2.970(2H,m), 3.45 ∼ 3.58(1H,m), 3.725(1H, s), 3.936(2H, t, *J* = 6Hz), 4.080(2H, s), 6.723(1H, dd, *J* = 9Hz, 3Hz), 6.973(1H, d, *J* = 3Hz), 7.12 ∼ 7.27(3H, m), 7.296(1H, d, *J* = 9Hz), 8.016(1H, dd, *J* = 9Hz, 5Hz)

### Example 23

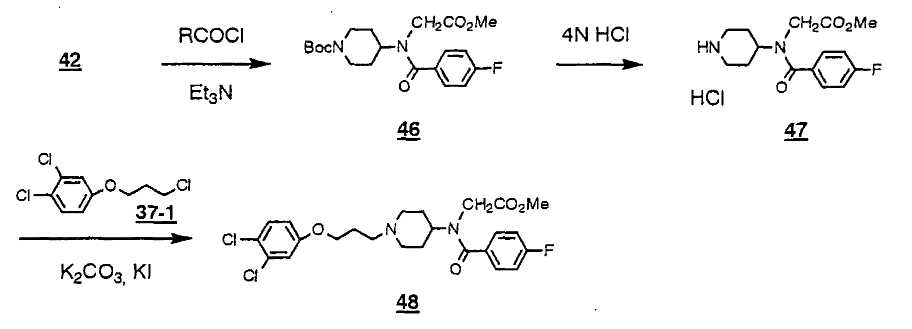

### (1) Preparation of 46: 4-{[1-( 4 -Fluoro-phenyl)-methanoyl]-methoxy carbonyl methyl-amino}-piperidine- 1 -carboxylic acid tert-butyl ester

Compound (42) (1.51g), p-fluorobenzoyl chloride (802 ml), triethyl amine (1.47ml) and 4-dimethyl aminopiridine (135mg) were dissolved in dichloromethane (28ml) and mixed at room temperature for 16 hours. The resultant reacted solution was washed with 10% oxalic acid solution, and thereafter washed with saturated aqueous carbonate potassium. The organic layer was washed with saturated brine, dried over MgSO₄ and the solvent was evaporated under reduced pressure. The resultant oily product was purified by silica gel chromatography (hexane: ethyl acetate=2:1) to give amorphous (46)(1.64g)(75%).
NMR (CDCl₃) δ ppm (300 MH_{z})
1.428(9H, s), 1.54∼1.88(4H, m), 2.42 ∼ 2.60(1H, m), 3.60 ∼ 3.80 (5H, m), 3.98∼4.22(4H, m), 7.10 ∼ 7.30(2H, m), 7.36 ∼ 7.52(2H, m)

### (2) Preparation of 47:{[1-(4-Fluoro-phenyl)-methanoyl]-piperidine-4-yl-amino}-acetic acid methyl ester hydrochloride

Compound (46) (1.64g) was dissolved in methanol (14ml). Thereafter, 4-N HCl/ethyl acetate solution (2.08ml) was added. After the solution was mixed and reacted at room temperature for 16 hours, the solvent was evaporated to give the crude corresponding hydrochloride (47)(1.34g)(97%).

### (3) Preparation of 48:{{1-[3-(3,4-Dichloro-phenoxy)-propyl]-piperidine-4-yl}-[1-(4-fluoro-phenyl)-methanoyl]-amino}-acetic acid methyl ester

To DMF solution (20ml) containing compound (37-1) (1.17g) and (47)(1.34g) were added potassium carbonate (1.12g) and potassium iodide (336mg) and mixed at 85°C for 40hours. After DMF solution was evaporated, to the residue was poured saturated aqueous potassium carbonate and extracted with ethyl acetate. The organic layer was washed with water and saturated brine, dried over MgSO₄ and the solvent was evaporated under reduced pressure. The resultant oily product was purified by silica gel chromatography (chloroform:methanol=50:1) to give the colorless oily product (48)(1.78g)(88%) and moreover corresponding hydrochloride.

| Elementary analysis (%):C24H27Cl2FN2O4 HCl(corresponding hydrochloride) | | | | | |
|---|---|---|---|---|---|
| Calcd. | C =54.00, | H =5.29, | N =5.25, | Cl =19.92, | F =3.56 |
| Found | C =53.75,H | H =5.30, | N =5,35,Cl | Cl =19.65, | F =3.39 |

NMR (CDCl₃) δ ppm (300 MH_{z})
1.500 ∼ 1.780(4H, m), 1.830 ∼ 2.000(4H, m), 2.400 ∼ 2.500(2H, m), 2.840 ∼ 2.960(2H, m), 3.450 ∼ 3.580(1H, m), 3.725(3H, s), 3.936(2H, t, J= 6Hz), 4.080(2H,s), 6.723(1H, dd, *J* = 9Hz, 3Hz), 6.973(1H, d, *J* = 3Hz), 7.120 ∼ 7.240(2H, m), 7.296(1H, d, *J* = 9Hz), 7.980 ∼ 8.500(2H, m)

### Example 24

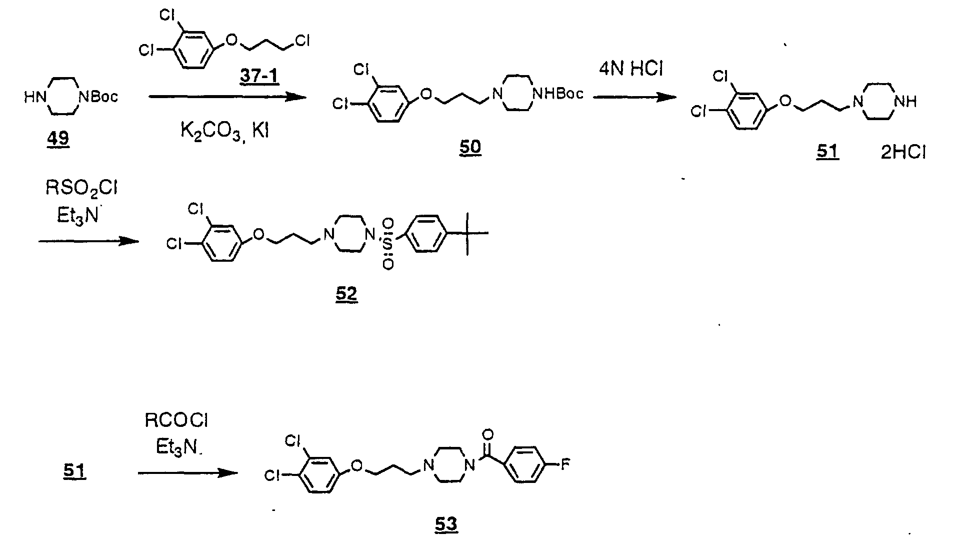

### (1) Preparation of 50:1-[3-(3,4-Dichloro-phenoxy)-propyl]-piperidine

To DMF (20ml) solution containing compound (37-1) 2.49g and tert-butyl 1-piperadin-carboxyrate (49) (1.29g) were added potassium carbonate (957mg) and potassium iodide (575mg) and the solution and mixed at 85°C for 11 hours. After DMF was evaporated, to the residue was poured saturated aqueous potassium carbonate and extracted with ethyl acetate. The organic layer was washed with water and saturated brine, dried over MgSO₄ and the solvent was evaporated under reduced pressure. The resultant oily product was purified by silica gel chromatography (chloroform:methanol=50:1) to give yellow oily product (50)(2.15g)(79%).
NMR (CDCl₃) δ ppm (300 MH_{z})
1.462(9H, s), 1.90 ∼ 2.00(2H, m), 2.381 ∼ 2.414 (4H, m), 2.504(2H, t, *J =* 7.2Hz), 3.419 ∼ 3.453(4H, m), 3.990(2H, t, *J =* 6.6Hz), 6.751(1H, dd, *J =* 9Hz, 3Hz), 6.998(1H, d, *J =* 3Hz), 7.308(1H, d, *J =* 9Hz)

### (2)Preparation of 51: 1-[3-(3,4-Dichloro-phenoxy)-propyl]-piperadine hydrochloride

Compound (50)(2.15g) was dissolved in methanol (22ml). Thereafter, 4N-HCl/ ethyl acetate solution (2.75ml) was added. After the solution was mixed and reacted at room temperature for 3 days, the solvent was evaporated to give white crystal (51) (1.67g) (84%).

### (3) Preparation of 52: 1-(4-tert-Butyl-benzenesulfonyl)-4-[3-(3,4-dichlorophenoxy)-propyl]-piperadine

Compound (51)(854mg), 4-tert buthylbenzenesulfonyl chloride (616mg) and triethylamine (627ml) were dissolved in dichloromethane (13ml) and mixed at room temperature for 16 hours. The resultant reacted solution was poured to saturated aqueous potassium carbonate and extracted with chloroform. The organic layer was washed with water and saturated brine, dried over MgSO₄ and the solvent was evaporated under reduced pressure. The resultant oily product was purified by silica gel chromatography (hexane: ethyl acetate=3:1) to give white crystal (52)(1.01g)(88%).
NMR (CDCl₃) δ ppm (300 MH_{z})
1.346(9H, s), 1.887(2H, quint, 7Hz), 2.502(2H, t, *J* = 7Hz), 2.520 ∼ 2.600(4H, m), 3.000 ∼ 3.100(4H, m), 3.920(2H, t, *J =* 7Hz), 6.704(1H, dd, *J =* 9Hz, 3Hz), 6.951(1H, d, *J =* 3Hz), 7.279 (1H, d, *J =* 9Hz), 7.531(2H, d, *J =* 8Hz), 7.676(2H, d, *J =* 8Hz)

### (4) Preparation of 53: 1-{4-[3-(3,4-Dichloro-phenoxy)-propyl]-piperadine-1-yl}-1-(4-fluoro-phenyl)-methanone

Compound (51) (817mg), 4-fluorobenzoyl chloride (320ml) and triethylamine (899ml) were dissolved in dichloromethane (12ml) and mixed at room temperature for 4 days. The resultant reacted solution was poured to saturated aqueous potassium carbonate and extracted with chloroform. The organic layer was washed with water and saturated brine, dried over MgSO₄ and the solvent was evaporated under reduced pressure. The resultant oily product was purified by silica gel chromatography (chloroform:methanol=40:1) to give colorless oily product (53)(1.14g) (quant) and moreover corresponding hydrochloride.

| Elementary analysis (%): C20H21Cl2FN2O2 HCl (corresponding hydrochloride) | | | | | |
|---|---|---|---|---|---|
| Calcd. | C =53.65, | H =4.95, | N =6.26, | Cl =23.75, | F =4.24 |
| Found | C =53.44, | H =4.82, | N =6.27, | Cl =23.49, | F =4.12 |

NMR (CDCl₃) δ ppm (300 MH_{z})
1.540 ∼ 1.800(2H, m), 1.973(2H, quint, 6Hz), 2.300 ∼ 2.620(4H, m), 3.400 ∼ 3.880(4H, m), 3.999(2H, t, *J* = 6Hz), 6.746(1H, dd, *J* = 9Hz, 3Hz), 6.995(1H, d, *J* = 3Hz), 7.098(2H, dd, *J* = 9Hz, 9Hz), 7.310(1H, d, *J* = 9Hz), 7.419(2H, dd, *J* = 9Hz, 5.4Hz)

### Example 25

According to above examples, below compound and/or the corresponding salt were prepared.

### Compound No.

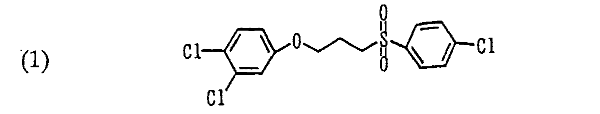

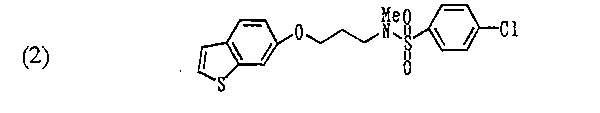

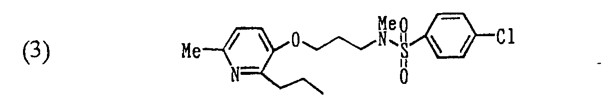

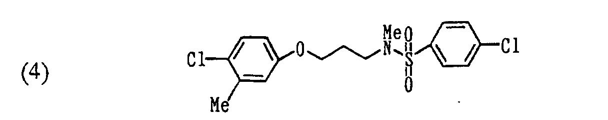

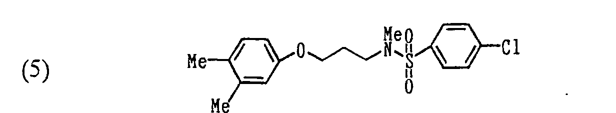

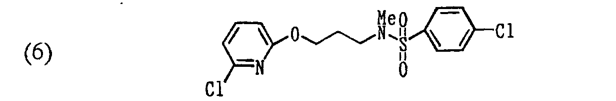

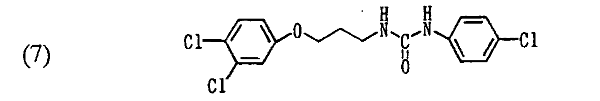

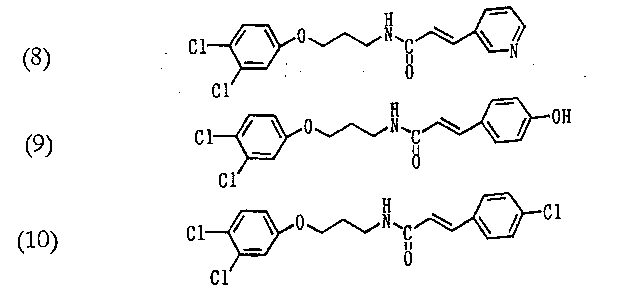

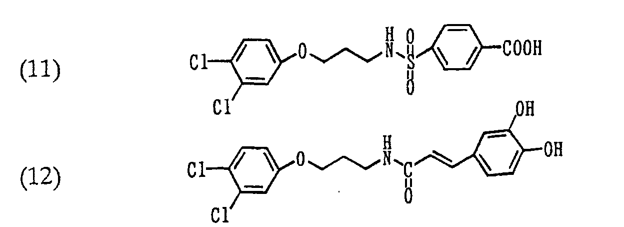

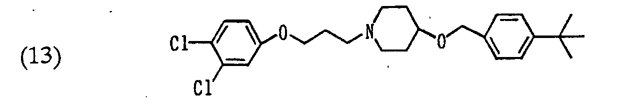

### Compound No.

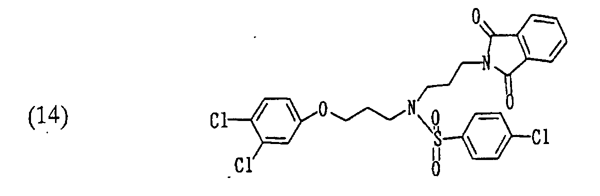

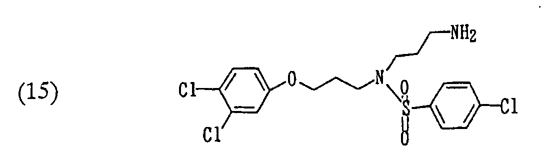

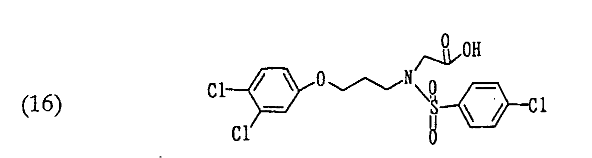

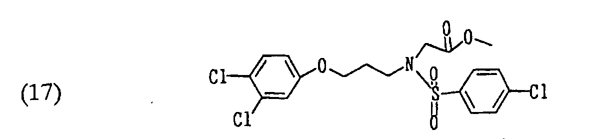

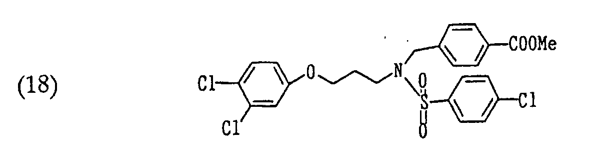

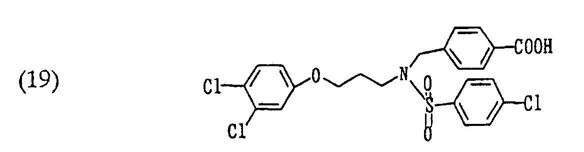

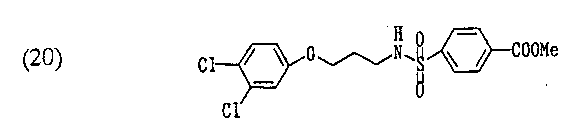

### Compound No.

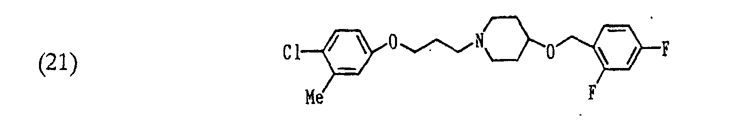

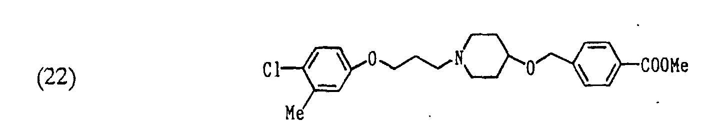

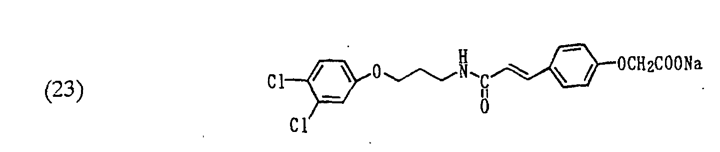

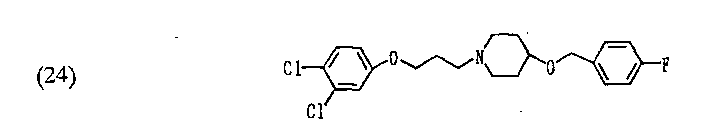

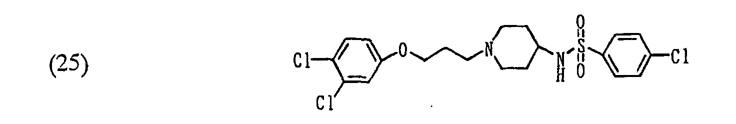

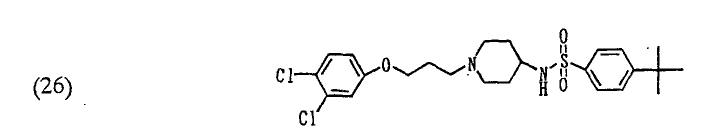

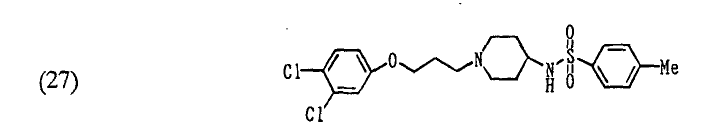

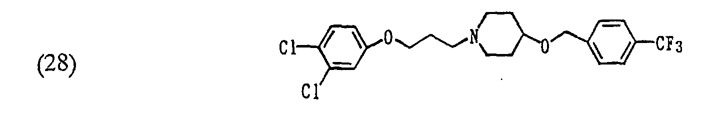

### Compound No.

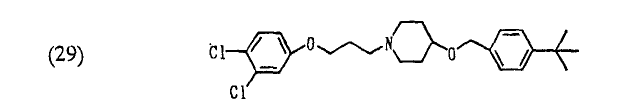

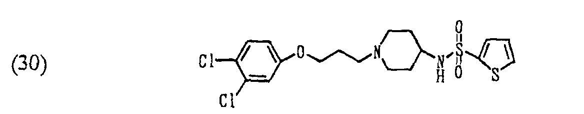

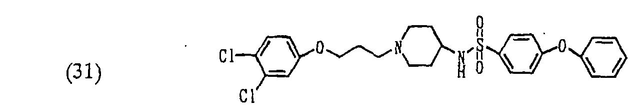

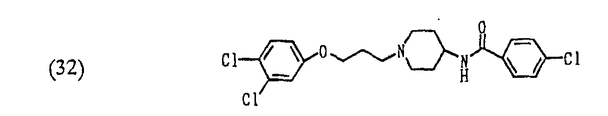

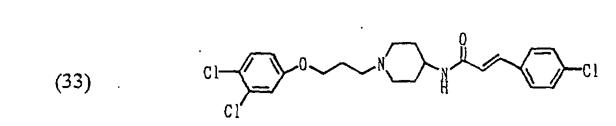

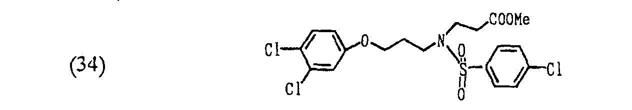

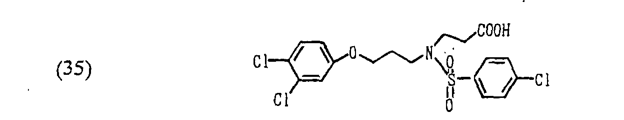

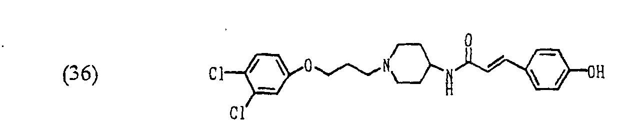

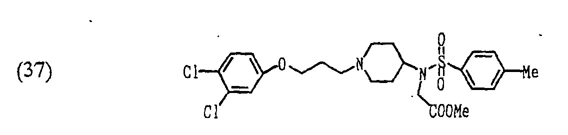

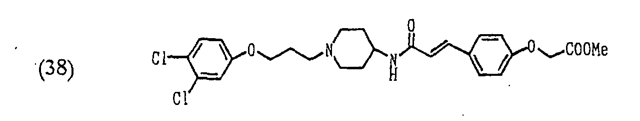

### Compound No.

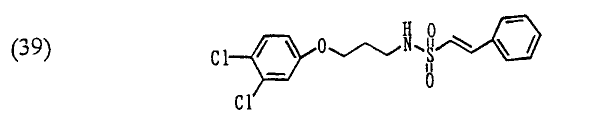

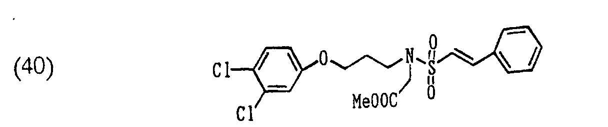

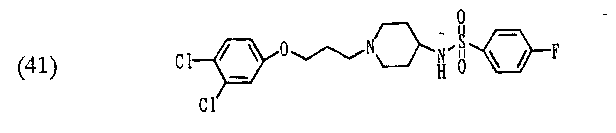

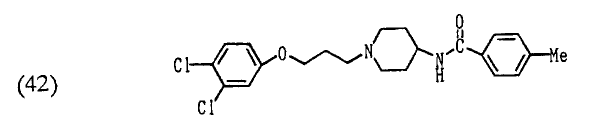

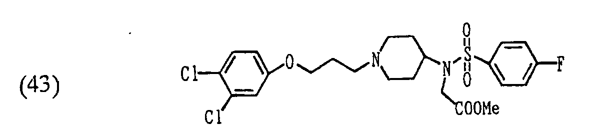

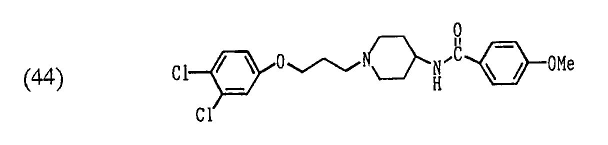

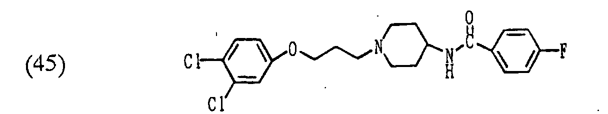

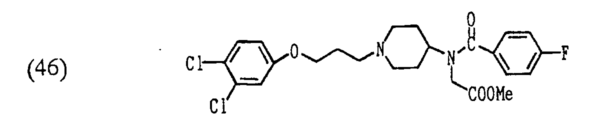

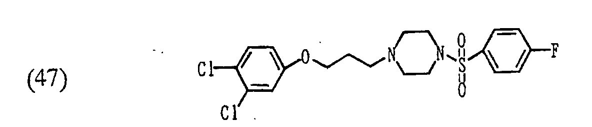

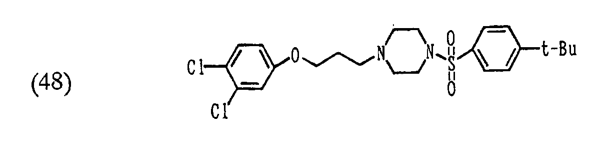

### Compound No.

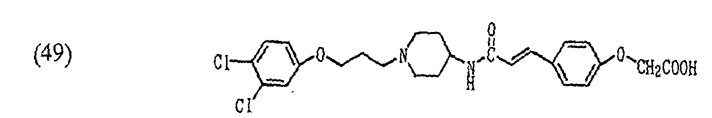

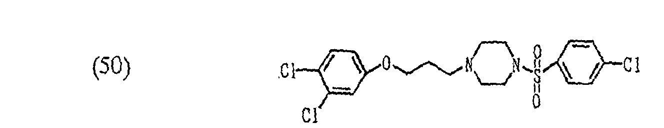

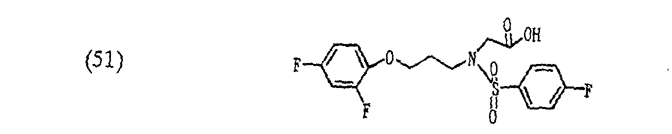

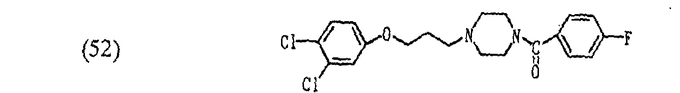

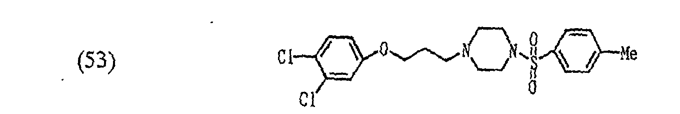

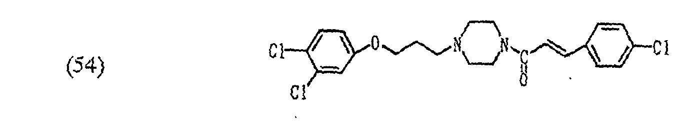

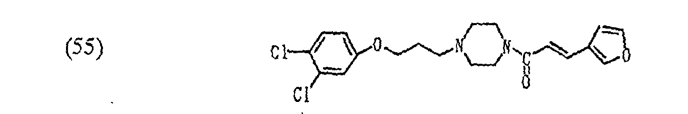

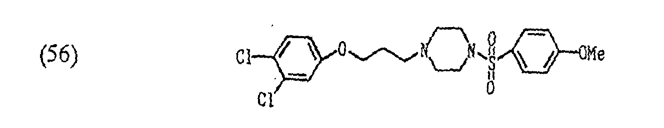

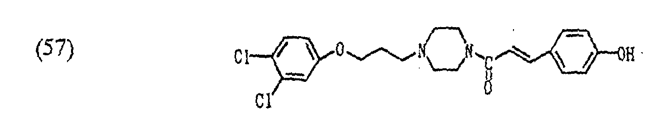

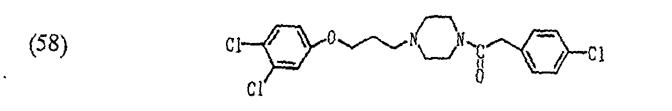

### Compound No.

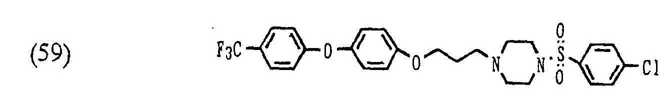

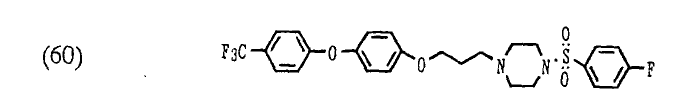

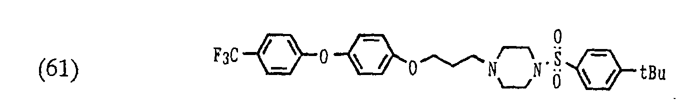

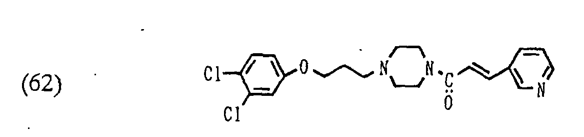

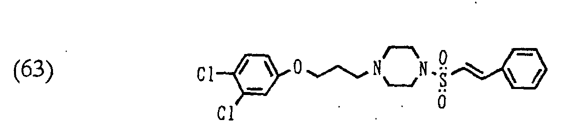

### Compound No.

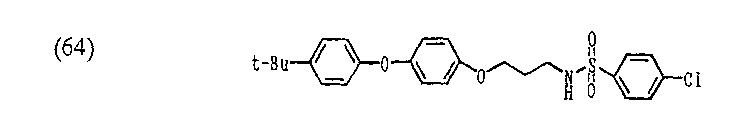

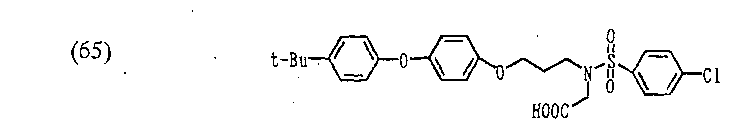

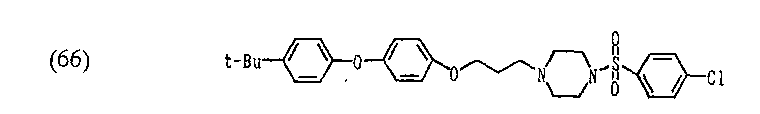

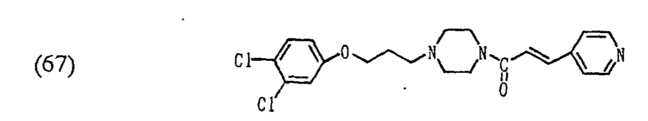

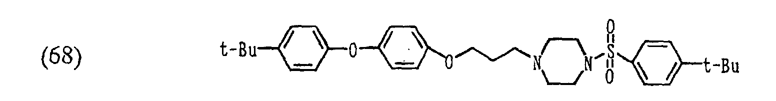

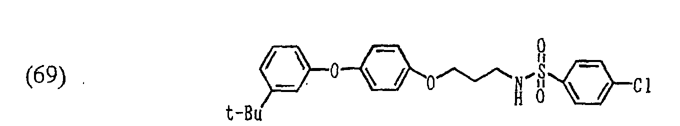

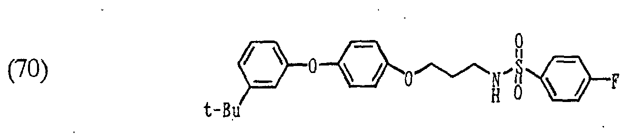

### Compound No.

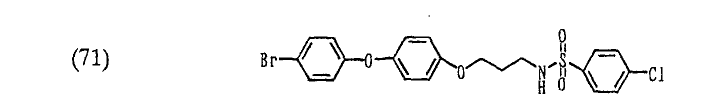

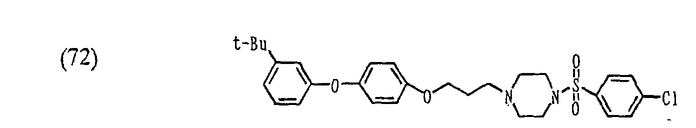

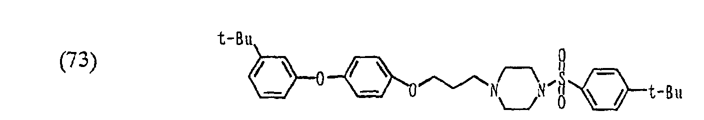

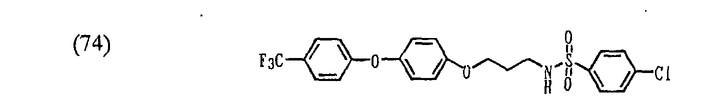

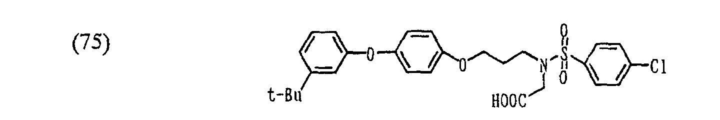

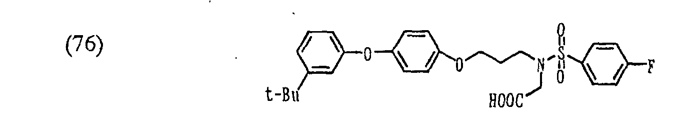

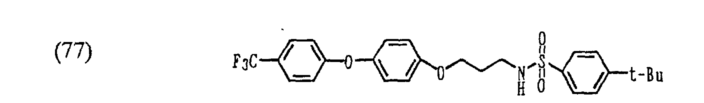

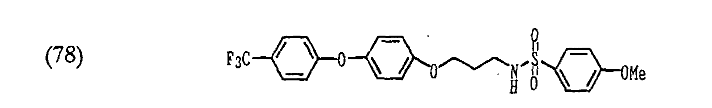

### Compound No.

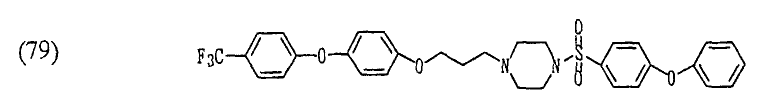

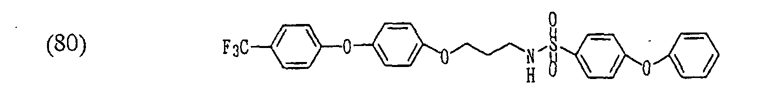

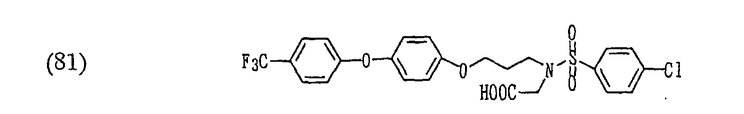

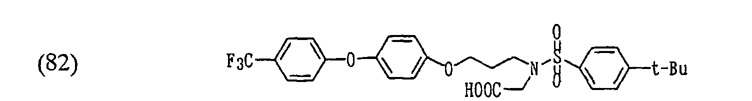

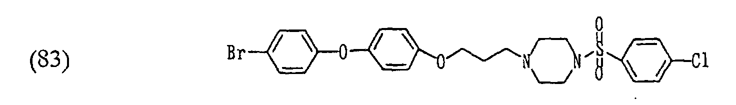

### Compound No.

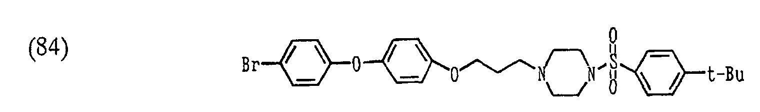

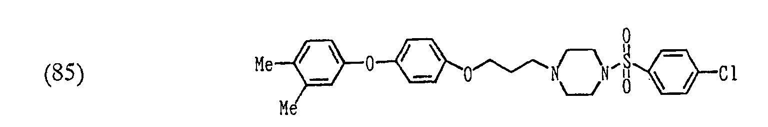

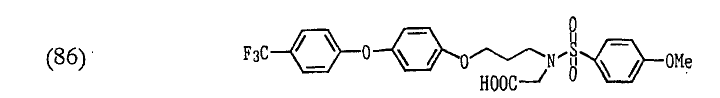

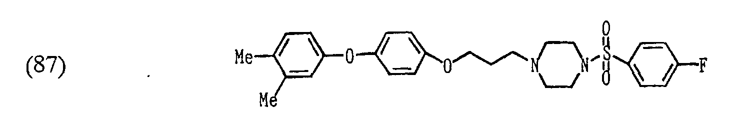

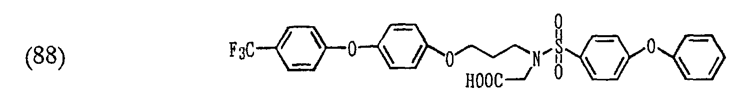

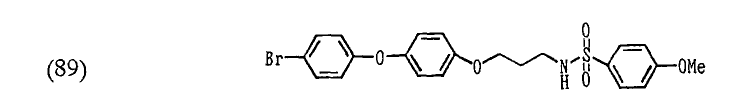

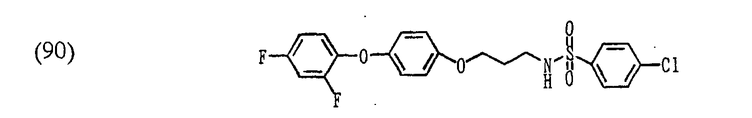

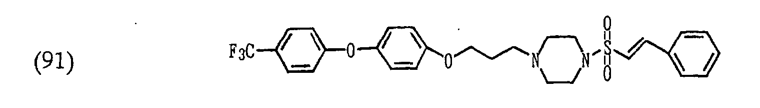

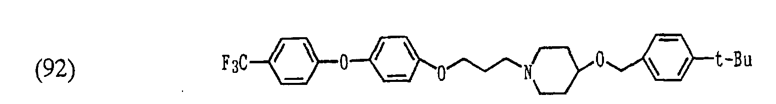

### Compound No.

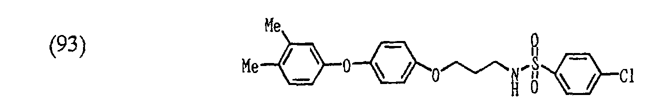

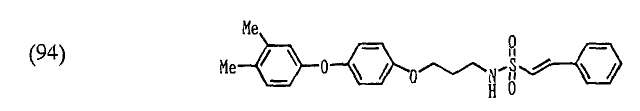

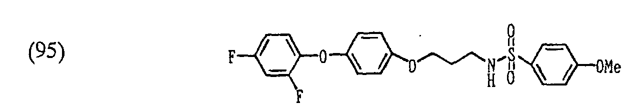

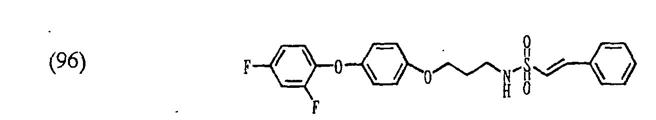

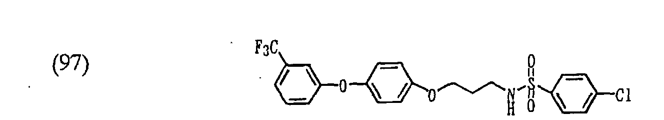

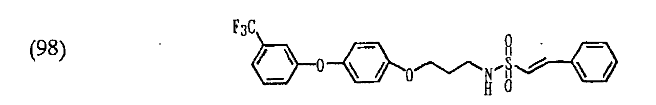

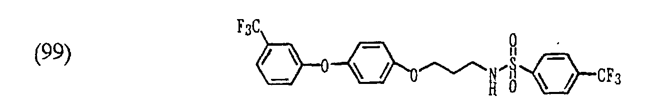

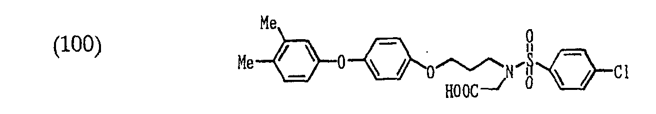

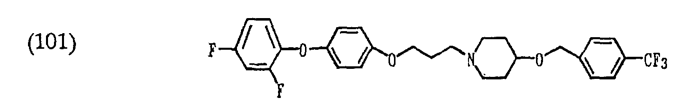

### Compound No.

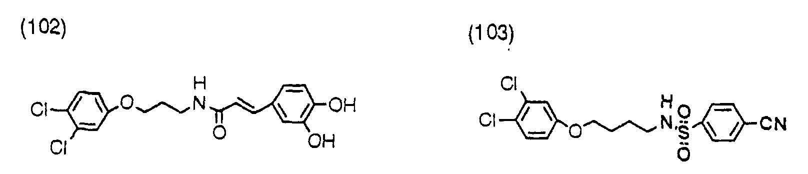

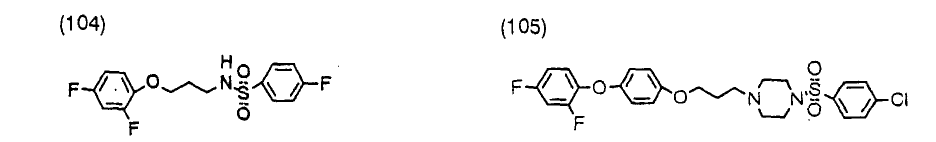

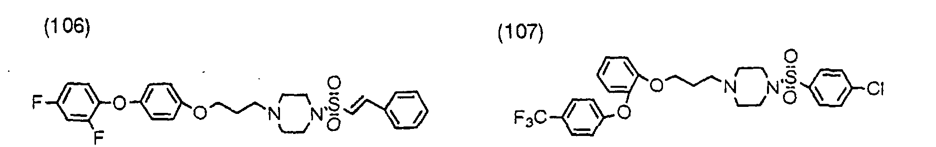

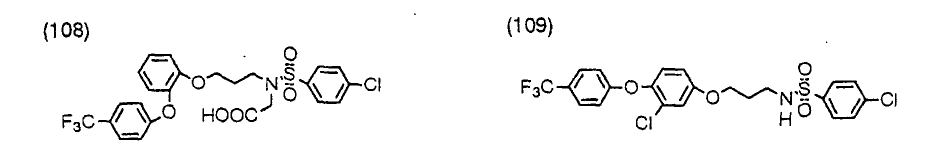

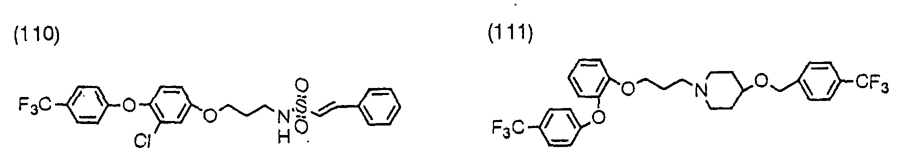

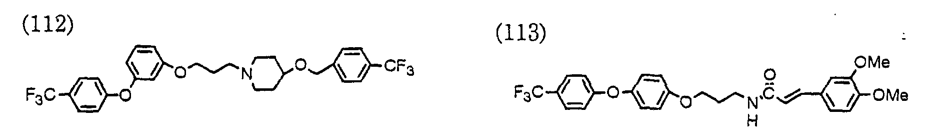

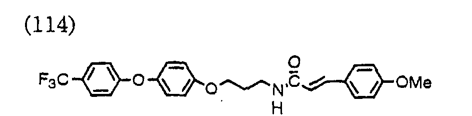

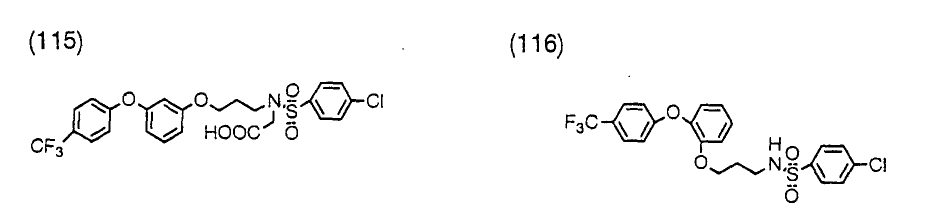

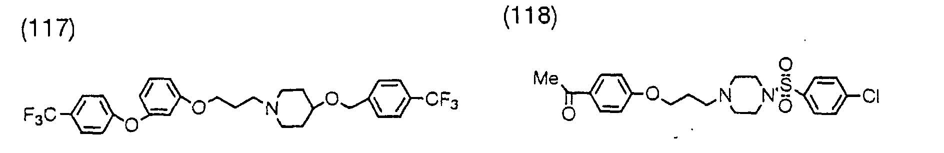

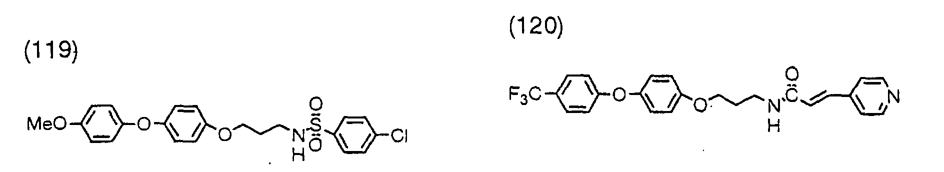

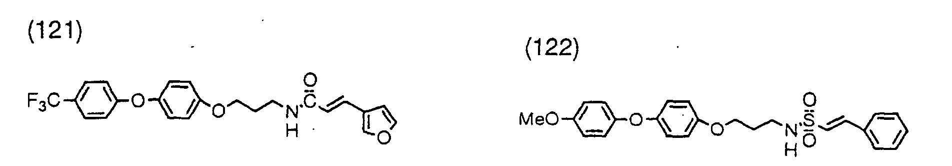

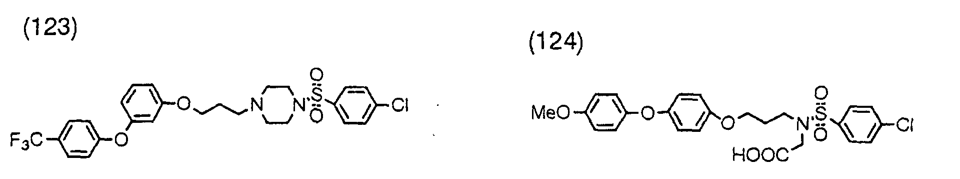

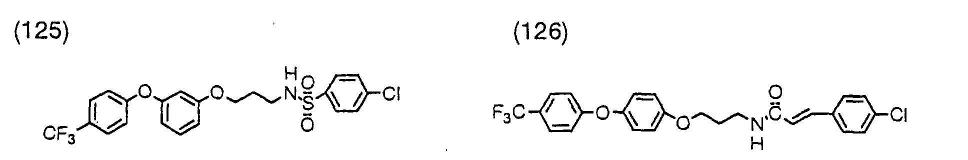

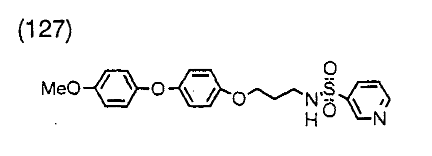

### Compound (1)

| Elementary analysis (%): C15H13Cl3O3S | | | | |
|---|---|---|---|---|
| Calcd. | C=47.45, | H=3.45, | Cl=28.01, | S=8.44 |
| Found | C=47.35, | H=3.51, | Cl=28.16, | S=8.52 |

### Compound (2)

| Elementary analysis (%): C18H18ClNO3S2 | | | | | |
|---|---|---|---|---|---|
| Calcd. | C=54.61, | H=4.58, | N=3.54, | Cl=8.95, | S=16.20 |
| Found | C=54.55, | H=4.56, | N=3.58, | Cl=9.22, | S=16.09 |

### Compound (3)

| Elementary analysis (%): C19H25ClN2O3S.HCl.1/3H2O | | | | | |
|---|---|---|---|---|---|
| Calcd. | C=51.94, | H=6.12, | N=6.38, | Cl=16.14, | S=7.30 |
| Found | C=51.70, | H=6.27, | N=6.73, | Cl=16.56, | S=6.91 |

NMR (CDCl₃) δ ppm (300 MH_{Z})
0.90∼1.05(2H,m), 1.60∼1.80(2H,m), 2.061(2H,quint,J=6Hz), 2.462(3H,s), 2.70∼2.80(2H,m), 2.796(3H,s), 3.229(2H,t,J=7Hz), 3.989(2H,t,J=6Hz), 6.909(1H,d,J=8Hz), 6.992(1H,d,J=8Hz), 7.487(2H,d,J=9Hz), 7.727(2H,d,9Hz)

### Compound (4)

| Elementary analysis (%): C17H19Cl2NO3S | | | | | |
|---|---|---|---|---|---|
| Calcd. | C=52.58, | H=4.93, | N=3.61, | Cl=18.26, | S=8.26 |
| Found | C=52.49, | H=4.88, | N=3.64, | Cl=18.20, | S=8.34 |

### Compound (5)

| Elementary analysis (%): C18H22ClNO3S | | | | | |
|---|---|---|---|---|---|
| Calcd. | C=58.77, | H=6.03, | N=3.81, | Cl=9.64, | S=8.71 |
| Found | C=58.75, | H=5.97, | N=3.84, | Cl=9.64, | S=8.80 |

### Compound (6)

| Elementary analysis (%): C15H16Cl2N2O3S | | | | | |
|---|---|---|---|---|---|
| Calcd. | C=48.01, | H=4.30, | N=7.46, | Cl=18.89, | S=8.54 |
| Found | C=47.89, | H=4.23, | N=7.43, | Cl=18.97, | S=8.56 |

### Compound (7)

| Elementary analysis (%): C16H15Cl3N2O2 | | | | |
|---|---|---|---|---|
| Calcd. | C=51.43, | H=4.05, | N=7.50, | Cl=28.46, |
| Found | C=51.40, | H=4.03, | N=7.60, | Cl=28.43, |

NMR (DMSO) δ ppm (300 MH_{Z})
1.877(2H,quint,J=6Hz), 3.235(2H,q,J=7Hz), 4.042(2H,t,6Hz), 6.20 ∼ 6.30 (1H,m), 6.974(1H,dd,J1=9Hz,J2=3Hz), 7.20∼7.30(3H,m), 7.410(2H,d,J=7Hz), 7.515(1H,d,J=9Hz), 8.592(1H,s)

### Compound (8)

| Elementary analysis (%): C17H16Cl2N2O2 | | | | |
|---|---|---|---|---|
| Calcd. | C=58.13, | H=4.59, | N=7.98, | Cl=20.19, |
| Found | C=58.10, | H=4.51, | N=8.06, | Cl=20.48, |

NMR (DMSO) δ ppm (300 MH_{Z})
1.915(2H,quint,J=6.6Hz), 4.056(2H,t,6.3Hz), 6.720(2H,d,16Hz), 6.976(1H,dd,J1=9Hz,J2=3Hz), 7.238(1H,d,J=3Hz), 7.41∼7.50(3H,m), 7.521(1H,d,J=9Hz), 7.95∼8.80(4H,m)

### Compound (9)

| Elementary analysis (%): C18H17Cl2NO3 | | | | |
|---|---|---|---|---|
| Calcd. | C=59.03, | H=4.68, | N=3.82, | Cl=19.36, |
| Found | C=59.09, | H=4.69, | N=3.96, | Cl=19.09, |

NMR (DMSO)δ ppm (300 MH_{Z})
1.905(2H,quint,J=6.6Hz), 4.051(2H,t,6.3Hz), 6.526(2H,d,16Hz), 6.74∼7.28(6H,m), 7.318(1H,d,16Hz), 7.521(1H,d,J=9Hz), 8.10∼ 8.25(1H,m), 9.50∼9.70(1H,m).

### Compound (10)

| Elementary analysis (%): C18H16Cl3NO2 | | | | |
|---|---|---|---|---|
| Calcd. | C=56.20, | H=4.19, | N=3.64, | Cl=27.65, |
| Found | C=56.13, | H=4.20, | N=3.76, | Cl=27.67, |

NMR (DMSO) δ ppm (300 MH_{Z})
1.912(2H,quint,J=6Hz), 4.052(2H,t,6Hz), 6.624(2H,d,16Hz), 6.972(1H,dd,J1=9Hz,J2=3Hz), 7.233(1H,d,J=3Hz), 7.35∼7.65(6H,m), 8.10∼8.30(1H,m)

### Compound (11)

| Elementary analysis (%): C16H15Cl2NO5S | | | | | |
|---|---|---|---|---|---|
| Calcd. | C=47.54, | H=3.74, | N=3.46, | Cl=17.54, | S=7.93, |
| Found | C=47.46, | H=3.67, | N=3.45, | Cl=17.70, | S=8.03, |

NMR (DMSO) δ ppm (300 MH_{Z})
1.803(2H, quint, J=7Hz), 2.952(2H,q,J=7Hz), 3.948(2H,t,6Hz), 6.876(1H,dd,J1=9Hz,J2=3Hz), 7.134(1H,d,J=3Hz), 7.477(1H,d,J=9Hz), 7.896(2H,d,J=8Hz), 8.098(2H,d,J=8Hz)

### Compound (14)

| Elementary analysis (%): C26H23Cl3N2O5S | | | | | |
|---|---|---|---|---|---|
| Calcd. | C=53.67, | H=3.98, | N=4.81, | Cl=18.28, | S=5.51, |
| Found | C=53.48, | H=3.98, | N=4.73, | Cl=18.39, | S=5.73, |

NMR (CDCl₃) δ ppm (300 MH_{Z})
1.943(2H,quint,J=7Hz), 2.069(2H,quint,J=7Hz), 3.240(2H,t,J=7Hz), 3.305(2H,t,J=7Hz), 3.708(2H,t,J=7Hz), 3.952(2H,t,J=6Hz), 6.698(1H,dd,J1=9Hz,J2=3Hz), 6.940(1H,d,J=3Hz), 7.270(1H,d,J=9Hz), 7.455(2H,d,J=9Hz), 7.68∼7.88(6H,m)

### Compound (15)

| Elementary analysis (%): C18H21Cl3N2O3S.C2H2O4 (corresponding oxalate) | | | | | |
|---|---|---|---|---|---|
| Calcd. | C=44.33, | H=4.28, | N=5.17, | Cl=19.63, | S=5.92 |
| Found | C=44.23, | H=4.27, | N=5.15, | Cl=19.58, | S=6.09 |

NMR (CDCl₃) δ ppm (300 MH_{Z})(free)
1.50∼1.80(2H,m), 1.70,5(2H,quint,J=7Hz), 2.046(2H,quint,J=7Hz), 2.7613(2H,t,J=6Hz), 3.245(2H,t,J=7Hz), 3.301(2H,d,J=7Hz), 3.932(2H,t,J=6Hz), 6.712(1H,dd,J1=9Hz,J2=3Hz), 6.950(1H,d,J=3Hz), 7.318(1H,d,J=9Hz), 7.462(2H,d,J=9Hz), 7.748(2H,d,J=9Hz)

### Compound (16)

| Elementary analysis (%): C17H16Cl3NO5S | | | | | |
|---|---|---|---|---|---|
| Calcd. | C=45.10, | H=3.56, | N=3.09, | Cl=23.49, | S=7.08 |
| Found | C=44.92, | H=3.52, | N=3.10, | Cl=23.27, | S=7.19 |

NMR (DMSO) δ ppm (300 MH_{Z})
1.898(2H,quint,J=6.3Hz), 3.944(2H,t,J=6Hz), 4.032(2H,s), 6.884(1H,dd,J1=9Hz,J2=3Hz), 7.131(1H,d,J=3Hz), 7.500(1H,d,J=9Hz), 7.594(2H,d,J=9Hz), 7.819(2H,d,J=9Hz)

### Compound (17)

| Elementary analysis (%): C18H18Cl3NO5S.1/5H2O | | | | |
|---|---|---|---|---|
| Calcd. | C=45.96, | H=3.94, | N=2.98, | Cl=22.61, |
| Found | C=46.30, | H=4.03, | N=3.19, | Cl=22.32, |

NMR (CDCl₃) δ ppm (300 MH_{Z})
2.019(2H,quint,J=6Hz), 3.431(2H,t,J=7Hz), 3.654(3H,s), 3.936(2H,t,J=6Hz), 4.092(2H,s), 6.688(1H,dd,J1=9Hz,J2=3Hz); 6.925(1H,d,J=3Hz), 7.309(1H,d,J=9Hz), 7.435(2H,d,J=9Hz), 7.777(2H,d,J=9Hz)

### Compound (18)

| Elementary analysis (%): C24H22Cl3NO5S | | | | | |
|---|---|---|---|---|---|
| Calcd. | C=53.10, | H=4.08, | N=2.58, | Cl=19.59, | S=5.91, |
| Found | C=53.10, | H=4.06, | N=2.59, | Cl=19.48, | S=5.94, |

NMR (CDCl₃) δ ppm (300 MH_{Z})
1.847(2H,quint,J=6Hz), 3.267(2H,t,J=6Hz), 3.708(2H,t,J=6Hz), 3.919(3H,s), 4.340(2H,s), 6.534(1H,dd,J1=9Hz,J2=3Hz), 6.757(1H,d,J=3Hz),
7.252(1H, d,J=9Hz), 7.351(2H,d,J=9Hz), 7.499(2H,d,J=9Hz), 7.780(2H,d,J=8.7Hz), 7.936(2H,d,J=8.7Hz)

### Compound (19)

| Elementary analysis (%): C23H20Cl3NO5S.1/2H2O | | | | | |
|---|---|---|---|---|---|
| Calcd. | C=51.36, | H=3.94, | N=2.60, | Cl=19.77, | S=5.96, |
| Found | C=51.12, | H=3,37, | N=2.63, | Cl=19.45, | S=5.92, |

NMR (DMSO)δ ppm (300 MH_{Z})
1.60∼1.74(2H,m), 3.221(2H,t,J=7Hz), 3.765(2H,t,J=6Hz), 4.359(2H,s), 6.783(1H,dd,J1=9Hz,J2=3Hz), 7.081(1H,d,J=3Hz), 7.201(2H,d,J=8Hz), 7.475(1H,d,J=9Hz), 7.647(2H,d,J=8Hz), 7.820(2H,d,J=8Hz), 7.873(2H,d,J=8Hz)

### Compound (20)

| Elementary analysis (%): C17H17Cl2NO5S | | | | | |
|---|---|---|---|---|---|
| Calcd. | C=48.81, | H=4.10, | N=3.35, | Cl=16.95, | S=7.67 |
| Found | C=48.89, | H=4.05, | N=3.40, | Cl=17.10, | S=7.67 |

NMR (DMSO)δ ppm (300 MH_{Z})
1.790(2H,quint,J=6.3Hz), 2.970(2H,t,J=7Hz), 3.894(3H,s), 3.903(2H,t,J=6.3Hz), 6.843(1H,dd,J1=9Hz,J2=3Hz), 7.081(1H,d,J=3Hz), 7.461(2H,d,J=9Hz), 7.907(2H,d,J=8.4Hz), 8.082(2H,d,J=8.4Hz)

### Compound (21)

| Elementary analysis (%): C22H26ClF3NO2.HCl (corresponding hydrochloride) | | | | | |
|---|---|---|---|---|---|
| Calcd. | C=59.20, | H=6.10, | N=3.14, | Cl=15.89, | F=8.53 |
| Found | C=59.19, | H=6.03, | N=3.18, | Cl=15.96, | F=8.53 |

NMR (CDCl₃) δ ppm (300 MH_{Z}) (free)
1.56∼2.26(8H,m), 2.328(3H,s), 2.500(2H,t,J=7Hz), 2.70∼2.85(2H,m), 3.36∼3.50(1H,m), 3.966(2H,t,J=6Hz), 4.545(2H,s), 6.64∼6.92(4H,m), 7.201(1H,d,J=9Hz), 7.408(2H,d,J=9Hz)

### Compound (22)

| Elementary analysis (%): C24H30Cl3NO4 | | | | |
|---|---|---|---|---|
| Calcd. | C=66.73, | H=7.00, | N=3.24, | Cl=8.21 |
| Found | C=66.34, | H=6.89, | N=3.26, | Cl=8.58 |

NMR (CDCl₃) δ ppm (300 MH_{Z})
1.45∼2.30(8H,m), 2.503(2H,t,J=7Hz), 2,70∼2.85(2H,m), 3.40∼3.60(1H,m), 3.914(3H,s), 3.967(2H,t,J=6Hz), 4.597(2H,s), 6.662(1H,dd,J1=9Hz,J2=3Hz), 6.764(1H,d,J=3Hz), 7.200(1H,d,J=9Hz), 7.416(2H,d,J=9Hz), 8.013(2H,d,J=9Hz)

### Compound (23)

| Elementary analysis (%): C20H18Cl2NO5Na.1/2H2O | | | | | |
|---|---|---|---|---|---|
| Calcd. | C=52.76, | H=4.21, | N=3.08, | Cl=15.57, | Na=5.05 |
| Found | C=52.73; | H=4.18, | N=3.28, | Cl=15.36, | Na=4.77 |

NMR (DMSO) δ ppm (300 MH_{Z})
1.907(2H,quint,J=7Hz), 4.064(2H,t,J=6Hz), 4.166(2H,s), 6.80∼7.30(7H,m), 7.323(1H,d,J=16Hz), 7.513(1H,d,J=9Hz), 8.55∼8.70(1H,m)

### Compound (24)

| Elementary analysis (%): C21H24Cl2FNO2.HCl (corresponding hydrochloride) | | | | | |
|---|---|---|---|---|---|
| Calcd. | C=55.20, | H=5.61, | N=3.12, | Cl=23.70, | F=4.23 |
| Found | C=56.03, | H=5.47, | N=13.17, | Cl=23.55, | F=4.08 |

NMR (CDCl₃)δ ppm (300 MH_{Z}) (free)
1.60∼2.24(8H,m), 2.474(2H,t,J=7Hz), 2.70∼2.82(2H,m),
3.35∼3.50(1H,m), 3.977(2H,t,J=6Hz), 4.499(2H,s), 6.72∼7.36(7H,m)

### Compound (25)

| Elementary analysis (%): C20H23Cl3N2O3S HCl /0.2(H₂O) (corresponding hydrochloride) | | | | | |
|---|---|---|---|---|---|
| Calcd. | C =46.71, | H =4.70, | N =5.45, | Cl =27.57, | S =6.23 |
| Found | C =46.38, | H =4.75, | N =5.41, | Cl =27.38, | S=6.19 |

### Compound (26)

| Elementary analysis (%): C24H32Cl2N2O3S HCl /0.2(MeOH) (corresponding hydrochloride) | | | | | |
|---|---|---|---|---|---|
| Calcd. | C =53.78, | H =6.21, | N =5.23, | Cl =19.84, | S =5.98 |
| Found | C =53.59, | H =6.28, | N =5.17, | Cl =19.61, | S =5.91 |

### Compound (27)

| Elementary analysis (%): C21H26Cl2N2O3S HCl (corresponding hydrochloride) | | | | | |
|---|---|---|---|---|---|
| Calcd. | C =51.07, | H =5.51, | N =5.67, | Cl =21.54, | S =6.49 |
| Found | C =50.77, | H =5.45, | N =5.73, | Cl =21.06, | S =6.54 |

NMR(CDCl₃) δ ppm (300 MH_{Z}) (free)
1.480∼2.130(8H, m), 2.400∼2.500(2H, m), 2.4,32(3H,s), 2.700∼2.820(2H, m), 3.100∼3.250(1H,m), 3.946(2H, t, *J* = 6Hz), 4.400∼4.570(1H, m), 6.725(1H, dd, *J* = 9Hz, 3Hz), 6.971(1H, d, *J* = 3Hz), 7.270∼7.340(3H, m), 7.763(2H, d, *J* = 9Hz)

### Compound (29)

| Elementary analysis (%): C25H33Cl2NO2.HCl (corresponding hydrochloride) | | | | |
|---|---|---|---|---|
| Calcd. | C=61.67, | H=7.04, | N=2.88, | Cl=21.84 |
| Found | C=61.58, | H=7.03, | N=2.96, | Cl=21.78 |

NMR (CDCl₃) δ ppm (300 MH_{Z}) (free)
1.313(9H,s), 1.60∼2.22(8H,m), 2,473(2H,t,J=7Hz),
2,70∼2.85(2H,m), 3.35∼3.50(1H,m), 3.976(2H,t,J=6Hz), 4.511(2H,s), 6.746(1H,dd,J1=911z,J2=3Hz), 6.995(1H,d,J=3Hz), 7.20∼7.40(5H,m)

### Compound (30)

| Elementary analysis (%): C18H22Cl2N2O3S2 HCl (corresponding hydrochloride) | | | | | |
|---|---|---|---|---|---|
| Calcd. | C =44.50, | H =4.47, | N =5.77, | Cl =21.89, | S =13.20 |
| Found | C =44.45, | H =4.72, | N =5.66, | Cl =21.80, | S =13.11 |

NMR(CDCl₃) δ ppm (300 MH_{Z}) ( free )
1.500∼2.300(8H, m), 2.450∼2.600(2H, m), 2.800∼2.900(2H, m), 3.250∼3.400(1H,m), 3.961(2H, t, J= 6Hz), 4.550∼4.700(1H, m), 6.729(1H, dd, J= 9Hz, 3Hz), 6.976(1H, d, J= 3Hz), 7.070∼7.100(1H, m), 7.302(1H, d, J= 9Hz), 7.560 ∼7.640(2H, m)

### Compound (31)

| Elementary analysis (%): C26H28Cl2N2O4SHCl (corresponding hydrochloride ) | | | | | |
|---|---|---|---|---|---|
| Calcd. | C =54.60, | H =5.11, | N =4.90, | Cl =18.60, | S =5.61 |
| Found | C =54.88, | H =5.07, | N =4.87, | Cl =18.36, | S =5.76 |

NMR (CDCl₃) δ ppm (300 MH_{Z}) (free)
1.500∼2.400(8H, m), 2.450∼2.570(2H, m), 2.700∼2.870(2H, m), 3.140∼3.280(1H,m), 3.954(2H, t, *J* = 6Hz), 4.460∼4.600(1H, m), 6.726(1H, dd, *J* = 9Hz, 3Hz), 6.973(1H, d, *J* = 3Hz), 7.000∼7.100(4H, m), 7.190∼7.260(1H, m), 7.298(1H, d, *J* = 9Hz), 7.380∼7.450(2H, m), 7.380∼7.450(2H, m)

### Compound (32)

| Elementary analysis (%): C21H23Cl3N2O2 HCl (corresponding hydrochloride) | | | | |
|---|---|---|---|---|
| Calcd. | C =52.74, | H =5.06, | N =5.86, | Cl =29.65 |
| Found | C =52.89, | H =5.01, | N =5.85, | Cl =29.44 |

NMR (CDCl₃)δ ppm (300 MH_{Z}) (free)
1.540∼1.690(2H, m), 1.988(2H, quint, *J* = 7.8Hz),2.020∼2.110(2H, m), 2.160 ∼2.280(2H, m), 2.558(2H, t, *J* = 7.2Hz), 2.900∼3.000(2H, m), 3.993(2H, t, *J* = 6Hz), 3.900∼4.200(1H, m), 5.900∼6.000(1H, m), 6.750(1H, dd, *J* = 9Hz, 3Hz), 7.001(1H, d, *J* = 3Hz), 7.310(1H, d, *J* = 9Hz), 7.407(2H, d, *J* = 9Hz), 7.695(2H, d, *J =* 8Hz)

### Compound (33)

| Elementary analysis (%): C23H25Cl3N2O2 HCl /0.1(MeOH) (corresponding hydrochloride) | | | | |
|---|---|---|---|---|
| Calcd. | C =54.78, | H =5.20, | N =5.56, | Cl =28.12 |
| Found | C =54.67, | H =5.24, | N =5.52, | Cl =27.94 |

NMR (CDCl₃) δ ppm (300 MH_{Z}) (free)
1.520∼1.700(2H, m), 1.960∼2.080(4H, m), 2.160∼2.300(2H, m), 2.579(2H, t, *J* = 7.2Hz), 2.900∼13.010(2H, m), 3.900∼4,1300(1H,m), 3.993(2H, t, *J* = 6Hz), 5.520∼5.590(1H, m), 6.343(1H, d, *J* = 15Hz), 6.751(1H, d, *J* = 9Hz, 3Hz), 7.000(1H, d, 3Hz), 7.312(1H, d, *J* = 9Hz), 7.340(2H, d, *J* = 8Hz), 7.430(2H, d, *J* = 9Hz), 7.571(1H, d, *J* = 15Hz).

### Compound (34)

| Elementary analysis (%): C19H20Cl3NO5S | | | | | |
|---|---|---|---|---|---|
| Calcd. | C=47.46, | H=4,19, | N=2.91, | Cl=22.12, | S=6.67 |
| Found | C=47.38, | H=4.17, | N=3.00, | Cl=22.02, | S=6.71 |

### Compound (35)

| Elementary analysis (%): C18H18Cl3NO5S | | | | | |
|---|---|---|---|---|---|
| Calcd. | C=46.32, | H=3.89, | N=3.00, | Cl=22.79, | S=6.87 |
| Found | C=46.18, | H=3.78, | N=3.06, | Cl=22.69, | S=6.89 |

NMR (DMSO)δ ppm (300 MH_{Z})
1.905(2H,quint,J=6Hz), 3.20∼3.42(6H,m), 3.963(2H,t,J=6Hz), 6.924(1H,dd,J1=9Hz,J2=3Hz), 7.179(1H,d,J=3Hz), 7.514(1H,d,J=9Hz), 7.650(2H,d,J=9Hz), 7.821(2H,d,J=9Hz)

### Compound (36)

| Elementary analysis (%): C23H26Cl2N2O3 HCl/0.1(MeOH) (corresponding hydrochloride) | | | | |
|---|---|---|---|---|
| Calcd. | C =56.80, | H =5.60, | N =5.77, | Cl =21.89 |
| Found | C =56.44, | H =5.55, | N =5.83, | Cl =21.52 |

NMR (CD3OD) δ ppm (300 MH_{Z}) (free)
1.500∼1.660(2H,m), 1.880∼2.050(4H, m), 2.120∼2.250(2H, m), 2.565(2H, t, *J* = 7.5Hz), 2.930∼3.040(2H, m), 3.740∼3.860(1H,m), 4.026(2H, t, *J* = 6Hz), 6.404(1H, d, *J* = 16Hz), 6.787(1H, d, *J* = 9Hz), 6.874(1H, dd, *J* = 9Hz, 3Hz), 7.098(1H, d, *J* = 3Hz), 7.360∼7.410(4H, m) , 7.449(1H, d, *J =* 16Hz)

### Compound (37)

| Elementary analysis (%): C24H30Cl2N2O5S HCl (corresponding hydrochloride) | | | | | |
|---|---|---|---|---|---|
| Calcd. | C =50.94, | H =5.52, | N =4.95, | Cl =18.79, | S =5.67 |
| Found | C =50.65, | H =5.44, | N =5.00, | Cl =18.66, | S =5.63 |

NMR (CDCl₃) δ ppm (300 MH_{Z}) (free)
1.500∼1.700(4H, m), 1.840∼2.040(4H, m), 2.380∼2.500(5H, m), 2.840∼2.960(2H, m), 3.500∼3.640(1H, m), 3.721(3H,s), 3.936(2H, t, *J* = 6Hz), 4.046(2H, s), 6.723(1H, dd, *J* = 9Hz, 3Hz), 6.970(1H, d, *J* = 3Hz), 7.294(1H, d, 9Hz), 7.299(2H, d, *J* = 8Hz), 7.836(2H, d, *J* = 8Hz)

### Compound (38)

| Elementary analysis (%): C26H30Cl2N2O5 HCl (corresponding hydrochloride) | | | | |
|---|---|---|---|---|
| Calcd. | C =55.98, | H =5.60, | N =5.02, | Cl =19.06 |
| Found | C =55.59, | H =5.56, | N =5.05, | Cl =18.68 |

NMR (CDCl₃) δ ppm (300 MH_{Z}) (free)
1.470∼1.610(2H, m), 1.900∼2.060(4H, m), 2.100∼2.240(2H, m), 2.528(2H, t, *J* = 7Hz), 2.710∼2.940(2H, m), 3.812(3H, s), 3.900∼4.000(1H,m), 3.985(2H, t, *J* = 6Hz), 4.656(2H, s), 5.440∼5.500(1H,m), 6.245(1H, d, *J* = 15Hz), 6.750(1H, dd, *J* = 9Hz, 3Hz), 6.889(2H, d, *J* = 9Hz), 6.999(1H, d, *J* = 3Hz), 7.306(1H, d, *J* = 9Hz), 7.445(2H, d, *J =* 9Hz), 7.559(1H, d, *J =* 15Hz)

### Compound (39)

| Elementary analysis (%): C17H17C12NO3S | | | | | |
|---|---|---|---|---|---|
| Calcd. | C=52.86, | H=4.44, | N=3.63, | Cl=18.36, | S=8.30 |
| Found | C=52.94, | H=4.38, | N=3.61, | Cl=18.31, | S=8.33 |

NMR (CDCl₃) δ ppm (300 MH_{Z}) (free)
2.046(2H,quint,J=6Hz), 3.296(2H,q,J=6Hz), 4.028(2H,t,J=6Hz), 4.58∼4.70(1H,m), 6.68∼6.75(2H,m), 6.951(1H,d,J=3Hz), 7.25∼7.52(7H,m)

### Compound (40)

| Elementary analysis (%): C20H21Cl2NO5S | | | | | |
|---|---|---|---|---|---|
| Calcd. | C=52.41, | H=4.62, | N=3.06, | Cl=15.47, | S=7.00 |
| Found | C=52.53, | H=4.48, | N=3.03, | Cl=15.43, | S=6.98 |

### Compound (41)

| Elementary analysis (%): C20H23Cl2FN2O3S HCl (corresponding hydrochloride) | | | | | | |
|---|---|---|---|---|---|---|
| Calcd. | C =48.25, | H =4.86, | N =5.63, | Cl =21.36, | F =3.82, | S =6.44 |
| Found | C =48.13, | H =4.78, | N =5.69, | Cl =21.23, | F =3.72, | S =6.37 |

NMR (CDCl₃)δ ppm (300 MH_{Z}) (free)
1.400∼1.550(2H, m), 1.600∼1.830(4H, m), 1.900(2H, t, *J* = 7Hz), 1.850∼2.100(2H, m), 2.448(2H, t, *J* = 7Hz), 2.700∼2.800(2H,m), 3.100∼3.250(1H,m), 3.940(2H, t, *J* = 7Hz), 4.540∼4.630(1H,m), 6.723(1H, dd, *J* = 9Hz, 3Hz), 6.970(1H, d, *J =* 3Hz), 7.189(2H, dd, *J* =9Hz, 9Hz), 7.902(2H, dd, *J* =9Hz, 5Hz)

### Compound (42)

| Elementary analysis (%): C22H26Cl2N2O2 HCl/0.1(Et2O) (corresponding hydrochloride) | | | | |
|---|---|---|---|---|
| Calcd. | C =57.72, | H =5.94, | N =6.12, | Cl =23.23 |
| Found | C =57.83, | H =6.07, | N =6.02, | Cl =22.86 |

NMR (CDCl₃) δ ppm (300 MH_{Z}) (free)
1.580∼1.640(2H, m), 1.965(2H, quint, *J* = 6Hz), 2.000∼2.240(4H, m), 1.850∼2.100(2H, m), 2.394(3H, s), 2.522(2H, t, *J* = 6Hz), 2.850∼4.080(1H,m), 5.900∼6.000(1H,m), 6.755(1H, dd, *J* = 9Hz, 3Hz), 7.004(1H, d, *J* = 3Hz), 7.231(2H, d, *J* =8Hz, 9Hz), 7.309(1H, d, *J* =9Hz), 7.647(2H, d, *J* =8Hz)

### Compound (43)

| Elementary analysis (%): C23H27Cl2FN2O5S HCl/0.1(EtOH) (corresponding hydrochloride ) | | | | | | |
|---|---|---|---|---|---|---|
| Calcd. | C =48.47, | H =4.95, | N =4.92, | Cl =18.66, | F =3.33, | S =5.63 |
| Found | C =48.50, | H =5.02, | N =4.88, | Cl =18.51, | F =3.31, | S =5.58 |

NMR (CDCl₃) δ ppm (300 MH_{Z}) (free)
1.480∼1.800(4H, m), 1.84∼2.02(4H, m), 2.443(2H, t, *J* = 7Hz), 2.850∼2.970(2H,m), 3.45∼3.58(1H,m), 3.725(1H, s), 3.936(2H, t, *J* = 6Hz), 4.080(2H, s), 6.723(1H, dd, *J* = 9Hz, 3Hz), 6.973(1H, d, *J* = 3Hz), 7.12∼7.27(3H, m), 7.296(1H, d, *J* =9Hz), 8.016(1H,dd, *J* =9Hz, 5Hz)

### Compound (44)

| Elementary analysis (%): C22H26Cl2N2O3 HCl (corresponding hydrochloride) | | | | |
|---|---|---|---|---|
| Calcd. | C =55.77, | H =5.74, | N =5.91, | Cl =22.45 |
| Found | C =55.57, | H =5.69, | N =6.07, | Cl =22.33 |

NMR (CDCl₃) δ ppm (300 MH_{Z}) (free)
1.470∼1.640(2H, m), 1.960(2H, quint, *J* = 7Hz), 2.000 ∼ 2.240(4H, m), 2.514(2H, t, *J =* 7Hz), 2.840∼2.940(2H, m), 3.849(3H, s), 3.989(2H, t, *J* = 7Hz), 5.840∼5.920(1H,m), 6.756(1H, dd, *J* = 9Hz, 3Hz), 6.923(2H, d, *J* = 9Hz), 7.005(1H, d, *J* =3Hz),7.309(1H, d, *J* = 9Hz), 7.718(2H, d, *J* =9Hz)

### Compound (45)

| Elementary analysis (%): C21H23Cl2FN2O2 HCl (corresponding hydrochloride) | | | | | |
|---|---|---|---|---|---|
| Calcd. | C =54.62, | H =5.24, | N =6.07, | Cl =23.03, | F =4.11 |
| Found | C =54.56, | H =5.23, | N =6.14, | Cl =22.91, | F =3.98 |

NMR (CDCl₃) δ ppm (300 MH_{Z}) (free)
1.500∼1.660(2H, m), 1.971(2H, quint, *J* = 8Hz), 2.000∼2.120(2H, m), 2.120∼2.250(2H, m), 2.532(2H, t, *J* = 8Hz), 2.860∼2.980(2H, m), 3.920∼4.080(1H,m), 3.990(3H, t, *J* = 8Hz), 5.900∼5.980(1H,m), 6.754(1H, dd, *J* = 9Hz, 3Hz), 7.003(2H, d, *J* = 3Hz), 7.109(2H, dd, *J* =9Hz, 9Hz), 7.310(1H, d, *J* = 9Hz), 7.764(2H, dd, *J* =9Hz, 5Hz)

### Compound (46)

| Elementary analysis (%): C24H27Cl2FN2O4 HCl (corresponding hydrochloride ) | | | | | |
|---|---|---|---|---|---|
| Calcd. | C =54.00, | H =5.29, | N =5.25, | Cl =19.92, | F =3.56 |
| Found | C =53.75, | H =5.30, | N =5.35, | Cl =19.65, | F =3.39 |

NMR (CDCl₃) δ ppm (300 MH_{Z}) (free)
1.500 ∼ 1.780(4H, m), 1.830∼2.000(4H, m), 2.400∼2.500(2H, m), 2.840∼2.960(2H, m), 3.450∼3.580(1H, m), 3.725(3H, s), 3.936(2H, t, *J* = 6Hz), 4.080(2H,s), 6.723(1H, dd, *J* = 9Hz, 3Hz), 6.973(1H, d, *J* = 3Hz), 7.120 ∼ 7.240(2H, m), 7.296(1H, d, *J* = 9Hz), 7.980∼8.500(2H, m)

### Compound (47)

| Elementary analysis (%): C19H21Cl2FN2O3S HC1/0.1(MeOH) (corresponding hydrochloride) | | | | | | |
|---|---|---|---|---|---|---|
| Calcd. | C =47.17, | H =4.58, | N =5.79, | Cl =21.98, | F =3.93, | S =6.63 |
| Found | C =47.10, | H =4.64, | N =5.75, | Cl =21.84, | F =3.90, | S =6.58 |

NMR (CDCl₃) δ ppm (300 MH_{Z}) (free)
1.885(2H, quint, 6Hz), 2.503(2H, t, *J* = 6Hz), 2.520∼2.600(4H, m), 2.960∼3.100(4H, m), 3.920(2H, t, *J* = 6Hz), 6.705(1H, dd, *J* = 9Hz, 3Hz), 6.948(1H, d, *J* = 3Hz), 7.220 (2H, dd, *J* = 9Hz, 9Hz), 7.284(1H, d, *J* = 3Hz), 7.740∼7.800(2H, m)

### Compound (48)

| Elementary analysis (%): C23H30Cl2N2O3S HCl (corresponding hydrochloride) | | | | | |
|---|---|---|---|---|---|
| Calcd. | C =52.93, | H =5.99, | N =5.37, | Cl =20.38, | S =6.14 |
| Found | C =52.87, | H =5.95, | N =5.40, | Cl =20.26, | S =6.10 |

NMR (CDCl₃) δ ppm (300 MH_{Z}) (free)
1.346(9H, s), 1.887(2H, quint, 7Hz), 2.502(2H, t, *J* = 7Hz), 2.520∼2.600(4H, m), 3.000∼3.100(4H, m), 3.920(2H, t, *J* = 7Hz), 6.704(1H, dd, *J* = 9Hz, 3Hz), 6.951(1H, d, *J* = 3Hz), 7.279 (1H, d, *J* = 9Hz), 7.531(2H, d, *J* = 8Hz), 7.676(2H, d, *J* = 8Hz)

### Compound (49)

| Elementary analysis (%): C25H28Cl2N2O5 HCl/0.1(Et2O) (corresponding hydrochloride) | | | | |
|---|---|---|---|---|
| Calcd. | C =55.21, | H =5.37, | N =5.15, | Cl =19.56 |
| Found | C =55.34, | H =5.49, | N =5.08, | Cl =19.29 |

### Compound (50)

| Elementary analysis (%): C19H21Cl3N2O3S.HCl (corresponding hydrochloride) | | | | | |
|---|---|---|---|---|---|
| Calcd. | C=45.62, | H=4.43, | N=5.60, | Cl=28.35, | S=6.41 |
| Found | C=45.44, | H=4.34, | N=5.66, | Cl=28.31, | S=6.36 |

NMR (CDCl₃) δ ppm (300 MH_{Z}) (free)
1.882(2H,quint,J=7Hz), 2.46∼2.60(6H,m), 2.94∼3.10(4H,m), 3.921(2H,t,J=6Hz), 6.706(1H,dd,J1=9Hz,J2=3Hz), 6.950(1H,d,J=3Hz), 7.284(1H,d,J=9Hz), 7.515(2H,d,J=9Hz), 7.697(2H,d,J=9Hz)

### Compound (51)

| Elementary analysis (%): C17H16F3NO5S | | | | | |
|---|---|---|---|---|---|
| Calcd. | C=50.62, | H=4.00, | N=3.47, | F=14.13, | S=7.95 |
| Found | C=50.43, | H=3.88, | N=3.54, | F=13.91, | S=7.84 |

NMR (DMSO) 6 ppm (300 MH_{Z})
1.917(2H,quint,J=7Hz), 3.347(2H,t,J=7Hz), 3.993(2H,t,J=6Hz), 4.022(2H,s), 6.94∼7.44(5H,m), 7.84∼7.96(2H,m)

### Compound (52)

| Elementary analysis (%): C20H21Cl2FN2O2 HCl (corresponding hydrochloride) | | | | | |
|---|---|---|---|---|---|
| Calcd. | C =53.65, | H =4.95, | N =6.26, | Cl =23.75, | F =4.24 |
| Found | C =53.44, | H =4.82, | N =6.27, | Cl =23.49, | F =4.12 |

NMR (CDCl₃) δ ppm (300 MH_{Z}) (free)
1.540∼1.800(2H, m), 1.973(2H, quint, 6Hz), 2.300∼2.620(4H, m), 3.400∼ 3.880(4H, m), 3.999(2H, t, *J* = 6Hz), 6.746(1H, dd, *J* = 9Hz, 3Hz), 6.995(1H, d, *J* = 3Hz), 7.098(2H, dd, *J* = 9Hz, 9Hz), 7.310(1H, d, *J* = 9Hz), 7.419(2H, dd, *J* = 9Hz, 5.4Hz)

### Compound (53)

| Elementary analysis (%): C20H24Cl2N2O3 HCl/0.1(H2O) (corresponding hydrochloride) | | | | | |
|---|---|---|---|---|---|
| Calcd. | C =50.06, | H =5.25, | N =5.84, | Cl =22.16, | S =6.68 |
| Found | C =49.87, | H =5.27, | N =5.82, | Cl =22.08, | S =6.66 |

### Compound (54)

| Elementary analysis (%): C22H23Cl3N2O2 HCl (corresponding hydrochloride) | | | | |
|---|---|---|---|---|
| Calcd. | C =53.79, | H =5.13, | N =5.70, | Cl =28.87 |
| Found | C =53.76, | H =4.96, | N =5.86, | Cl =28.73 |

NMR (CDCl₃) δ ppm (300 MH_{Z}) (free)
1.972(2H, quint, 6Hz), 2.460∼2.580(6H, m), 3.600∼3.780(4H, m), 4.009(2H, t, *J =* 6Hz), 6.756(1H, dd, *J* = 9Hz, 3Hz), 6.848(1H, d, *J* = 15Hz), 7.006(1H, d, *J* = 3Hz), 7.314 (1H, d, *J* = 9Hz), 7.343(2H, d, *J* = 8Hz), 7.621(1H, d, *J* = 15Hz)

### Compound (55)

| Elementary analysis (%): C20H22Cl2N2O3 HCl /0.1(H2O) (corresponding hydrochloride) | | | | |
|---|---|---|---|---|
| Calcd. | C =53.77, | H =5.41, | N =6.27, | Cl =23.71 |
| Found | C =53.76, | H =5.26, | N =6.42, | Cl =23.35 |

NMR (CDCl₃) δ ppm (300 MH_{Z}) (free)
1.971(2H, quint, 6Hz), 2.450∼2.580(4H, m), 3.580∼3.800(4H, m), 4.008(2H, t, *J* = 6Hz), 6.595(1H, d, *J* = 15Hz), 6.580∼6.600(1H, m), 6.759(1H, dd, *J =* 9Hz, 3Hz), 7.008(1H, d, *J* = 3Hz), 7.316 (1H, d, *J =* 9Hz), 7.410∼7.440(1H, m), 7.575(2H, d, *J* = 15Hz), 7.629(1H, s)

### Compound (56)

| Elementary analysis (%): C20H24Cl2N2O4S.HCl (corresponding hydrochloride) | | | | | |
|---|---|---|---|---|---|
| Calcd. | C=48.45, | H=5.08, | N=5.65, | Cl=21.45, | S=6.47 |
| Found | C=48.22, | H=5.07, | N=5.85, | Cl=21.16, | S=6.42 |

NMR (CDCl₃) δ ppm (300 MH_{Z}) (free)
1.878(2H,quint,J=6Hz), 2.45∼2.60(6H,m), 2.92∼3.08(4H,m), 3.871(3H,s), 3.916(2H,t,J=6Hz), 6.703(1H,dd,J1=9Hz,J2=3Hz), 6.948(1H,d,J=3Hz), 6.994(2H,d,J=9Hz), 7.277(1H,d,J=9Hz), 7.691(2H,d,J=9Hz)

### Compound (57)

| Elementary analysis (%): C22H24Cl2N2O3 HCl (corresponding hydrochloride) | | | | |
|---|---|---|---|---|
| Calcd. | C =56.01, | H =5.34, | N =5.94, | Cl =22.54 |
| Found | C =55.98, | H =5.26, | N =6.06, | Cl =22.20 |

NMR (CDCl₃) δ ppm (300 MH_{Z}) (free)
1.978(2H, quint, 6Hz), 2.470∼2.600(4H, m), 3.620∼3.840(4H, m), 4.000(2H, t, *J* = 6Hz), 6.714(1H, d, *J* = 15Hz), 6.747(1H, dd, *J* = 9Hz, 3Hz), 6.851 (2H, d, *J* = 9Hz), 6.997(1H, d, *J* = 3Hz), 7.308 (1H, d, *J* = 9Hz), 7.383(2H, d, *J* = 9Hz), 7.621(1H, d, *J =* 15Hz)

### Compound (58)

| Elementary analysis (%): C21H23Cl3N2O2 HCl (corresponding hydrochloride) | | | | |
|---|---|---|---|---|
| Calcd. | C =52.74, | H =5.06, | N =5.86, | Cl =29.65 |
| Found | C =52.52, | H =5.04, | N =5.96, | Cl =29.83 |

NMR (CDCl₃) δ ppm (300 MH_{Z}) (free)
1.925(2H, quint, 6Hz), 2.250∼2.360(2H, m), 2.360∼2.450(2H, m), 2.481(2H, t, *J* = 6Hz), 3.370∼3.520(2H, m), 3.570∼3.710(2H, m), 3.686(2H, s), 3.974(2H, t, 6Hz), 6.734(1H, dd, *J* = 9Hz, 3Hz), 6.984(1H, d, *J* = 3Hz), 7.174 (2H, d, *J* = 8Hz), 7.302 (2H, d, *J* = 8Hz),

### Compound (59)

| Elementary analysis (%): C26H26ClF3N2O4S.HCl (corresponding hydrochloride) | | | | | | |
|---|---|---|---|---|---|---|
| Calcd. | C=52.80, | H=4.60, | N=4.74, | Cl=11.99, | F=9.64, | S=5.42 |
| Found | C=52.63, | H=4.42, | N=4.83, | Cl=11.70, | F=9.43, | S=5.36 |

NMR (CDCl₃) δ ppm (300 MH_{Z}) (free)
1.919(2H,quint,J=6.3Hz), 2.50∼2.62(6H,m), 2.98∼3.12(4H,m), 3.961(2H,t,J=6.3Hz), 6.84∼7.02(6H,m), 7.48∼7.72(6H,m)

### Compound (60)

| Elementary analysis (%): C26H26F4N2O4S.HCl.1/10H2O (corresponding hydrochloride) | | | | | | |
|---|---|---|---|---|---|---|
| Calcd. | C=54.23, | H=4.59, | N=4.86, | Cl=6.16, | F=13.20, | S=5.57 |
| Found | C=54.01, | H=4.72, | N=4.85, | Cl=6.05, | F=13.09, | S=5.56 |

NMR (CDCl₃) δ ppm (300 MH_{Z}) (free)
1.914(2H,quint,J=6.6Hz), 2.50∼2.60(6H,m), 2.98∼3.12(4H,m), 3.956(2H,t,J=6.6Hz), 6.868(2H,d,J=9Hz), 6.92∼7.00(4H,m), 7.16∼7.26(2H,m), 7.529(2H,d,J=9Hz), 7.74∼7.82(2H,m)

### Compound (61)

| Elementary analysis (%): C30H35F3N2O4S.HCl.1/4H2O (corresponding hydrochloride) | | | | | | |
|---|---|---|---|---|---|---|
| Calcd. | C=58.34, | H=5.96, | N=4.54, | Cl=5.74, | F=9.23, | S=5.19 |
| Found | C=58.38, | H=6.02, | N=4.46, | Cl=5.54, | F=8.82, | S=5.09 |

NMR (CDCl₃) δ ppm (300 MH_{Z}) (free)
1.345(9H,s), 1.915(2H,quint,J=6.3Hz), 2.50∼2.62(6H,m), 2.98∼3.14(4H,m), 3.956(2H,t,J=6.3Hz), 6.84∼7.00(6H,m), 7.50∼7.70(6H,m)

### Compound (62)

| Elementary analysis (%): C21H23Cl2N3O2 | | | | |
|---|---|---|---|---|
| Calcd. | C =60.01, | H =5.52, | N =10.00, | Cl =16.87 |
| Found | C =59.90, | H =5.49, | N =10.05, | Cl =16.58 |

NMR (CDCl₃) δ ppm (300 MH_{Z})
1.983(2H, quint, 6Hz), 2.460∼2.600(6H, m), 3.600∼3.850(4H, m), 4.020(2H, t, *J* = 6Hz), 6.764(1H, dd, *J* = 9Hz, 2Hz), 6.958(1H, d, *J* = 15Hz), 7.015(1H, d, *J =* 2Hz), 7.280∼7.360(2H, m), 7.662 (1H, d, *J* = 15Hz), 7.817 (1H, d, *J* = 8Hz), 8.581(2H, d, *J* = 5Hz), 8.766(1H, s)

### Compound (63)

| Elementary analysis (%): C21H24Cl2N2O3S HCl (corresponding hydrochloride) | | | | | |
|---|---|---|---|---|---|
| Calcd. | C =51.28, | H =5.12, | N =5.70, | Cl =21.62, | S =6.52 |
| Found | C =51.24, | H =5.04, | N =5.79, | Cl =21.26, | S =6.47 |

NMR (CDCl₃)δ ppm (300 MH_{Z}) (free)
1.929(2H, quint, 6Hz), 2.490∼2.630(6H,m), 3.180∼3.290(4H, m), 3.961(2H, t, *J* = 6Hz), 6.677(1H, d, *J* = 15Hz), 6.725(1H, dd, *J* = 9Hz, 3Hz), 6.973(1H, d, *J* = 3Hz), 7,288 (1H, d, *J* = 9Hz), 7.450(1H, d, *J* = 15Hz), 7.380∼7.520(6H, m) Compound (64)

| Elementary analysis (%): C25H28ClNO4S | | | | | |
|---|---|---|---|---|---|
| Calcd. | C =63.35, | H =5.95, | N =2.95, | Cl =7.48, | S =6.76 |
| Found | C =63.11, | H =5.79, | N =3.00, | Cl =7.28, | S =6.77 |

NMR (CDCl₃) δ ppm (300 MH_{Z})
1.312(9H, s), 1.961(2H, quint, *J* = 6Hz), 3.218(2H, q, *J* = 6Hz), 3.959(2H, t, *J =* 6Hz), 4.820~4.890(1H, m), 6.794(2H, d, *J* = 9Hz), 6.879(2H, d, *J* = 9Hz), 6.960(2H, d, *J* = 9Hz), 7.318 (2H, d, *J* = 9Hz), 7.453(2H, d, *J* = 9Hz), 7.784(2H, d, *J* = 9Hz)

### Compound (65)

| Elementary analysis (%): C27H30ClNO6S | | | | | |
|---|---|---|---|---|---|
| Calcd. | C =60.95, | H =5.68, | N =2.63, | Cl =6.66, | S =6.03 |
| Found | C =60.83, | H =5.72, | N =2.70, | Cl =6.37, | S =5.91 |

### Compound (66)

| Elementary analysis (%): C29H35ClN2O4S.HCl (corresponding hydrochloride) | | | | | |
|---|---|---|---|---|---|
| Calcd. | C=60.10, | H=6.26, | N=4.83, | Cl=12.23, | S=5.53 |
| Found | C=59.93, | H=6.29, | N=4.87, | Cl=11.92, | S=5.50 |

NMR (CDCl₃) δ ppm (300 MH_{Z}) (free)
1.303(9H,s), 1.892(2H,quint,J=6Hz), 2.48∼2.62(6H,m), 2.96∼3.12(4H,m), 3.931(2H,t,J=6Hz), 6.78∼6.96(6H,m), 7.298(2H,d,J=9Hz), 7.507(2H,d,J=9Hz), 7.693(2H,d,J=9Hz)

### Compound (67)

| Elementary analysis (%): C21H23Cl2N3O2 | | | | |
|---|---|---|---|---|
| Calcd. | C =60.01, | H =5.52, | N =10.00, | Cl =16.87 |
| Found | C =59.91, | H =5.34, | N =10.08, | Cl =16.32 |

NMR (CDCl₃)δ ppm (300 MH_{Z})
1.976(2H, quint, *J* = 6Hz), 2.460 ∼ 2.600(6H, m), 3.600 ∼ 3.830(4H, m), 4.012(2H, t, *J* = 6Hz), 6.754(1H, dd, *J* = 9Hz, 3Hz), 7.006(1H, d, *J* = 3Hz), 7.037(1H, d, *J* = 15Hz), 7.338 (1H, d, *J =* 9Hz), 7.358 (1H, d, *J =* 6Hz), 7.567(1H, d, *J* = 15Hz), 8.632(2H, d, 6Hz)

### Compound (68)

| Elementary analysis (%): C33H44N2O4S.HCl.1/10H2O (corresponding hydrochloride) | | | | | |
|---|---|---|---|---|---|
| Calcd. | C=65.73, | H=7.55, | N=4.65, | Cl=5.88, | S=5.32 |
| Found | C=65.72, | H=7.66, | N=4.47, | Cl=5.53, | S=5.12 |

NMR (CDCl₃) δ ppm (300 MH_{Z}) (free)
1.301(9H,s), 1.342(9H,s), 1.898(2H,quint,J=6Hz), 2.44∼2.64(6H,m), 2.94∼3.12(4H,m), 3.931(2H,t,J=6Hz), 6.78∼6.96(6H,m), 7.296(2H,d,J=8.4Hz), 7.526(2H,d,J=8.4Hz), 7.676(2H,d,J=8.4Hz)

### Compound (69)

| Elementary analysis (%): C25H28ClNO4S | | | | | |
|---|---|---|---|---|---|
| Calcd. | C=63.35, | H=5.95, | N=2.95, | Cl=7.48, | S=6.76 |
| Found | C=63.33, | H=5.90, | N=2.97, | Cl=7.61, | S=6.75 |

NMR (CDCl₃) δ ppm (300 MH_{Z})
1.300(9H,s), 1.954(2H,quint,J=6Hz), 3.210(2H,q,J=6Hz), 3.955(2H,t,J=6Hz), 4.90∼5.00(1H,m), 6.66∼7.26(8H,m), 7.446(2H,d,J=9Hz), 7.794(2H,d,J=9Hz)

### Compound (70)

| Elementary analysis (%): C25H28FNO4S | | | | | |
|---|---|---|---|---|---|
| Calcd. | C=65.62, | H=6.17, | N=3.06, | F=4.15, | S=7.01 |
| Found | C=65.40, | H=5.99, | N=3.14, | F=4.09, | S=7.04 |

NMR (CDCl₃) δ ppm (300 MH_{Z})
1.300(9H,s), 1.958(2H,quint,J=6Hz), 3.209(2H,q,J=6Hz), 3.962(2H,t,J=6Hz), 4.80∼4.94(1H,m), 6.64∼7.28(10H,m), 7.84∼7.92(2H,m)

### Compound (71)

| Elementary analysis (%): C21H19BrClNO4S | | | | | | |
|---|---|---|---|---|---|---|
| Calcd. | C =50.77, | H =3.85, | N =2.82, | Br =16.08, | Cl =7.14, | S =6.45 |
| Found | C =50.72, | H =3.78, | N =2.90, | Br =15.81, | Cl =6.97, | S =6.35 |

### Compound (72)

| Elementary analysis (%): C29H35ClN2O4S HCl (corresponding hydrochloride) | | | | | |
|---|---|---|---|---|---|
| Calcd. | C =60.10, | H =6.26, | N =4.83, | Cl =12.23, | S =5.53 |
| Found | C =59.84, | H =6.11, | N =4.88, | Cl =11.97, | S =5.37 |

NMR (CDCl₃) δ ppm (300 MH_{Z}) [free]
1.291(9H, s), 1.897(2H, quint, *J* = 6Hz), 2.490∼2.610(6H, m), 2.98∼3.10(4H, m), 3.937(2H, t, *J* = 6Hz), 6.650∼6.700(1H, m), 6.823(2H, d, *J* = 9Hz), 6.940(2H, d, *J* = 9Hz), 7.020∼7.100(2H, m), 7.203 (1H, t, *J* = 8Hz), 7.510 (2H, d, *J =* 9Hz), 7.695(2H, d, *J =* 9Hz)

### Compound (73)

| Elementary analysis (%): C33H44N2O4S HCl (corresponding hydrochloride) | | | | | |
|---|---|---|---|---|---|
| Calcd. | C =65.92, | H =7.54, | N =4.66, | Cl =5.90, | S =5.33 |
| Found | C =65.76, | H =7.46, | N =4.68, | Cl =5.80, | S =5.30 |

NMR (CDCl₃)δ ppm (300 MH_{Z}) (free)
1.289(9H, s), 1.342(9H, s), 1.900(2H. quint, *J* = 6Hz), 2.490∼2.610(6H, m), 2.77∼3.11(2H, m), 3.937(2H, t, *J* = 6Hz), 6.650∼6.700(1H, m), 6.821(2H, d, *J =* 9Hz), 6.936(2H, d, *J =* 9Hz), 7.030 (1H, t, *J =* 2Hz), 7.050∼7.095(1H, m), 7.200 (1H, t, *J =* 7Hz), 7.527 (2H, d, *J =* 9Hz), 7.676(2H, d, *J =* 9Hz)

### Compound (74)

| Elementary analysis (%): C22H19ClF3NO4S | | | | | | |
|---|---|---|---|---|---|---|
| Calcd. | C=54.38, | H=3.94, | N=2.88, | Cl=7.30, | F=11.73, | S=6.60 |
| Found | C=54.18, | H=4.09, | N=13.05, | Cl=7.34, | F=11.56, | S=6.47 |

NMR (CDCl₃) δ ppm (300 MH_{Z})
1.981(2H,quint,J=6Hz), 3.227(2H,q,J=6Hz), 3.987(2H,t,J=6Hz), 4.70∼4.84(1H,m), 6.82∼7.04(6H,m), 7.462(2H,d,J=9Hz), 7.547(2H,d,J=9Hz), 7.803(2H, d, J=9Hz)

### Compound (75)

| Elementary analysis (%): C27H30ClNO6S.2H2O | | | | | |
|---|---|---|---|---|---|
| Calcd. | C=57.09, | H=6.013, | N=2.47, | Cl=6.24, | S=5.68 |
| Found | C=56.73, | H=5.43, | N=2.71, | Cl=6.21, | S=5.55 |

NMR (CD3OD) δ ppm (300 MH_{Z})
1.282(9H,s), 1.992(2H,quint,J=6Hz), 3.505(2H,t,J=7Hz), 3.870(2H,s), 3.923(2H,t,J=6Hz), 6.66∼7.24(8H,m), 7,480(2H,d,J=9Hz), 7.850(2H,d,J=9Hz)

### Compound (76)

| Elementary analysis (%): C27H30FNO6S.1/2H2O | | | | | |
|---|---|---|---|---|---|
| Calcd. | C=61.82, | H=5.96, | N=2.67, | F=3.62, | S=6.11 |
| Found | C=61.96, | H=5.79, | N=2.83, | F=3.51, | S=6.12 |

NMR (CD3OD) δ ppm (300 MH_{Z})
1.277(9H,s), 1.988(2H,quint,J=6Hz), 3.470(2H,t,J=7Hz), 3.935(2H,t,J=6Hz), 4.055(2H,s), 6.64∼7.26(10H,m), 7.85∼7.95(2H,m)

### Compound (77)

| Elementary analysis (%): C26H28F3NO4S.1/10H2O | | | | | |
|---|---|---|---|---|---|
| Calcd. | C=61.31, | H=5.58, | N=2.75, | F=11.19, | S=6.29 |
| Found | C=61.30, | H=5.63, | N=2.83, | F=10.71, | S=6.11 |

NMR (CDCl₃) δ ppm (300 MH_{Z}) (free)
1.340(9H,s), 1.995(2H,quint,J=6Hz), 3.211(2H,q,J=6Hz), 4.007(2H,t,J=6Hz), 4.70∼4.85(1H,m), 6.84∼7.02(6H,m), 7.48∼7.82(6H,m)

### Compound (78)

| Elementary analysis (%): C23H22F3NO5S | | | | | |
|---|---|---|---|---|---|
| Calcd. | C=57.37, | H=4.61, | N=2.91, | F=11.84, | S=6.66 |
| Found | C=57.34, | H=4.60, | N=3.08, | F=11.69, | S=6.66 |

### Compound (79)

| Elementary analysis (%): C32H31F3N2O5S.HCl.1/4H20 (corresponding hydrochloride) | | | | | | |
|---|---|---|---|---|---|---|
| Calcd. | C=58.80, | H=5.01, | N=4.29, | Cl=5.42, | F=8.72, | S=4.91 |
| Found | C=58.79, | H=5.27, | N=4.22, | Cl=5.18, | F=8.21, | S=4.73 |

NMR (CDCl₃)δ ppm (300 MH_{Z}) (free)
1.925(2H,quint,J=6Hz), 2.48∼2.64(6H,m), 2.96∼3.12(4H,m), 3.964(2H,t,J=6Hz), 6.84∼7.45(13H,m), 7.530(2H,d,J=9Hz), 7.699(2H,d,J=9Hz)

### Compound (80)

| Elementary analysis (%): C28H24F3NO5S | | | | | |
|---|---|---|---|---|---|
| Calcd. | C=61.87, | H=4.45, | N=2.58, | F=10.49, | S=5.90 |
| Found | C=61.69, | H=4.40, | N=2.67, | F=10.25, | S=5.88 |

NMR (CDCl₃) δ ppm (300 MH_{Z}) (free)
1.994(2H,quint,J=6Hz), 3.218(2H,q,J=6Hz), 4.005(2H,t,J=6Hz), 4.72 ∼ 4.80(1H,m), 6.82∼7.45(13H,m), 7.537(2H,d,J=9Hz), 7.812(2H,d,J=9Hz)

### Compound (81)

| Elementary analysis (%): C24H21ClF3NO6S | | | | | | |
|---|---|---|---|---|---|---|
| Calcd. | C=52.99, | H=3.89, | N=2.58, | Cl=6.52, | F=10.48, | S=5.90 |
| Found | C=52.80, | H=3.81, | N=2.68, | Cl=6.34, | F=10.33, | S=5.83 |

NMR (DMSO) δ ppm (300 MH_{Z})
1.926(2H,quint,J=7Hz), 3.936(2H,t,J=6Hz), 4.046(2H,s), 6.90∼7.14(6H,m), 7.634(2H,d,J=9Hz), 7.702(2H,d,J=9Hz), 7.843(2H,d,J=9Hz)

### Compound (82)

| Elementary analysis (%): C28H30F3NO6S .1/10H2O | | | | | |
|---|---|---|---|---|---|
| Calcd. | C=59.27, | H=5.36, | N=2.47, | F=10.04, | S=5.65 |
| Found | C=59.07, | H=5.32, | N=2.57, | F=9.86, | S=5.56 |

### Compound (83)

| Elementary analysis (%): C25H26BrClN2O4S HCl /0.1(Et2O) (corresponding hydrochloride) | | | | | | |
|---|---|---|---|---|---|---|
| Calcd. | C =49.85 | ,H =4.52, | N =4.65, | Br =13.26, | Cl =11.77, | S =5.32 |
| Found | C =50.03, | H =4.63, | N =4.59, | Br =1,3.10, | Cl =11.63, | S =5.26 |

NMR (CDCl₃) δ ppm (300 MH_{Z}) (free)
1.898(2H, quint, *J* = 6Hz), 2.480∼2.600(6H, m), 2.980 ∼ 3.100(4H, m), 3.936(2H, t, *J* = 6Hz), 6.770∼6.860(4H, m), 6.927(2H, d, *J* = 9Hz), 7.377 (2H, d, *J* = 9Hz), 7.513 (2H, d, *J =* 9Hz), 7.696(2H, d, *J* = 9Hz)

### Compound (84)

| Elementary analysis (%): C29H35BrN2O4S HCl/0.5(MeOH) (corresponding hydrochloride) | | | | | | |
|---|---|---|---|---|---|---|
| Calcd. | C =55.82, | H =5.81, | N =4.49, | Br =12.80, | Cl =5.68, | S =5.14 |
| Found | C =55.36, | H =5.98, | N =4.38, | Br =12.48, | Cl =5.54, | S =5.01 |

NMR (CDCl₃) δ ppm (300 MH_{Z}) (free)
1.343(9H, s), 1.902(2H, quint, *J* = 6Hz), 2.480∼2.610(6H, m), 2.960∼3.100(4H, m), 3.936(2H, t, *J =* 6Hz), 6.796(2H, d, *J =* 9Hz), 6.831 (2H, d, *J =* 9Hz), 6.923 (2H, d, *J =* 9Hz), 7.375(2H, d, *J =* 9Hz), 7.528(2H, d, *J =* 9Hz), 7.677(2H, d, *J =* 9Hz)

### Compound (85)

| Elementary analysis (%): C27H31ClN2O4S.HCl (corresponding hydrochloride) | | | | | |
|---|---|---|---|---|---|
| Calcd. | C=58.80, | H=5.85, | N=5.08, | Cl=12.86, | S=5.81 |
| Found | C=58.75, | H=5.78, | N=5.20, | Cl=12.52, | S=5.79 |

NMR (CDCl₃) δ ppm (300 MH_{Z}) (free)
1.886(2H,quint,J=6Hz), 2.207(6H,s), 2.48∼2.60(6H,m), 2.96∼3.10(4H,m), 3.924(2H,t.J=6Hz), 6.64∼7.28(7H,m), 7.504(2H,d,J=9Hz), 7.690(2H,d,J=9Hz)

### Compound (86)

| Elementary analysis (%): C25H24F3NO7S | | | | | |
|---|---|---|---|---|---|
| Calcd. | C=55.65, | H=4.48, | N=2.60, | F=10.56, | S=5.94 |
| Found | C=55.50, | H=4.44, | N=2.68, | F=10.33, | S=5.87 |

NMR (DMSO) δ ppm (300 MH_{Z})
1.908(2H,quint,J=6Hz), 3.822(3H,s), 3.937(2H,t,J=6Hz), 3.971(2H,s), 6.92∼7.12(8H,m), 7.66∼7.80(4H,m)

### Compound (87)

| Elementary analysis (%): C27H31FN2O4S.HCl (corresponding hydrochloride) | | | | | | |
|---|---|---|---|---|---|---|
| Calcd. | C=60.61, | H=6.03, | N=5.24, | Cl=6.63, | F=3.55, | S=5.99 |
| Found | C=60.51, | H=6.04, | N=5.36, | Cl=6.47, | F=3.47, | S=5.91 |

NMR (CDCl₃) δ ppm (300 MH_{Z}) (free)
1.894(2H,quint,J=7Hz), 2.210(6H,s), 2.48∼2.52(6H,m), 2.98∼3.10(4H,m), 3.928(2H,t,J=6Hz), 6.64∼7.82(11H,m)

### Compound (88)

| Elementary analysis (%): C39H26F3NO7S.2H2O | | | | | |
|---|---|---|---|---|---|
| Calcd. | C=56.51, | H=4.74, | N=2.20, | F=8.94, | S=5.03 |
| Found | C=56.88, | H=4.51, | N=2.26, | F=8.51, | S=4.95 |

### Compound (89)

| Elementary analysis (%): C22H22BrNO5S /0.1(Et2O) | | | | | |
|---|---|---|---|---|---|
| Calcd. | C =53.67, | H =4.50, | N =2.84, | Br =16.23, | S =6.51 |
| Found | C =53.83, | H =4.64, | N =2.80, | Br =15.99, | S =6.42 |

NMR (CDCl₃) δ ppm (300 MH_{Z})
1.951(2H, quint, *J =* 6Hz), 3.177(2H, q, *J =* 6Hz), 3.855(4H, s), 3.959(2H, t, *J =* 6Hz), 4.70∼4.79 (1H, m), 6.814(4H, d, *J =* 9Hz), 6.940 (2H, d, *J =* 9Hz), 6.946 (2H, d, *J =* 9Hz), 7.390 (2H, d, *J =* 9Hz), 7.794(2H, d, *J =* 9Hz)

### Compound (90)

| Elementary analysis (%): C21H18ClF2NO4S | | | | | | |
|---|---|---|---|---|---|---|
| Calcd. | C =55.57, | H =4.00, | N =3.09, | Cl =7.81, | F =8.37, | S =7.06 |
| Found | C =55.50, | H =3.97, | N =3.17, | Cl =7.69, | F =8.22, | S =7.03 |

NMR (CDCl₃)δ ppm (300 MH_{Z})
1.951(2H, quint, *J* = GHz), 3.207(2H, q, *J* = 6Hz), 3.944(2H, t, *J* = 6Hz), 4.80∼4.87 (1H, m), 6.750∼7.030 (7H, m) , 7.444 (2H, d, *J* = 9Hz), 7.784(2H, d, *J* = 9Hz)

### Compound (91)

| Elementary analysis (%): C28H29F3N2O4S.HCl (corresponding hydrochloride) | | | | | | |
|---|---|---|---|---|---|---|
| Calcd. | C=57.68, | H=5.19, | N=4.80, | Cl=6.08, | F=9.77, | S=5.50 |
| Found | C=57.62, | H=5.11, | N=4.90, | Cl=5.95, | F=9.64, | S=5.48 |

NMR (CDCl₃) δ ppm (300 MH_{Z}) (free)
1.954(2H,quint,J=6Hz), 2.50∼2.65(6H,m), 3.15∼3.30(4H,m), 3.992(2H,t,6Hz), 6.63∼7.55(15H,m)

### Compound (92)

| Elementary analysis (%): C32H38F3NO3S.HCl (corresponding hydrochloride) | | | | | |
|---|---|---|---|---|---|
| Calcd. | C=66.48, | H=6.80, | N=2.42, | Cl=6.13, | F=9.86 |
| Found | C=66.39, | H=6.80, | N=2.58, | Cl=6.07, | F=9.68 |

NMR (CDCl₃) δ ppm (300 MH_{Z}) (free)
1.315(9H,s), 1.60∼2.25(8H,m), 2.528(2H,t,J=7Hz), 2.76∼2.86(2H,m), 3.38∼3.50(1H,m), 4.015(2H,t,6Hz), 4.518(2H,s), 6.87∼7.57(12H,m)

### Compound (93)

| Elementary analysis (%): C23H24ClNO4S | | | | | |
|---|---|---|---|---|---|
| Calcd. | C=61.94, | H=5.42, | N=3.14, | Cl=7.95, | S=7.19 |
| Found | C=61.92, | H=5.45, | N=3.16, | Cl=7.83, | S=7.07 |

NMR (CDCl₃) δ ppm (300 MH_{Z}) (free)
1.951(2H,quint,J=6Hz), 2.220(6H,s), 3.208(2H,q,J=6Hz), 3.949(2H,t,6Hz), 4.86∼4.94(1H,m), 6.66∼7.08(7H,m), 7.446(2H,d,J=8.4Hz), 7.789(2H,d,8.4Hz)

### Compound (94)

| Elementary analysis (%): C25H27NO4S | | | | |
|---|---|---|---|---|
| Calcd. | C=68.62, | H=6.22, | N=3.20, | S=7.33 |
| Found | C=68.45, | H=6.131, | N=13.22, | S=7.26 |

NMR (CDCl₃) δ ppm (300 MH_{Z}) (free)
2.042(2H,quint,J=6Hz), 2.10(6H,s), 3.300(2H,q,J=6.3Hz), 4.030(2H,t,6Hz), 4.70∼4.92(1H,m), 6.65∼7.52(14H,m)

### Compound (95)

| Elementary analysis (%): C22H21F2NO5S | | | | | |
|---|---|---|---|---|---|
| Calcd. | C =58.79, | H =4.71, | N =3.12, | F =8.45, | S =7.13 |
| Found | C =58.72, | H =4.68, | N =3.23, | F =8.30, | S =7.11 |

NMR (CDCl₃) δ ppm (300 MH_{Z})
1.934(2H, quint, *J* = 6Hz), 3.165(2H, q, *J* = 6Hz), 3.049(3H, s), 3.934(2H, t, *J* = 6Hz), 4.72∼4.86 (1H, m), 6.750∼7.010 (9H, m), 7.784(2H, d, *J* = 9Hz)

### Compound (96)

| Elementary analysis (%): C23H21F2NO4S | | | | | |
|---|---|---|---|---|---|
| Calcd. | C =62.01, | H =4.75, | N =3.14, | F =8.53, | S =7.20 |
| Found | C =61.87, | H =4.76, | N =3.24, | F =8.22, | S =7.09 |

NMR (CDCl₃) δ ppm (300 MH_{Z})
2.047(2H, quint, *J =* 6Hz), 3.310(2H, q, *J =* 6Hz), 4.039(2H, t, *J =* 6Hz), 4.63∼4.74 (1H, m), 6.729(1H, d, *J =* 15Hz), 6.760∼7.010 (6H, m), 7.310∼7.490(4H, m), 7.487(1H, d, *J =* 15Hz),

### Compound (97)

| Elementary analysis (%): C22H19F3ClNO4S | | | | | | |
|---|---|---|---|---|---|---|
| Calcd. | C =54.38, | H =3.94, | N =2.88, | Cl =7.30, | F =11.73, | S =6.60 |
| Found | C =54.41, | H =3.93, | N =3.03, | Cl =7.30, | F =11.53, | S =6.67 |

NMR (CDCl₃)δ ppm (300 MH_{Z})
1.978(2H, quint, *J =* 6Hz), 3.226(2H, q, *J =* 6Hz), 3.982(2H, t, *J =* 6Hz), 4.80∼4.84 (1H, m), 6.841(2H,d, *J =* 9Hz), 6.982(2H,d, *J =* 9Hz), 7.080∼7.450 (4H, m), 7.459(2H, d, *J =* 9Hz), 7.803(2H, d, *J =* 9Hz)

### Compound (98)

| Elementary analysis (%): C24H22F3NO4S | | | | | |
|---|---|---|---|---|---|
| Calcd. | C =60.37, | H =4.64, | N =2.93, | F =11.94, | S =6.72 |
| Found | C =60.28, | H =4.59, | N =3.05, | F =11.67, | S =6.60 |

NMR (CDCl₃) δ ppm (300 MH_{Z})
2.076(2H, quint, *J =* 6Hz), 3.333(2H, q, *J* = 6Hz), 4.077(2H, t, *J* = 6Hz), 4.62∼4.65(1H, m), 6.746(1H,d, *J* = 15Hz), 6.86∼7.00(4H,m), 7.06∼7,18 (2H, m), 7.26∼7.49 (7H, m), 7.503(1H, d, *J =* 15Hz)

### Compound (99)

| Elementary analysis (%): C23H19F6NO4S /0.1(Et2O) | | | | | |
|---|---|---|---|---|---|
| Calcd. | C =53.18, | H =3.69, | N =2.70, | F =21.94, | S =6.17 |
| Found | C =53.34, | H =3.83, | N =2.66, | F =21.64, | S =6.09 |

NMR (CDCl₃)δ ppm (300 MH_{Z})
2.000(2H, quint, *J =* 6Hz), 3.265(2H, q, *J =* 6Hz), 3.999(2H, t, *J =* 6Hz), 4.86∼4.93(1H, m), 6.846(2H,d, *J =* 9Hz), 6.983(2H,d, *J =* 9Hz), 7.07∼7.18 (2H, m), 7.25∼7.33 (1H, m), 7.136∼7.44 (1H, m), 7.762 (2H, d, *J=* 9Hz), 7.999 (2H, d, *J* = 9Hz)

### Compound (100)

| Elementary analysis (%): C25H26ClNO6S | | | | | |
|---|---|---|---|---|---|
| Calcd. | C=59,58, | H=5.20, | N=2.78, | Cl=7.03, | S=6.36 |
| Found | C=59.42, | H=5.16, | N=2.84, | Cl=5.92, | S=6.27 |

NMR (DMSO) δ ppm (300 MH_{Z})
1.902(2H,quint,J=7Hz), 2.171(6H,s), 3.886(2H,t.J=6Hz), 4.037(2H,s), 6.60∼7.13(7H,m), 7.621(2H,d,J=9Hz), 7.831(2H,d,J=9Hz)

### Compound (101)

| Elementary analysis (%): C28H28F5NO3.HCl (corresponding hydrochloride) | | | | | |
|---|---|---|---|---|---|
| Calcd. | C=60.27, | H=5.24, | N=2.51, | Cl=6.35, | F=17.02 |
| Found | C=60.27, | H=5.21, | N=2.66, | Cl=6.44, | F=16.86 |

NMR (CDCl₃) δ ppm (300 MH_{Z}) (free)
1.60∼2.24(8H,m), 2.506(2H,t,J=7Hz), 2.70∼2.83(2H,m), 3.38∼3.50(1H,m)) 3.979(2H,t.J=7Hz), 6.75∼7.00(7H,m), 7.461(2H,d,J=8Hz), 7.600(2H,d,J=8Hz)

### Compound (102)

| Elementary analysis (%): C18H17Cl2NO4.1/4H2O | | | |
|---|---|---|---|
| Calcd. | C=55.90, | H=4.56, | N=3.62 |
| Found | C=55.78, | H=4.41, | N=3.58 |

### Compound (103)

| Elementary analysis (%): C17H16Cl2N2O3S | | | | | |
|---|---|---|---|---|---|
| Calcd. | C=51.14, | H=4.04, | N=7.02, | Cl=17.76, | S=8.03 |
| Found | C=51.11, | H=4.08, | N=6.97, | Cl=17.81, | S=8.13 |

### Compound (104)

NMR (CDCl₃) δ ppm (300 MH_{Z}) (free)
1.976(2H,quint,J=6Hz), 3.222(2H,q,J=6Hz), 4.025(2H,t,J=6Hz), 4.85∼5.00(1H,m), 6.74∼7.20(5H,m), 7.84∼7.92(2H,m)

### Compound (105)

| Elementary analysis (%): C25H25ClF2N2O4S.HCl (corresponding hydrochloride) | | | | | | |
|---|---|---|---|---|---|---|
| Calcd. | C=53.67, | H=4.68, | N=5.01, | Cl=12.67, | F=6.79, | S=5.73 |
| Found | C=53.45, | H=4.73, | N=5.01, | Cl=12.38, | F=6.55, | S=5.68 |

NMR (CDCl₃) δ ppm (300 MH_{Z}) (free)
1.887(2H,quint,,J=7Hz), 2.48 ∼ 2.60(6H,m), 2.95 ∼ 3.10(4H,m), 3.920(2H,t.J=6Hz), 6.76∼7.00(7H,m), 7.501(2H,d,J=9Hz), 7.695(2H,d,J=9Hz)

### Compound (106)

| Elementary analysis (%): C27H28F2N2O4S.HCl.1/2(C2H4)20 (corresponding hydrochloride) | | | | | | |
|---|---|---|---|---|---|---|
| Calcd. | C=59.23; | H=5.83, | N=4.76, | Cl=6.03, | F=6.46, | S=5.45 |
| Found | C=59.00, | H=6,16, | N=4.69, | Cl=5.62, | F=6.08, | S=5.17 |

NMR (CDCl₃)δ ppm (300 MH_{Z}) (free)
1.930(2H,quint,J=7Hz), 2.50 ∼ 2.60(6H,m), 3.15 ∼ 3.30(4H,m), 3.959(2H,t.J=6Hz), 6.676(1H,d,J=15Hz), 6.76∼7.00(7H,m), 7.38∼7.52(6H,m)

### Compound (107)

| Elementary analysis (%): C26H27N2O4Cl2F3S | | | | | | |
|---|---|---|---|---|---|---|
| Calcd. | C=52.80, | H=4.60, | N=4.74, | Cl=11.99, | F=9.64, | S=5.42 |
| Found | C=52.71, | H=4.63, | N=4.77, | Cl=11.56, | F=9.31, | S=5.42 |

NMR (CDCl₃) δ ppm (300 MH_{Z})
2.21∼2.28 (2H, m), 2.57∼2.68 (4H, m), 3.25∼3.29 (4H, m), 3.77∼3.82 (2H, m), 4.03 (2H, t, *J =* 5.4 Hz), 6.94∼7.26 (6H, m), 7.52∼7.60 (4H, m), 7.68 (2H, d, *J =* 8.7 Hz)

### Compound (108)

| Elementary analysis (%): C24H21NO6ClF3S | | | | | | |
|---|---|---|---|---|---|---|
| Calcd. | C=52.99, | H=3.89, | N=2.85, | Cl=6.52, | F=10.48, | S=5.90 |
| Found | C=52.87, | H=3.80, | N=2.67, | Cl=6.51, | F=1029, | S=5.90 |

NMR (CDCl₃) δ ppm (300 MH_{Z})
1.84∼1.92 (2H, m), 3.18 (2H, t, *J* =6.9 Hz), 3.93 (2H, s), 3.98 (2H, t, *J* = 5.6 Hz), 6.92 (2H, d, *J =* 8.6 Hz), 6.96-7.02 (2H, m), 7.08-7.10 (1H, m), 7.17-7.23 (1H, m), 7.41 (2H, d, *J =* 8.7 Hz),7.50 (2H, d, *J =* 8.6 Hz), 7.68 (2H, d, *J =* 8.7 Hz)

### Compound (109)

| Elementary analysis (%): C22H18Cl2F3NO4S 0.1Et2O | | | | | | |
|---|---|---|---|---|---|---|
| Calcd. | C=50.78, | H=3.49, | N=2.69, | Cl=13.63, | S=6.16, | F=10.96 |
| Found | C=50.93, | H=3.63, | N=2.66, | Cl=13.43, | S=6.08, | F=10.80 |

NMR (CDCl₃) δ ppm (300 MH_{Z})
1.997(2H, quint, *J =* 6Hz), 3.221(2H, q, *J =* 6Hz), 3.996(2H, t, *J =* 6Hz ), 4.65 ∼ 4.74(1H, m), 6.785(1H, dd, *J =* 8Hz, 3Hz), 6.930(2H, d, *J =* 9Hz), 6.970(1H, d, *J* = 3Hz), 7.053(1H, d, *J* = 9Hz), 7.481(2H, d, *J* = 8Hz), 7.554(1H, d, *J* = 8Hz), 7.811(2H, d, *J =* 8Hz)

### Compound (110)

| Elementary analysis (%): C24H21F3ClNSO4 | | | | | | |
|---|---|---|---|---|---|---|
| Calcd. | C=56.31, | H=4.13, | N=2.74, | Cl=6.93, | S=6.26, | F=11.13 |
| Found | C=56.15, | H=4.13, | N=2.80, | Cl=6.72, | S=6.11, | F=10.88 |

NMR (CDCl₃) δ ppm (300 MH_{Z})
2.080(2H, quint, *J =* 6Hz), 3.317(2H, q, *J =* 6Hz), 4.071(2H, t, *J =* 6Hz ), 4.68 ∼ 4.80(1H, m), 6.71 ∼ 7.05(6H, m), 7.35 ∼ 7.58(8H, m)

### Compound (111)

| Elementary analysis (%); C29H29F6NO3.C2H2O4 (corresponding oxalate) | | | | |
|---|---|---|---|---|
| Calcd. | C=57.85, | H=4.86, | N=2.18, | F=17.71 |
| Found | C=57.68, | H=5.10, | N=2.29, | F=17.57 |

NMR (CDCl₃)δ ppm (300 MH_{Z})
1.54∼2.00(8H,m), 2.117(2H,t,J=7.8Hz), 2.50∼2.62(2H,m), 3.30∼3.40(1H,m), 3.969(2H,t,J=5.7Hz), 4.573(2H,s), 6.924(2H,d,J=8.7Hz), 6.95 ∼ 7.23(4H,m), 7.452(2H,d,J=8Hz), 7.504(2H,d,J=8.7Hz), 7.595(2H,d,J=8.7Hz)

### Compound (112)

NMR (CDCl₃)δ ppm (300 MH_{Z})
1.60∼2.30(8H,m), 2.499(2H,t,J=7Hz), 2.70∼2.85(2H,m), 3.35∼3.50(1H,m), 3.992(2H,t,J=6.3Hz), 4.592(2H,s), 6.55∼6.76(3H,m), 7.052(2H,d,J=8Hz), 7.20∼7.30(1H,m), 7.459(2H,d,J=8Hz), 7.570(2H,d,J=8Hz), 7.597 (2H,d,J=8Hz)

### Compound (113)

NMR (CDCl₃) δ ppm (300 MH_{Z})
2.095(2H,quint,J=6.6Hz), 3.620(2H,q,J=6.6Hz), 3.900(6H,s), 4.078(2H,t,J=6Hz), 5.90∼6.02(1H,m), 6.275(1H,d,J=15.3Hz), 6.80∼7.64(12H,m)

### Compound (114)

NMR (CDCl₃) δ ppm (300 MH_{Z})
2.093(2H,quint,J=6.3Hz), 3.615(2H,q,J=6.3Hz), 3.829(3H,s), 4.080(2H,t,J=5.7Hz) 5.84∼5.95(1H,m), 6,255(1H,d,J=15.6Hz), 6.85∼7.05(8H,m), 7.448(2H,d,J=9Hz), 7.533(2H,d,J=9Hz), 7.584(1H,d,J=15.6Hz)

### Compound (115)

| Elementary analysis (%): C24H21ClF3NO6S/0.5(H2O) 0.1(iPr2O) | | | | | | |
|---|---|---|---|---|---|---|
| Calcd. | C =52.47, | H =4.19, | N =2.49, | Cl =6.30, | F =10.12 | S =5.69 |
| Found | C =52.66, | H =3.87, | N =2.67, | Cl =6.44, | F =9.92, | S =5.85 |

NMR (CDCl₃) δ ppm (300 MHz) (Free)
1.99∼ 2.08(2H, m), 3.45(2H, t, *J =* 7.1Hz), 3.95(2H, t, *J =* 5.7Hz), 4.11(2H, s), 6.45(1H, m), 6.62∼6.68(2H, m), 7.06(2H, d, *J =* 8.6Hz), 7.24∼7.29(1H, m), 7.43(2H, d, *J =* 8.6Hz), 7.58(2H, d, *J =* 8.6Hz), 7.77(2H, d, *J =* 8.6Hz)

### Compound (116)

| Elementary analysis (%): C22H19ClF3NO4S | | | | | | |
|---|---|---|---|---|---|---|
| Calcd. | C =54.38, | H =3.94, | N =2.88, | Cl =7.30, | F =11.73, | S =6.60 |
| Found | C =54.32, | H =3.97, | N =2.96, | Cl =7.08, | F =11.51, | S =6.67 |

NMR(CDCl₃) δ ppm (300 MHz)
1.79∼1.87(2H, m), 2.97∼3.03(2H, m), 4.01(2H, t, *J =* 5.6Hz), 5.013(1H, t, *J =* 6.0Hz), 6.38∼7.11(5H, m), 7.17∼7.23(1H, m), 7.37(2H, d, *J =* 8.6Hz), 7.55(2H, d, *J =* 8.4Hz), 7.65(2J, d, *J =* 8.6Hz)

### Compound (117)

C29H29F6NO3
NMR (CDCl₃) δ ppm (300 MHz)
1.60∼2.30(8H, m), 2.499(2H, t, J=7Hz), 2.70∼2.85(2H, m), 3.35∼3.50(1H, m), 3.992(2H, t, J=6.3Hz), 4.592(2H, s), 6.55∼6.76(3H, m), 7.052(2H, d, J=8Hz), 7.20∼7.30(1H, m), 7.459(2H, d, J=8Hz), 7.570(2H, d, J=8Hz), 7.597(2H, d, J=8Hz)

### Compound (118)

| Elementary analysis (%): C25H25ClN2O4S | | | | | |
|---|---|---|---|---|---|
| Calcd. | C =57.72, | H =5.77, | N =6.41, | Cl =8.11, | S =7.34 |
| Found | C =57.68, | H =5.81, | N =6.43, | Cl =7.89, | S =7.34 |

NMR (CDCl₃) δ ppm (300 MHz)
1.88∼1.97(2H, m), 2.51∼2.57(6H, m), 2.55(3H, s), 3.04(4H, br), 4.02(2H, t, *J =* 6.3 Hz), 6.88(2H, d, *J =* 9.3Hz), 7.52(2H, d, *J =* 8.7Hz), 7.70(2H, d, *J* = 8.7Hz), 7.71(2H, d, *J =* 9.3Hz)

### Compound (119)

C22H22ClNO5S
NMR (CDCl₃) δ ppm (300 MHz)
1.948(2H, quint, *J =* 6Hz), 3.208(2H, q, *J =* 6Hz), 3.795(3H, s), 3.943(2H, t, *J =* 6Hz ), 4.83∼4.92(1H, m), 6.75∼6.96(8H, m), 7.536(2H, d, *J =* 8Hz), 7.787(2H, d, *J* = 8Hz)

### Compound (120)

C24H21F3N2O3
NMR (CDCl₃)δ ppm (300 MHz)
2.108(2H,quint,J=6Hz), 3.641(2H,q,J=6.3Hz), 4.087(2H,t,J=6Hz), 6.10 ∼ 6.30(1H,m), 6.562(1H,d,J=15.9Hz), 6.88∼7.05(6H,m), 7.339(2H,d,J=5.7Hz), 7.50∼7.62(3H,m), 8.55∼8.75(2H,m)

### Compound (121)

C23H20F3NO4
NMR (CDCl₃) δ ppm (300 MHz)
2.085(2H,quint,J=6.3Hz), 3.606(2H,q,J=6.3Hz), 4.073(2H,t,J=6Hz), 5.80 ∼ 5.95(1H,m), 6.115(1H,d,15.3Hz), 6,555(1H,s), 6.88 ∼ 7.08(6H,m), 7.42 ∼ 7.68(5H,m)

### Compound (122)

C24H25NO5S
NMR (CDCl₃) δ ppm (300 MHz)
2.048(2H, quint, *J =* 6Hz), 3.313(2H, q, *J =* 6Hz), 3.793(3H, s), 4.039(2H, t, *J =* 6Hz ), 4.67∼4.77(1H, m), 6.732(1H, d, *J* = 15Hz ), 6.78∼6.94(7H, m), 7.38∼7.48(5H, m), 7.491(1H, d; *J =* 15Hz )

### Compound (123)

| Elementary analysis (%): C26H26ClF3N2O4S | | | | | | |
|---|---|---|---|---|---|---|
| Calcd. | C =56.27, | H =4.72, | N =5.05, | Cl =6.39, | F =10.27, | S =5.78 |
| Found | C =56.08, | H =4.82, | N =5.09, | Cl =6.27, | F =10.10, | S =5.62 |

NMR (CDCl₃) δ ppm (300 MHz)
1.85∼1.93(2H, m), 2.49∼2.56(6H, m), 3.03(4H, br), 3.93(2H, t, *J =* 6.3Hz), 6.55 ∼6.70(3H, m), 7.04(2H, d, *J* = 8.6Hz), 7.21∼7.27(1H, m), 7.51(2H, d, *J* = 8.6Hz), 7.57(2H, d, *J =* 8.6Hz), 7.69(2H, d, *J =* 8.6Hz)

### Compound (124)

C24H24ClNO7S
NMR (CD₃OD) δ ppm (300 MHz)
1.974(2H, quint, *J =* 6Hz), 3.471(2H, t, *J =* 6Hz), 3.763(3H, s), 3.902(2H, t, *J =* 6Hz), 4.094(2H, s), 6.78∼6.91(7H, m), 7.495(2H, d, *J =* 9Hz), 7.824(2H, d, *J =* 9Hz)

### Compound (125)

NMR (CDCl₃) δ ppm (300 MHZ) C22H19ClF3NO4S
1.90∼2.00(2H, m), 3,12∼3.23(2H, m), 3.96(2H, t, *J* = 5.6Hz), 4.76(1H, t, *J* = 5.6Hz), 6.53∼6.54(1H, m), 6.63∼6.68(2H, m), 7.06(2H, d, *J =* 8.4Hz), 7.24∼7.30(1H, m), 7.45(2H, d, *J* = 8.7Hz), 7.58(2H, d, *J* = 8.4Hz), 7.78(2H, d, *J* = 8.7Hz).

### Compound (126)

C25H21ClF3NO3
NMR (CDCl₃) δ ppm (300 MHz)
2.099(2H, quint, J=6.3Hz), 3.626(2H,q, J=6.3Hz), 4.083(2H, t, J=6Hz), 5.90∼6.03(1H,m), 6.352(1H, d, J=15.6Hz), 6.90∼7.05(6H, m), 7.341(2H, d, J=8.7Hz), 7.430(2H, d, J=8.7Hz), 7.50∼7.62(3H, m)

### Compound (127)

C21H22N2O5S
NMR (CDCl₃) δ ppm (300 MHz)
1.979(2H, quint, *J =* 6Hz), 3.263(2H, q, *J =* 6Hz), 3.795(3H, s), 3.969(2H, t, *J =* 6Hz), 5.00∼5.10(1H, m), 6.74∼6.96(8H, m), 7.429(1H, dd, J1= 9Hz, J2=5Hz), 8.10∼8.17(1H, m), 8.787(1H, d, *J =* 5Hz), 9.090(1H, s)

### Experimental Example 1

The present compound was administered subcutaneously for 7 days to 7-8 week aged male KK-Ay mouse (CLEA JAPAN, INC). After the mouse fasted for 24 hours, oral glucose tolerance test (OGTT) was conducted. In OGTT, glucose (3 g/kg) was administered orally. After 15 minites, the blood was collected from abdominal aorta by a syringe containing 50 U heparin and centrifuged 12000xg for five minites. Glucose concentration in blood (:Glu) was measured by New Blood · Sugar · Test (Boehringer Ingelheim) and insulin concentration in blood(:Ins) was measured by Insulin-EIA Test(GLAOZYME). The result is shown below. Each value in table 1 is an avarage value of six mouse. In table 2, each concentration after 15 minites is shown by percent versus each value in control group of mouse to which administered 0.5 % methylcellulose solution after OGTT test.

**(Table 1)**

| (Dose : 80 mg/kg) | | | | | |
|---|---|---|---|---|---|
| | | Initial Experiment | 7 days after | From 7 days to 24 hours fasting | After 15 minites of glucose administration |
| | Glu | 318 | 318 | 170 | 727 |
| Control | Ins | 97 | 97 | 5 | 17 |
| Example 1 | Glu | 318 | 219 | 89 | 567 |
| (9a) | Ins | 97 | 55 | 5 | 31 |

**(Table 2-1)**

| KK-Ay(8-9w) Dose:80mg/kg [ ] :40mg/kg | | | |
|---|---|---|---|
| OGTT(3g/kg):15min.after | | | |
| Example | CompoundNo | Glu | Ins |
| 1 | 9a | **78 | 179 |
| 2 | 9b | *85 | *210 |
| 3 | 9c | 88 | *218 |
| 4 | 9d | **77 | **273 |
| 5 | 9e | *84 | *231 |
| 6 | 9f | 88 | *228 |
| 7 | 9g | *84 | 146 |
| 10 | 9J | 82 | **225 |
| 11 | 9K | **85 | 223 |
| 13 | 17 | 87 | *258 |
| 15 | 26 | **88 | 193 |
| 17 | 25 | **83 | 140 |

| | | | |
|---|---|---|---|
| *: The reliability of significant difference is 95% or more ; | | | |
| **: The reliability of significant difference is 99% or more | | | |

**(Table 2-2)**

| KK-Ay(8-9w) Dose:80mg/kg [ ] :40mg/kg | | |
|---|---|---|
| OGTT(3g/kg):15min.after | | |
| Example 25 Compound No | Glu | Ins |
| 3 | [**57] | [**468] |
| 8 | [91] | [*224] |
| 9 | [88] | [186] |
| 1 2 | [**61] | [**491] |
| 1 3 | **48[**69] | **253[**398] |
| 1 5 | **29 | **239 |
| 1 6 | **53 | **279 |
| 1 7 | **71 | **253 |
| 1 8 | **76 | **200 |
| 1 9 | **45 | **314 |
| 2 4 | 92 | 185 |
| 2 6 | **67 | **346 |
| 2 7 | 92 | **210 |
| 2 8 | 88 | *207 |
| 3 0 | **78 | **290 |
| 3 2 | *72 | 152 |
| 3 3 | **59 | 206 |
| 3 4 | *75 | 248 |
| 3 5 | **74 | 162 |
| 3 6 | **63 | *494 |
| 3 8 | **67 | **265 |
| 3 9 | **53 | **302 |
| 4 7 | *73 | 180 |
| 7 1 | *87 | 142 |
| 8 1 | **51 | **387 |

| | | |
|---|---|---|
| *: The reliability of significant difference is 95% or more ; | | |
| **: The reliability of significant difference is 99% or more | | |

After 7 days from initial experience, the glucose concentration in blood of group administered the present compound before experience decreased compared with control group. Accordingly, insulin concentration also. decreased. Namely, glucose availability increased by administering the present compound, consequently glucose concentration in blood decreased. Futhermore, OGGT 15 minites after, in control group, response of insulin secretion against glucose administration was weak and glucose value became high. However, in group administered the present compound, insulin acted effectively accompanied with increase of insulin and the value of glucose became lower compared with control group.

### Formulation Example 1

The compound 9a of Example 1, crystalline cellulose, magnesium stearate and the like, each proper amount was mixed and the mixture was tabletted to give tablets.

### Formulation Example 2

After the compound 9a of Example 1, lactose and magnesium stearate and the like were mixed and the mixture was extruded to give granules.

### Formulation Example 3

The granule of Formulation Example 2 was capsulated to give capsule.

### Industrial Applicability

The present compound is for use as a preventive and therapeutic agent for diabetes and the like.

## Claims

1. A composition for use as a preventive or therapeutic agent for diabetes, containing a compound of the formula (I): 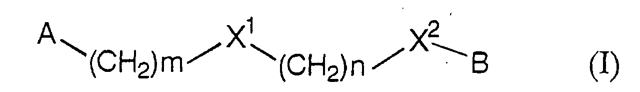 wherein A is optionally substituted aryl or optionally substituted heteroaryl; B is lower alkyl, optionally substituted aryl, optionally substituted aryl(lower)alkyl, optionally substituted aryl(lower)alkenyl, optionally substituted heteroaryl, optionally substituted heteroaryl(lower)alkyl, or optionally substituted heteroaryl(lower)alkenyl;
X¹ is -O-, -S-, or -NR^{a}- wherein R^{a} is hydrogen or lower alkyl;
X² is -NR^{b}CO-, -CONR^{b}-, -NR^{b}CONR^{b}-, -SO₂-, -NR^{b}SO₂-, -D-, -D-O-, -D-CO-, -D-SO₂-, -D-NR^{b}CO- or -D-NR^{b}SO₂- wherein D is a divalent heterocyclic group; R^{b} is hydrogen or optionally substituted lower alkyl;
m is an integer of 0 to 3;
n is an integer of 2 to 5;
prodrug, pharmaceutically acceptable salt, or solvate thereof.

2. The composition for use as a preventive or therapeutic agent for diabetes described in claim 1 wherein A is optionally substituted phenyl, optionally substituted naphtyl, optionally substituted pyridyl, optionally substituted quinoryl, optionally substituted isoquinoryl, optionally substituted furyl, optionally substituted thienyl, optionally substituted benzofuryl, or optionally substituted benzothienyl.

3. The composition for use as a preventive or therapeutic agent for diabetes described in claim 2 wherein A is optionally substituted phenyl.

4. The composition for use as a preventive or therapeutic agent for diabetes described in claim 3 wherein A is phenyl substituted with one to three halogens.

5. The composition for use as a preventive or therapeutic agent for diabetes described in claim 4 wherein A is phenyl substituted with two halogens.

6. The composition for use as a preventive or therapeutic agent for diabetes described in claim 3 wherein A is phenyl substituted with halogen and lower alkyl.

7. The composition for use as a preventive or therapeutic agent for diabetes described in claim 3 wherein A is phenyl substituted with lower alkyl or haloganated lower alkyl.

8. The composition for use as a preventive or therapeutic agent for diabetes described in claim 3 wherein A is phenyl substituted with substituted phenyloxy.

9. The composition for use as a preventive or therapeutic agent for diabetes described in claim 3 wherein A is phenyl which is substituted with one or more group(s) selected from the group consisting of halogen, lower alkyl, lower alkoxy and haloganated lower alkyl and phenyl which is substituted with substituted phenyloxy.

10. The composition for use as a preventive or therapeutic agent for diabetes described in claim 1 wherein B is lower alkyl, optionally substituted phenyl, optionally substituted benzyl, optionally substituted 2-phenylvinyl, optionally substituted pyridyl, optionally substituted 2-pyridylvinyl, optionally substituted furyl, optionally substituted 2-furylvinyl, optionally substituted thienyl, optionally substituted 2-thienylvinyl, or optionally substituted thiomorphorinyl.

11. The composition for use as a preventive or therapeutic agent for diabetes described in claim 1 wherein when B is an optionally substituted group, the substituent being selected from the group consisting of halogen, amino, carboxyl, hydroxy, cyano, lower alkyl, optionally substituted alkoxy, lower alkoxycarbonyl, halogenated lower alkyl, aryloxy, and heteroaryloxy.

12. The composition for use as a preventive or therapeutic agent for diabetes described in claim 1 wherein X¹ is O.

13. The composition for use as a preventive or therapeutic agent for diabetes described in claim 1 wherein X² is -NHCO-, -NHCONH-, NR^{b}SO₂, -SO₂-, -D-, -D-O-, -D-SO₂-, -D-NR^{b}CO- or -D-NR^{b}-SO₂- wherein D is piperidin-1,4-diyl or piperazin-1,4-diyl, and R^{b} is the same as above.

14. The composition for use as a preventive or therapeutic agent for diabetes described in claim 1 wherein m is 0.

15. The composition for use as a preventive or therapeutic agent for diabetes described in claim 1 wherein n is 3.

16. The composition for use as a preventive or therapeutic agent for diabetes described in claim 1 wherein A is optionally substituted phenyl; B is lower alkyl, optionally substituted phenyl, optionally substituted benzyl, optionally substituted 2-phenylvinyl, optionally substituted pyridyl, optionally substituted 2-pyridylvinyl, optionally substituted furyl, optionally substituted 2-furylvinyl, optionally substituted thienyl, optionally substituted 2-thienylvinyl or optionally substituted thiomorphoryl; X¹ is O; X² is -NHCO-, -NHCONH-, -SO₂-, -NR^{b} SO₂-, -D-, -D-O-, -D-SO₂-, -D-NR^{b}CO- or -D-NR^{b}SO₂- wherein D is piperizin-1,4-diyl or piperazin-1,4-diyl, R^{b} is the same meaning as above); m is 0; and n is 3.

17. A compound of the formula (II): 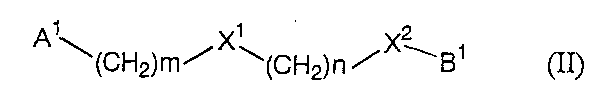 wherein A¹ is any one of groups shown by the formulae: 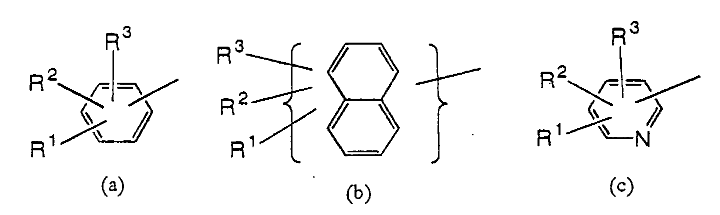 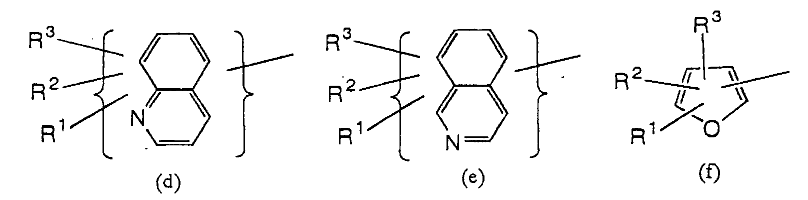 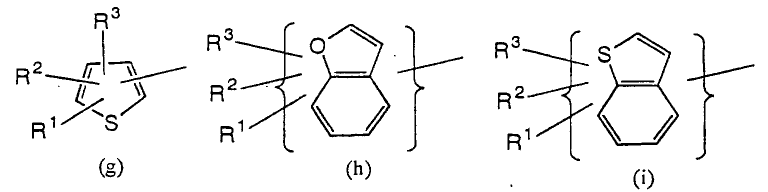 wherein R¹,R² and R³ are each independently hydrogen, halogen, lower alkyl, lower alkoxy, halogenated lower alkyl, or substituted phenyloxy;
B¹ is lower alkyl or any one of groups shown by the formulae: 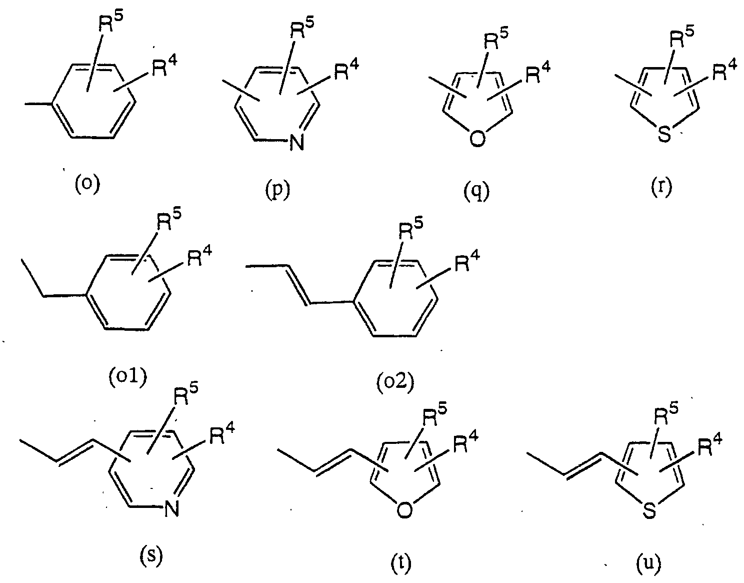 wherein R⁴ and R⁵ are each independently hydrogen, halogen, amino, carboxyl, hydroxy, cyano, lower alkyl, optionally substituted lower alkoxy, lower alkoxy carbonyl, halogenated lower alkyl, aryloxy or heteroaryloxy;
X¹ is -O-, -S- or -NR^{a}- wherein R^{a} is hydrogen or lower alkyl; X² is -NR^{b}CO-, -CONR^{b}-, -NR^{b}CONR^{b}-, -SO₂-, -NR^{b}SO₂-, or any one of groups shown by the formulae: 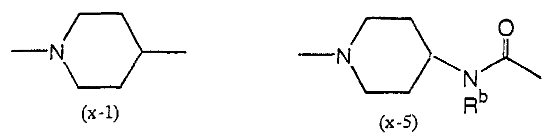 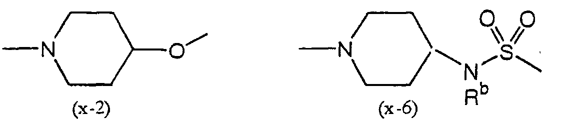 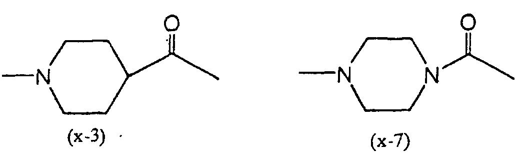 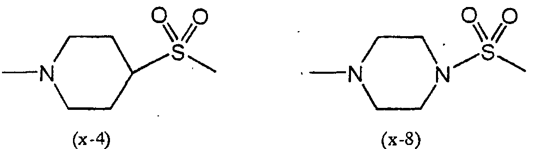 wherein each R^{b} is independently hydrogen or optionally substituted lower alkyl;
m is an integer of 0 to 3;
n is an integer of 2 to 5;
excluding the following 1) and 2):
1) A¹ is the group of (a), R¹ is hydrogen, R² and R³ are chloro; B¹ is the group of (o), R⁴ is chloro or methyl, R⁵ is hydrogen; X' is O; X² is NR^{b} SO₂; m is 0; and n is 2, and
2) A¹ is the group of (a), R¹ is hydrogen, R² and R³ are any group selected from the group consisting of hydrogen, halogen and lower alkoxy; X¹ is O; X² is the group of (x-1); B¹ is lower alkyl or phenyl; m is 0; and n is 3; prodrug, pharmaceutically acceptable salt or solvate thereof.

18. The compound described in claim 17 wherein A¹ is any one of groups shown by the formulae: 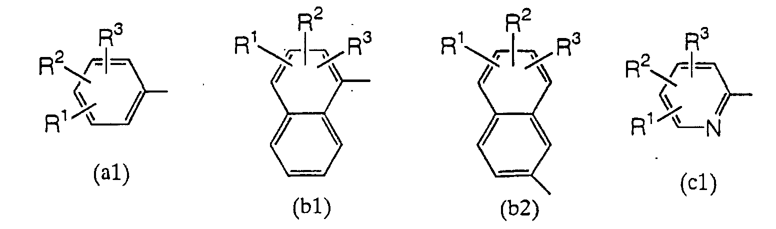 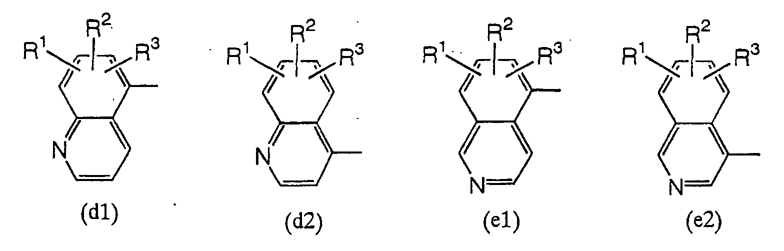 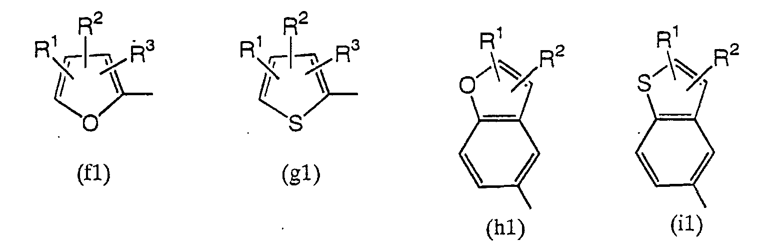 wherein each symbol is the same meaning as described above; B¹ is any one of groups shown by the formulae: 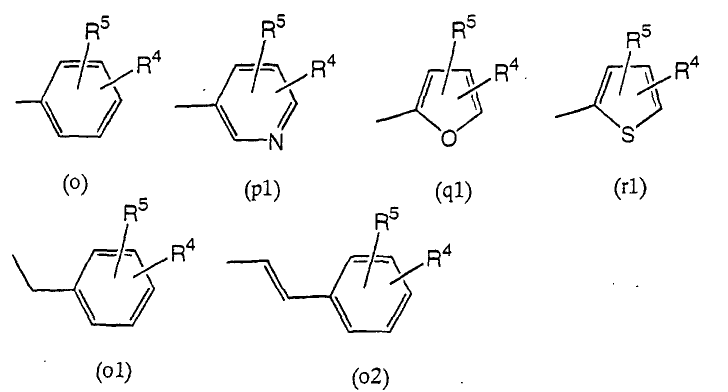 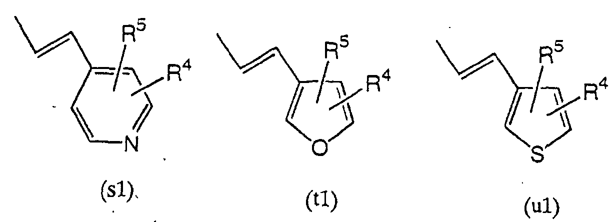 wherein R⁴ and R⁵ are the same as described above.

19. The compound described in claim 18 wherein A¹ is the group of (a1); R¹ is hydrogen; R² is halogen, lower alkyl, lower alkoxy, halogenated lower alkyl, or substituted phenyloxy; and R³ is hydrogen, halogen, lower alkyl, lower alkoxy, or halogenated lower alkyl.

20. The compound described in claim 19 wherein R³ is hydrogen.

21. The compound described in claim 18 wherein B¹ is the group of (o), (o1) or (o2); R⁴ is hydrogen, and R⁵ is hydrogen, halogen, amino, carboxyl, hydroxy, cyano, lower alkyl, optionally substituted lower alkoxy, lower alkoxycarbonyl, halogenated lower alkyl, aryloxy, or heteroaryloxy.

22. The compound described in claim 17 or 18 wherein X¹ is O.

23. The compound described in claim 17 wherein X² is -NHCO-, -NHCONH-, -NHSO₂- or any one of groups shown by the formulae:

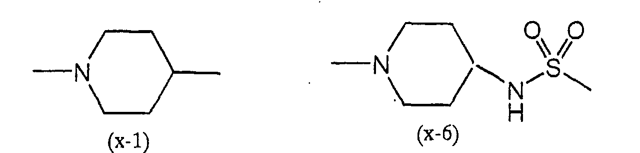

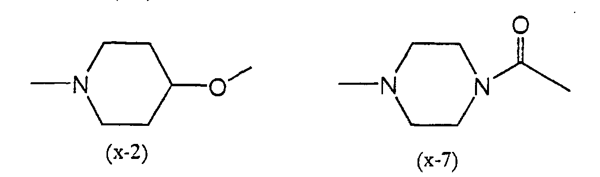

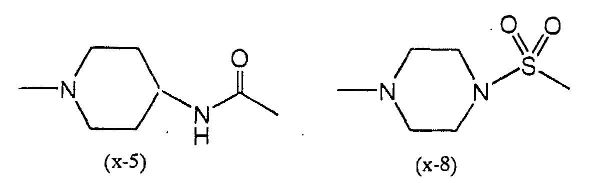

24. The compound described in claim 17 or 18 wherein m is 0.

25. The compound described in claim 17 or 18 wherein n is 3.

26. The compound described in claim 17 or 18 wherein A¹ is the group of (a1); R¹ is hydrogen; R² is halogen, lower alkyl, lower alkoxy, halogenated lower alkyl or substituted phenyloxy; R³ is hydrogen, halogen, lower alkyl, lower alkoxy or halogenated lower alkyl; B¹ is the group of (o), (o1) or (o2); R⁴ is hydrogen, R⁶ is hydrogen, halogen, amino, carboxyl, hydroxy, cyano, lower alkyl, optionally substituted lower alkoxy, lower alkoxycarbonyl, halogenated. lower alkyl, aryloxy or heteroaryloxy; X¹ is O; X² is -NHCO-, -NHCONH-, -NHSO₂- or any one of groups shown by 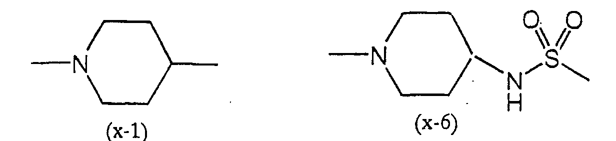 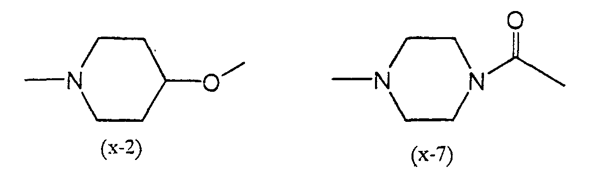 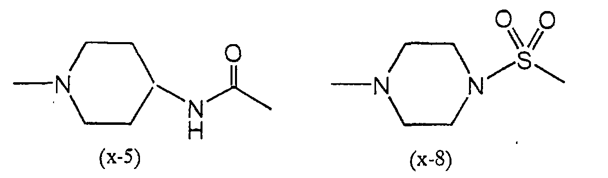 m is 0 and n is 3.

27. The compound described in claim 26 wherein R³ is hydrogen; and R⁵ is hydrogen, halogen, carboxyl, hydroxy, lower alkyl, optionally substituted lower alkoxy, lower alkoxycarbonyl or halogenated lower alkyl.

28. A pharmaceutical composition containing a compound described in any one of claims 17 to 27.

29. A composition for use as a preventive or therapeutic agent for diabetes containing a compound described in any one of claims 17 to 27.

30. A composition for use as a preventive or therapeutic agent for diabetes containing a compound described in claim 27.

31. A method for the prevention or therapy of diabetes, which comprises administering a compound described in any one of claims 1 to 27.

32. Use of a compound described in any one of claims 1 to 27 for production of a composition for use as a preventive or therapeutic agent for diabetes.
